(19) Europäisches Patentamt / European Patent Office / Office européen des brevets

(11) **EP 4 274 802 B1**

(12) **EUROPEAN PATENT SPECIFICATION**

(45) Date of publication and mention
of the grant of the patent:
**15.07.2026 Bulletin 2026/29**

(21) Application number: **22736998.0**

(22) Date of filing: **04.01.2022**

(51) International Patent Classification (IPC):
**B82Y 15/00** (2011.01)    **C12N 15/113** (2010.01)
**C12N 15/115** (2010.01)    **C12Q 1/68** (2018.01)
**C12Q 1/6825** (2018.01)    **C12Q 1/6897** (2018.01)

(52) Cooperative Patent Classification (CPC):
(C-Sets available)
**C12N 15/111; C12Q 1/44; C12Q 1/6825;**
**C12Q 1/6897; G01N 33/542;** B82Y 5/00;
C12N 2310/16; C12N 2310/20; C12N 2310/3519;
C12N 2320/50; G01N 2333/9005; G01N 2333/922
(Cont.)

(86) International application number:
**PCT/US2022/011191**

(87) International publication number:
**WO 2022/150311 (14.07.2022 Gazette 2022/28)**

(54) **MODULAR KINETICALLY-CONTROLLED FUNCTIONAL RNA CONSTRUCTS AND RELATED COMPOSITIONS, SYSTEMS, AND METHODS**

MODULARE KINETISCH GESTEUERTE FUNKTIONELLE RNA-KONSTRUKTE UND ZUGEHÖRIGE ZUSAMMENSETZUNGEN, SYSTEME UND VERFAHREN

CONSTRUCTIONS D'ARN FONCTIONNELLES À COMMANDE CINÉTIQUE MODULAIRE ET COMPOSITIONS, SYSTÈMES ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2021 US 202163133950 P**

(43) Date of publication of application:
**15.11.2023 Bulletin 2023/46**

(73) Proprietor: **University of Washington**
**Seattle, Washington 98105-4721 (US)**

(72) Inventors:
• **CAROTHERS, James, M.**
**Seattle, Washington 98105-4721 (US)**
• **ZALATAN, Jesse**
**Seattle, Washington 98105-4721 (US)**
• **SPARKMAN-YAGER, David**
**Seattle, Washington 98105-4721 (US)**
• **FONTANA, Jason**
**Seattle, Washington 98105-4721 (US)**

(74) Representative: **MacLean, Martin Robert et al**
**Mathys & Squire**
**The Shard**
**32 London Bridge Street**
**London SE1 9SG (GB)**

(56) References cited:
**US-A1- 2014 051 090    US-A1- 2016 046 934**
**US-A1- 2020 032 253    US-A1- 2020 255 835**
**US-A1- 2020 268 907**

• **WIDOM JULIA R ET AL: "Ligand Modulates Cross-Coupling between Riboswitch Folding and Transcriptional Pausing", MOLECULAR CELL, ELSEVIER, AMSTERDAM, NL, vol. 72, no. 3, 1 November 2018 (2018-11-01), pages 541, XP085522303, ISSN: 1097-2765, DOI: 10.1016/ J.MOLCEL.2018.08.046**

- FAREED ABOUL-ELA ET AL: "Linking aptamer-ligand binding and expression platform folding in riboswitches: prospects for mechanistic modeling and design : Linking aptamer-ligand binding and expression platform folding in riboswitches", WILEY INTERDISCIPLINARY REVIEWS: RNA, vol. 6, no. 6, 11 September 2015 (2015-09-11), United Kingdom, pages 631 - 650, XP055728367, ISSN: 1757-7004, DOI: 10.1002/wrna.1300
- PHAM HOANG LONG ET AL: "Engineering a riboswitch-based genetic platform for the self-directed evolution of acid-tolerant phenotypes", vol. 8, no. 1, 4 September 2017 (2017-09-04), UK, XP093198780, ISSN: 2041-1723, Retrieved from the Internet <URL:https://www.nature.com/articles/s41467-017-00511-w.pdf> DOI: 10.1038/s41467-017-00511-w
- YUCHEN LIU ET AL: "Directing cellular information flow via CRISPR signal conductors", NATURE METHODS, vol. 13, no. 11, 1 November 2016 (2016-11-01), New York, pages 938 - 944, XP055462690, ISSN: 1548-7091, DOI: 10.1038/nmeth.3994
- QUENTIN R. V. FERRY ET AL: "Rational design of inducible CRISPR guide RNAs for de novo assembly of transcriptional programs", NATURE COMMUNICATIONS, vol. 8, 3 March 2017 (2017-03-03), pages 14633, XP055533650, DOI: 10.1038/ncomms14633
- BURKE CASSANDRA R., SPARKMAN-YAGER DAVID, CAROTHERS JAMES M.: "Multi-state design of kinetically-controlled RNA aptamer ribosensors", BIORXIV, 3 November 2017 (2017-11-03), XP055956268, [retrieved on 20220830], DOI: 10.1101/213538

Remarks:

The complete document including Reference Table(s) and the Sequence Listing(s) can be downloaded from the EPO website

(52) Cooperative Patent Classification (CPC): (Cont.)

C-Sets
C12Q 1/6825, C12Q 2525/205, C12Q 2525/301, C12Q 2527/113;
C12Q 1/6897, C12Q 2525/205, C12Q 2525/301, C12Q 2527/113

**Description**

This invention was made with Government support under Grant Nos. CBET 1844152. CBET- 18441 52-001, EF-1935087, and MCB 1817623. awarded by the National Science Foundation. The Government has certain rights in the invention.

STATEMENT REGARDING SEQUENCE LISTING

The sequence listing associated with this application is provided in text format in lieu of a paper copy. The name of the text file containing the sequence listing is 3915-P1 161WOUW_Seq_List_FINAL_20220104_ST25. The text file is 13 KB; was created on January 4, 2022. and is being submitted via EFS-Web with the filing of the specification.

BACKGROUND

RNA biomolecules have been proposed for use as molecular switches for sensing applications. For example, the development of high-value industrial bioproducts such as enzymes, biofuels, biomaterials and biochemicals constitute a multi-billion dollar industry. However, current technologies to synthesize and assemble genetic variants in engineered microbes to produce desired bioproducts greatly exceeds the capacity to screen those variants for improved production titers, rates and yields, resulting in major bottlenecks hindering production development and optimization. RNA biomolecular switches can theoretically be employed as biosensors to sense the production of bioproducts or necessary intermediates, and monitor the efficiency of each step in synthesis pathways.

However, engineered RNA biomolecular switches have heretofore implicitly relied on thermodynamic control where the production of signals resulting from an input state results from complex equilibria between a plethora of transient, ill-defined structure-states. This makes reliable and rational production of sensitive biomolecular sensors at the scale needed for the vast diversity of potential target ligands elusive. There has been a lack of significant success broadly in the field of computational design of functional RNA molecules. Furthermore, optimizing the sensitivity of existing RNA sensors to detect the low concentrations of metabolites available inside a cell has proven challenging.

Despite the advances in the art of biomolecular sensors, there remains a need for a systematic design pipeline to robustly produce sensitive RNA biomolecular switches. The present disclosure addresses these and related needs.

Widom JR, Nedialkov YA, Rai V, Hayes RL, Brooks CL 3rd, Artsimovitch I, Walter NG. Ligand Modulates Cross-Coupling between Riboswitch Folding and Transcriptional Pausing. Mol Cell. 2018 Nov 1;72(3):541-552.e6. doi: 10.1016/j.molcel.2018.08.046. PMID: 30388413; PMCID: PMC656538 describes ligand modulation cross-coupling between riboswitch folding and transactional pausing. Burke, C.R., Sparkman-Yager, D. and Carothers, J.M., 2017. Multi-state design of kinetically-controlled RNA aptamer ribosensors, bioRxiv, p.213538 describes Multi-state design of kinetically-controlled RNA aptamer ribosensors.

SUMMARY

The invention is defined by the claims. The invention provides a kinetically-controlled RNA biosensor construct, comprising from 5' to 3':

> a sensor domain that specifically binds to a ligand of interest; and
> an output domain configured to modulate a detectable output signal when folded into an active conformation;
> wherein the output domain, when transcribed, folds into the active conformation when the sensor domain is bound to the ligand of interest, and wherein the output domain, when transcribed, folds into an inactive conformation when the sensor domain is not bound to the ligand of interest,
> wherein the sensor domain comprises:

>> an aptamer domain, wherein the aptamer domain comprises, from 5' to 3', a stem sequence, a linker target sequence, an aptamer subsequence, and a stem target sequence; and
>> an overhang sequence 5' of the aptamer domain and, optionally, a linker sequence 3' of the aptamer domain; and
>> wherein the output domain comprises, from 5' to 3':

>>> a stem target sequence,
>>> an overhang target sequence, and
>>> an output subsequence; and
>>> wherein the overhang sequence of the sensor domain is the reverse complement of at least a portion of the overhang target sequence of the output domain.

In some embodiments: the sensor domain comprises the linker sequence.

In some embodiments: the stem sequence of the sensor domain is the reverse complement of at least a portion of the stem target sequence of the sensor domain and is the reverse complement of at least a portion of the stem target sequence of the output domain: and/or the linker sequence of the sensor domain is the reverse complement of at least a portion of linker target sequence of the sensor domain (in any combination.). In some embodiments, the linker target sequence is the reverse complement to a portion of the aptamer subsequence, optionally wherein the portion of the aptamer subsequence is a discontinuous portion. In some embodiments, when the sensor domain is bound to the ligand of interest the stem sequence of the sensor domain is hybridized to the stem target sequence of the sensor domain thereby permitting folding of the output domain into the active conformation.

In some embodiments, when the sensor domain is not bound to the ligand of interest the overhang sequence of the sensor domain is hybridized to the portion of the overhang target sequence of the output domain, the stem sequence of the sensor domain is hybridized to the portion of the stem target sequence of the output domain, and/or the linker target sequence of the sensor domain is hybridized to the portion of the linker sequence in the sensor domain, thereby permitting folding of the output domain into the inactive conformation. In some embodiments, at least two of the overhang sequence of the sensor domain, the stem sequence of the sensor domain, and the linker target sequence of the sensor domain form a continuous helix stem structure when hybridized to at least a portion of the overhang target sequence of the output domain, a portion of the stem target sequence of the output domain, and a portion the linker sequence of the sensor domain, respectively, thereby permitting the output subsequence of the output domain to fold into the inactive conformation.

In some embodiments: the overhang sequence is between 0 and about 15 nucleotides in length: the stem sequence is between 0 and about 15 nucleotides in length: and/or the linker sequence is between 0 and about 15 nucleotides in length.

In some embodiments, the construct further comprises a timer domain disposed between the aptamer domain and the linker sequence of the sensor domain, wherein the timer domain has a length up to about 150 nucleotides in length. In some embodiments, the timer domain is configured to form a stem-and-loop structure with each end of the timer domain forming part of a stem of the stem-and-loop structure. In some embodiments, the timer domain further comprises at least one transcriptional pause sequence. In some embodiments, the timer domain comprises a plurality of transcriptional pause sequences that are the same or different. In some embodiments, at least one transcriptional pause sequence is derived from a thiamine pyrophosphate (TPP)-sensing thiC riboswitch from *Escherichia coli.*

In some embodiments, the output domain is folded into an active conformation the output domain is or comprises a functional ribozyme, a functional nuclease guide RNA (gRNA), a ribosome binding site, a transcriptional terminator, or RNA aptamer. In some embodiments, when the output domain is folded into an active conformation the output domain is or comprises a functional gRNA, wherein the functional gRNA is between about 40 and about 400 nucleotides in length.

In some embodiments, the gRNA is designed using the method described herein. In some embodiments, the functional gRNA associates with a nuclease, optionally selected from Cas9, Cas12a, Cas13. derivatives thereof, and the like. In some embodiments, the nuclease has ablated nuclease function. In some embodiments, the nuclease confers CRISPR activation (CRISPRa) function. In some embodiments, the nuclease confers CRISPR inhibition (CRISPRi) function. In some embodiments, the nuclease has nuclease function.

In some embodiments, the output domain is configured to induce the detectable output signal when folded into the active conformation. In some embodiments, the output domain is configured to reduce the detectable output signal when folded into the active conformation. In some embodiments, the ligand of interest is a chemical, a metabolite, a protein, a peptide, a small molecule, optionally a drug molecule or drug precursor molecule, and the like.

In some embodiments, the kinetically-controlled RNA biosensor construct is designed using a computer-implemented method comprising:

determining, by a computing device, one or more candidate kinetically-controlled RNA biosensor sequences:

for each of the one or more candidate kinetically-controlled RNA biosensor sequences:

predicting, by the computing device, one or more folded structures that the kinetically-controlled RNA biosensor sequence forms over time;
determining, by the computing device, one or more metrics for the kinetically-controlled RNA biosensor sequence based on the predicted one or more folded structures; and
choosing, by the computing device, one or more of the one or more candidate kinetically-controlled RNA biosensor sequences to be provided for synthesis based on the scores.

In another aspect, the disclosure provides a polynucleotide molecule comprising a sequence encoding the kinetically-controlled RNA biosensor construct described herein.

In another aspect, the disclosure provides a vector comprising the polynucleotide molecule described herein operatively linked to a promoter.

In another aspect, the disclosure provides a cell comprising the polynucleotide molecule or the vector described herein. In some embodiments, the cell is prokaryotic. In some embodiments, the cell is eukaryotic. In some embodiments, the cell is engineered to further comprise an expression construct comprising a reporter gene operatively linked to a synthetic promoter, wherein the output domain of the kinetically-controlled RNA biosensor construct is or comprises a functional gRNA when folded into an active conformation, and the functional gRNA hybridizes to the synthetic promoter. In some embodiments, the synthetic promoter contains a Protospacer Adjacent Motif (PAM) positioned between about 40 and about 120 bases 5' of the transcription start site. In some embodiments, the cell is engineered to further express a nuclease with ablated nuclease functionality and a transcription factor, wherein the expressed nuclease and transcription factor associate with the functional gRNA and are configured to facilitate transcription of the reporter gene when the gRNA hybridizes with the synthetic promoter of the expression construct. In some embodiments, the cell is engineered or treated to modify expression or production of the ligand.

In another aspect, the disclosure provides a biosensor system that comprises a first expression cassette comprising sequence encoding the kinetically-controlled RNA biosensor construct as described herein; and an RNA polymerase and NTPs sufficient to facilitate synthesis of the kinetically-controlled RNA biosensor construct.

In some embodiments, the system further comprises protein translation elements selected from ribosomes, tRNAs, aminoacyl-tRNA synthetase, initiation factors, elongation factors, termination factors, amino acids, ATP. GTP, and/or translation co-factors, in any combination. In some embodiments, the system further comprises an expression construct comprising a reporter gene operatively linked to a synthetic promoter, wherein the output domain of the kinetically-controlled RNA biosensor construct is or comprises a functional gRNA when folded into an active conformation, and the functional gRNA hybridizes to the synthetic promoter. In some embodiments, the system further comprises a nuclease with ablated nuclease functionality, and a transcription factor. The nuclease and transcription factor associate with the functional gRNA and are configured to facilitate transcription of the reporter gene when the associated gRNA hybridizes with the synthetic promoter of the expression construct.

The disclosure provides a method of detecting a ligand of interest. The method comprises synthesizing the kinetically-controlled RNA biosensor construct as described herein in an environment that may contain the ligand of interest, and detecting an output signal. A detected output signal indicates binding of the ligand of interest to the sensor domain.

In some instances, the environment is an *in vitro* environment capable of facilitating transcription of the kinetically-controlled RNA biosensor construct. In some instances, the *in vitro* environment comprises an RNA polymerase, NTPs, and a template DNA molecule as described herein to facilitate synthesis of the kinetically-controlled RNA biosensor construct. In some embodiments, the environment is a cell-free synthesis environment. In some instances, the cell-free synthesis environment comprises protein translation elements selected from ribosomes, tRNAs, aminoacyl-tRNA synthetase, initiation factors, elongation factors, termination factors, amino acids, ATP, GTP, and/or translation co-factors, in any combination. In some instances, the environment comprises a cell lysate. In some instances, the environment is in a cell. In some instances, the cell is engineered to further comprise an expression construct comprising a reporter gene operatively linked to a synthetic promoter, wherein the output domain of the kinetically-controlled RNA biosensor construct is or comprises a functional gRNA when folded into an active conformation, and the functional gRNA hybridizes to the synthetic promoter. In some instances, the cell is engineered to further express a nuclease with ablated nuclease functionality and a transcription factor, wherein the expressed nuclease and transcription factor associate with the functional gRNA and are configured to modulate transcription of the reporter gene when the gRNA hybridizes with the synthetic promoter of the expression construct. In some instances, the nuclease with ablated nuclease functionality confers CRISPR activation (CRISPRa) function, and wherein the reporter gene encodes a fluorescent protein, an antibiotic resistance protein, beta-galactosidase, and the like. In some instances, the nuclease with ablated nuclease functionality confers CRISPR inhibition (CRISPRi) function, and wherein the reporter gene encodes a fluorescent protein, an antibiotic resistance protein, beta-galactosidase, and the like, or an endogenous gene. In some instances, the output domain of the kinetically-controlled RNA biosensor construct is or comprises a functional gRNA when folded into an active conformation, and the functional gRNA hybridizes to a target sequence of interest, and wherein the cell is engineered to further express a nuclease that has nuclease function. In some instances, the cell is engineered to modify production of a ligand of interest in the cell. In some instances, the method further comprises subjecting the cell to experimental conditions suspected to modify production of the ligand of interest in the cell. In some instances, the ligand of interest is a compound contacted to the cell or a metabolite thereof.

The disclosure provides a computer-implemented method for designing kinetically-controlled functional RNA molecules. The method comprises:

determining, by a computing device, one or more candidate kinetically-controlled functional RNA sequences:

for each of the one or more candidate kinetically-controlled functional RNA sequences:

predicting, by the computing device, one or more folded structures that the kinetically-controlled functional RNA sequence forms over time; and

determining, by the computing device, one or more metrics for the kinetically-controlled functional RNA sequence based on the predicted one or more folded structures; and

choosing, by the computing device, one or more of the one or more candidate kinetically-controlled functional RNA sequences to be provided for synthesis based on the metrics.

In some instances, determining the one or more metrics for the kinetically-controlled functional RNA sequence based on the predicted one or more folded structures includes at least one of

determining an energy of a predicted folded structure for the kinetically-controlled functional RNA sequence;
comparing an energy of a predicted folded structure for the kinetically-controlled functional RNA sequence to energies of other predicted folded structures for the kinetically-controlled functional RNA sequence: and
determining a barrier energy for converting a predicted folded structure for the kinetically-controlled functional RNA sequence to a target folded structure.

In some instances, predicting one or more folded structures that the kinetically-controlled functional RNA sequence forms over time includes conducting a constraint folding analysis. In some instances, conducting a constraint folding analysis includes:

specifying a predetermined folded structure for a portion of the kinetically-controlled functional RNA sequence; and
predicting an overall folded structure for the kinetically-controlled functional RNA sequence given the predetermined folded structure for the portion of the kinetically-controlled functional RNA sequence.

In some instances, predicting one or more folded structures that the kinetically-controlled functional RNA sequence forms over time includes comparing two or more co-transcriptional folding pathways. In some embodiments, comparing two or more co-transcriptional folding pathways includes:
predicting a first structure for a first incomplete portion of the kinetically-controlled functional RNA sequence while being transcribed:

predicting a second structure for a second incomplete portion of the kinetically-controlled functional RNA sequence while being transcribed, where the second incomplete portion of the kinetically-controlled functional RNA sequence includes the first incomplete portion of the kinetically-controlled functional RNA sequence and one or more additional nucleotides of the kinetically-controlled functional RNA sequence; and
choosing the first structure or the second structure for use in a subsequent structural prediction for a third incomplete portion of the kinetically-controlled functional RNA sequence that includes a subsequent set of one or more additional nucleotides of the kinetically-controlled functional RNA sequence.

In some instances, choosing the first structure or the second structure includes:

determining a barrier energy for converting from the first structure to the second structure;
determining a time for adding the subsequent set of one or more additional nucleotides of the kinetically-controlled functional RNA sequence to the second incomplete portion of the kinetically-controlled functional RNA sequence;
choosing the first structure in response to determining that the barrier energy is too high for the second incomplete portion of the kinetically-controlled functional RNA sequence to transition from the first structure to the second structure during the time for adding the subsequent set of one or more additional nucleotides; and
choosing the second structure in response to determining that the barrier energy is not too high for the second incomplete portion of the kinetically-controlled functional RNA sequence to transition from the first structure to the second structure during the time for adding the subsequent set of one or more additional nucleotides.

In some instances, the kinetically-controlled functional RNA is or comprises a guide RNA (gRNA) molecule. In some instances, the kinetically-controlled functional RNA is or comprises a kinetically-controlled biosensor molecule.

The disclosure provides a non-transitory computer-readable medium having computer-executable instructions stored thereon that, in response to execution by one or more processors of a computing device, cause the computing device to perform actions of a method as described herein.

The disclosure provides a computing device configured to perform actions of a method as described herein.

## DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

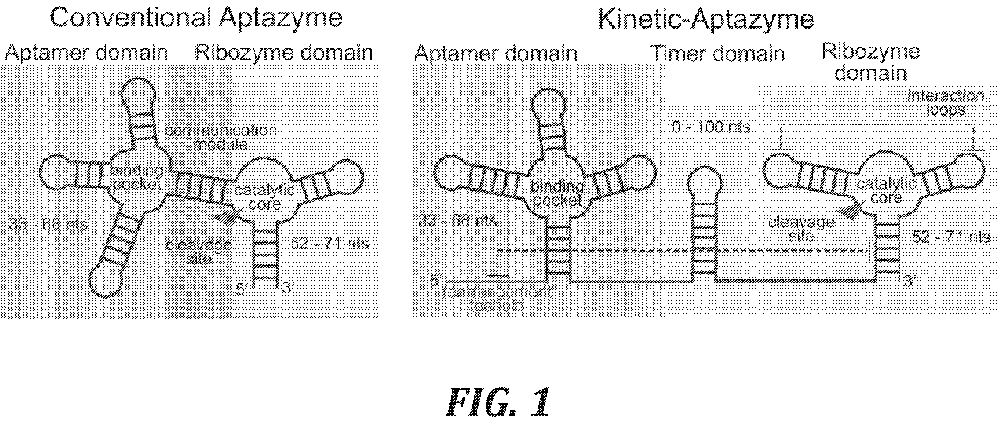

FIGURE 1 schematically illustrates a comparison of the conventional strategy for assembling aptazymes with the novel kinetically-controlled biosensor molecular architecture, applied for certain embodiments of the disclosure. Instead of encoding structural transitions in a hybrid stem (communication module), the kinetically-controlled biosensor architecture allows transitions to be encoded in the intervening sequence, preserving parental part performance, and enabling the possibility of co-transcriptionally decoupling the ligand-binding and cleavage activities.

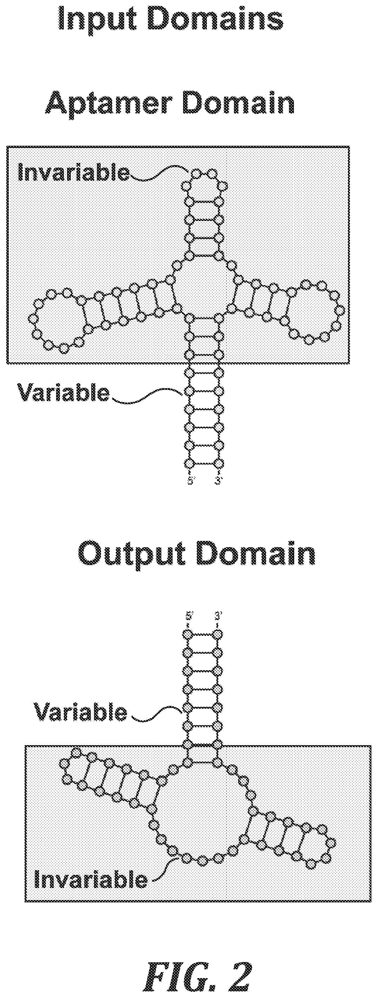

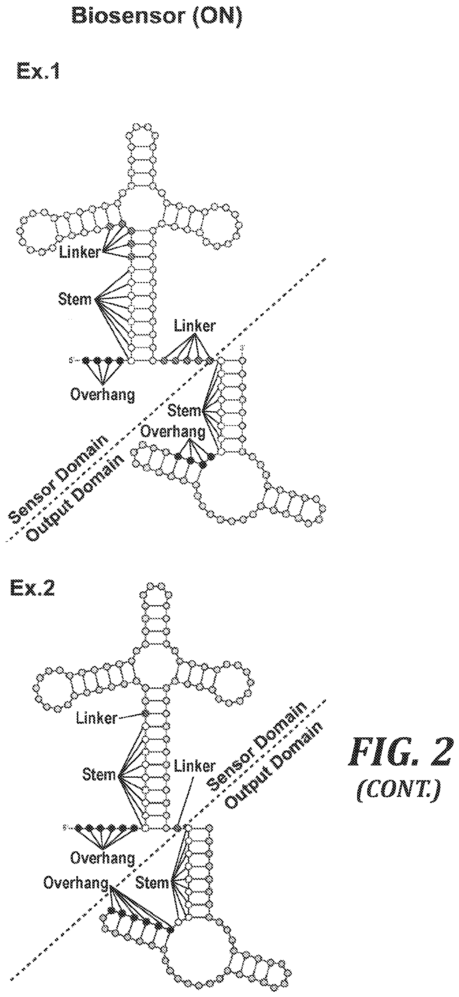

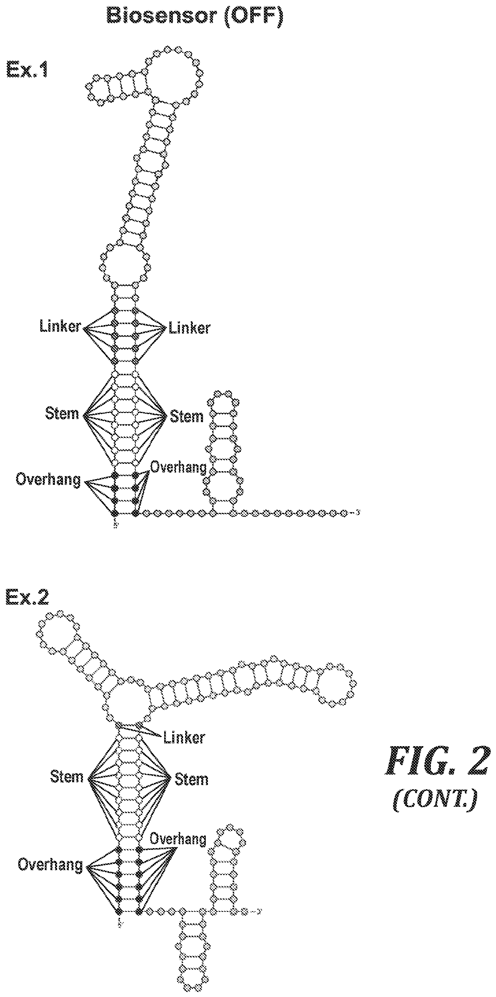

FIGURE 2 is a schematic illustration of an embodiment of the kinetically-controlled RNA biosensor construct disclosed herein. The RNA biosensor construct generally has a Sensor domain (i.e., an Input domain), which itself comprises an aptamer domain sequence for binding a target ligand, and an Output domain that results in a measurable signal depending on the ligand-binding status of the target ligand to the aptamer that is transcribed before the Output domain. The length and sequence of the closing stem can generally be varied, so long as Watson-Crick base-pairing is maintained. The internal regions that possess more complex structures and activities are generally invariable in order to maintain function. "Ex. 1" and "Ex. 2" illustrate exemplary biosensors possessing the same input domains, but different lengths of Overhang. Stem, and Linker. Each of "Ex. 1" and "Ex. 2" are illustrated in ON (active conformation for modulation of detectable output signal) or OFF (inactive conformation for lack of modulation of detectable output signal). The Overhang, Stem, and Linker sequences are both shaded and labeled as such, while the unlabeled regions of the same shade are the reverse-complement of that region.

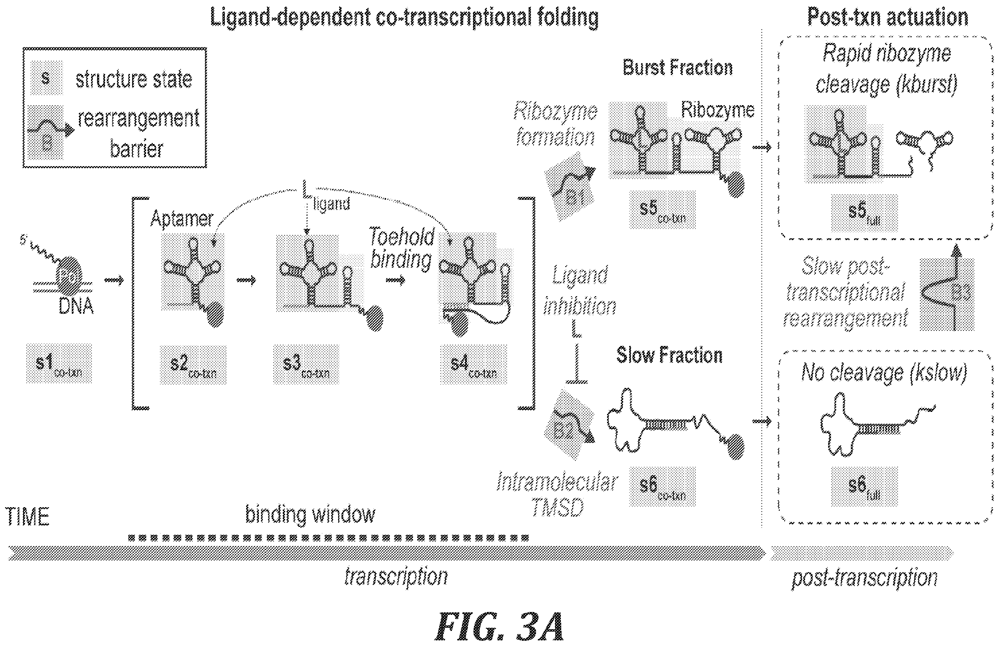

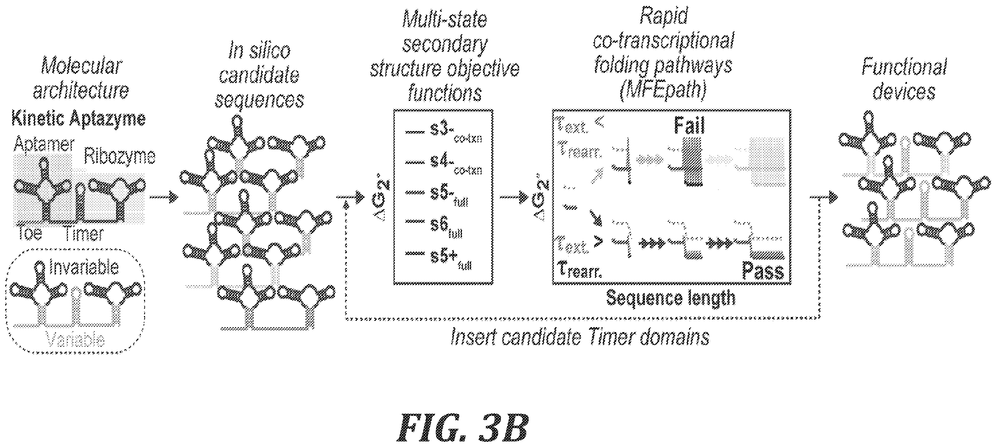

FIGURES 3A and 3B schematically illustrate biosensors for engineering kinetically-controlled binding and actuation. 3A) Kinetically-controlled biosensors are RNA biomolecules actively transcribed in the presence of a RNA polymerase enzyme to sense a target ligand. Moreover, the fate of a kinetically-controlled biosensor is decided co-transcriptionally. Kinetic aptazymes (K-A) are one exemplary embodiment of kinetically-controlled biosensors presented for illustration purposes. After the aptamer domain is transcribed, if no ligand is present, the toehold binds its target at the 5' end of the ribozyme domain, and the kinetically-controlled biosensor undergoes rapid, intramolecular toehold-mediated strand displacement into an inactive conformation (B2). In the presence of ligand, the aptamer is stabilized, allowing the K-A to fold rapidly into the catalytically-active structure ensemble (B1). Large predicted B3 barriers should result in slow structural interconversion from the S6 to the S5 state post-transcriptionally therefore very low background cleavage. 3B) In order to identify co-transcriptionally-functioning switches a molecular architecture must first be chosen that defines the variable and constant regions. Next, candidate sequences are evaluated using thermodynamic objective functions. Then, surviving sequences are analyzed for rapid co-transcriptional folding, using a novel dead end elimination algorithm that efficiently ignores sequences that fall into significant kinetic traps (MFEpath). Optionally, candidate Timer domains are introduced into functional devices, and reevaluated using the thermodynamic and co-transcriptional objective functions.

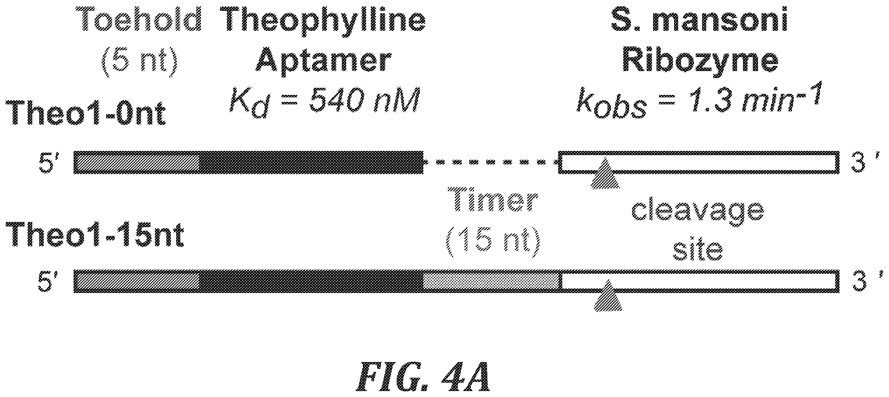

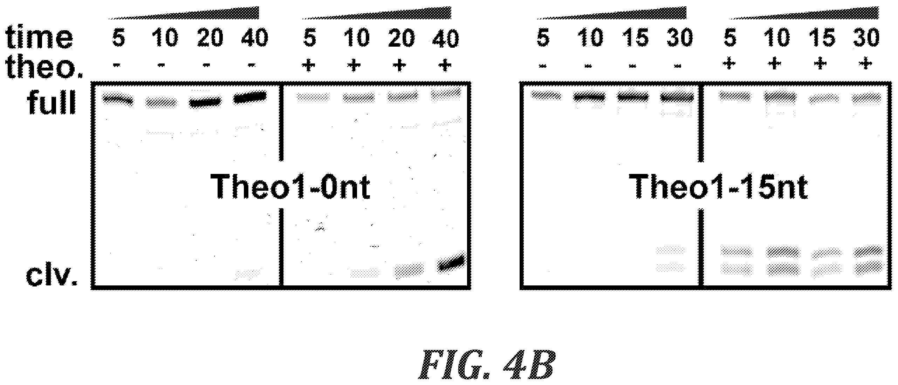

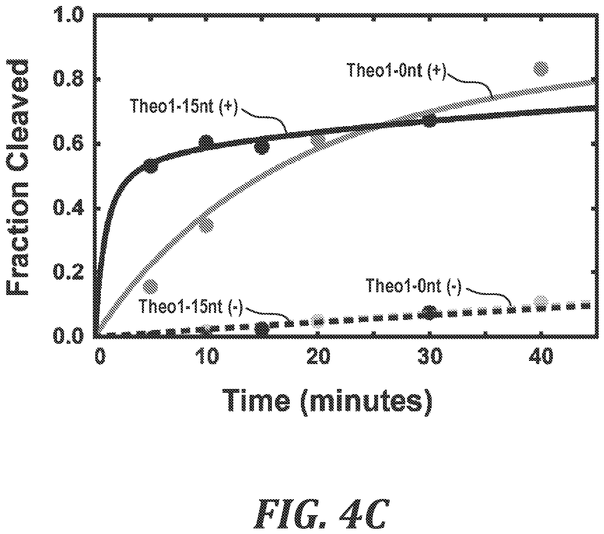

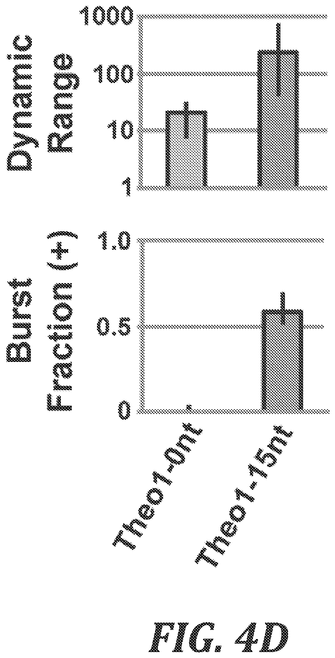

FIGURES 4A-4E illustrate that the Timer domain enables rapid, biphasic ligand-dependent function. 4A-4D) A theophylline-responsive kinetically-controlled biosensor designed with 0-nt Timer exhibits slow, monophasic, ligand-dependent in vitro co-transcriptional cleavage (Theo1-0nt). The addition of a minimal 15-nt Timer results in a kinetically-controlled biosensor exhibiting rapid, biphasic, ligand-dependent cleavage and a dynamic range ($k_{obs}^{+}/k_{obs}^{-}$) of 237. 4E) For the 20 kinetically-controlled biosensors with low background (UBF< 0. 1, $k_{avg}^{-}$ < 0.01 min-1), a Timer domain is necessary to access ligand-dependent burst phase cleavage.

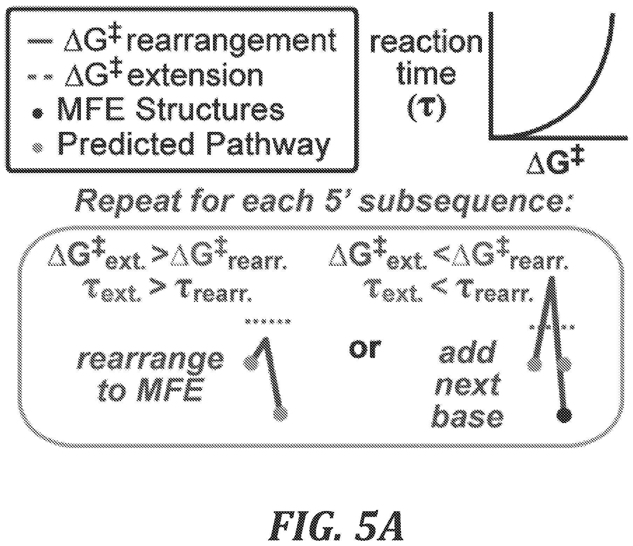

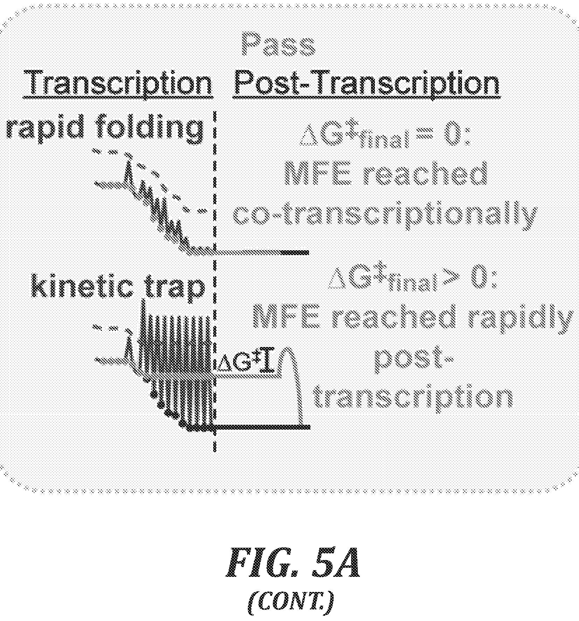

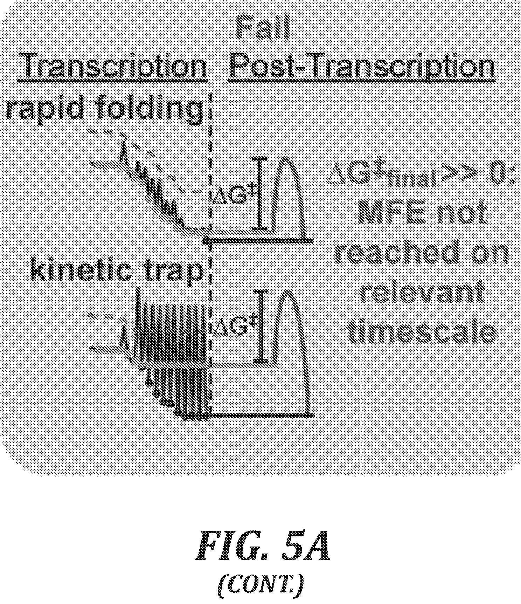

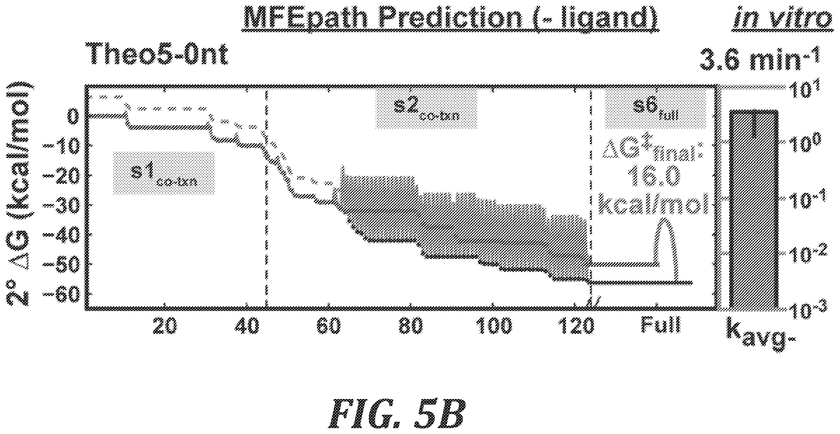

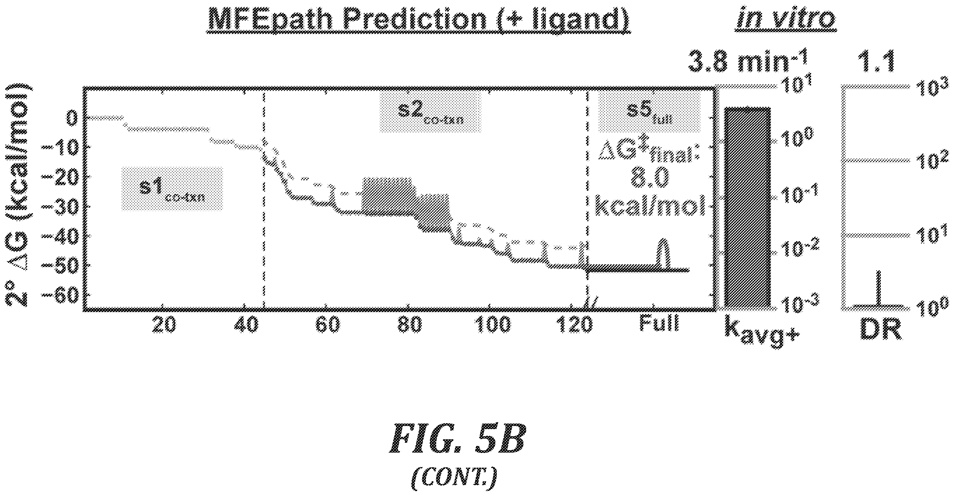

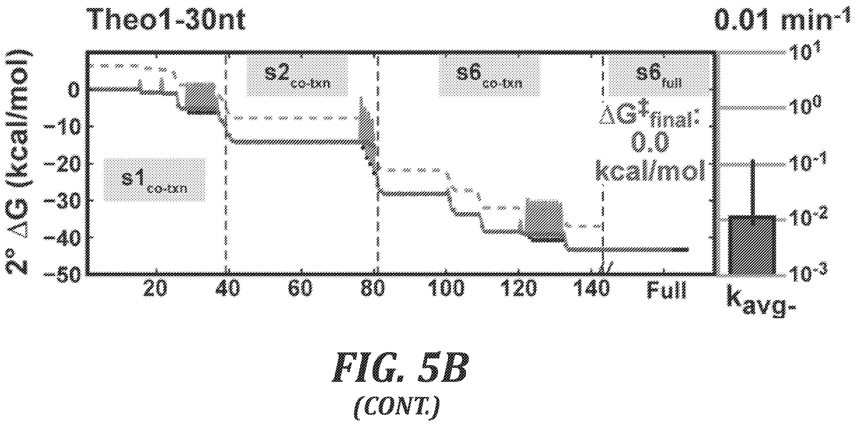

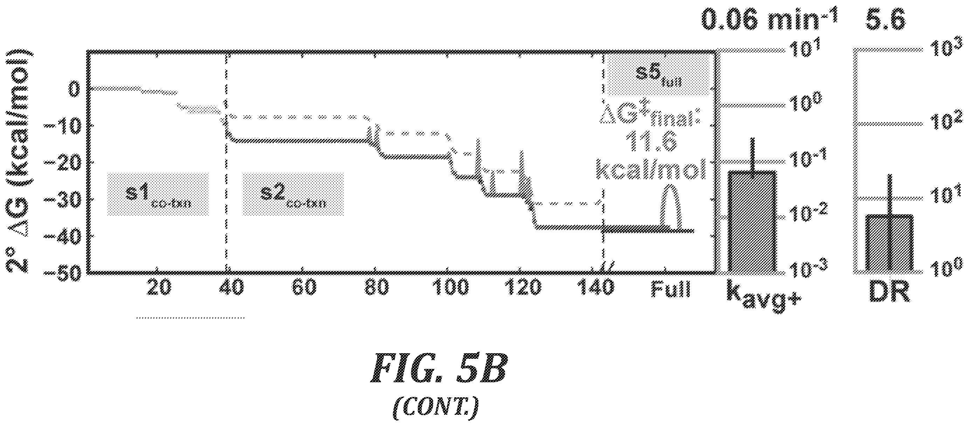

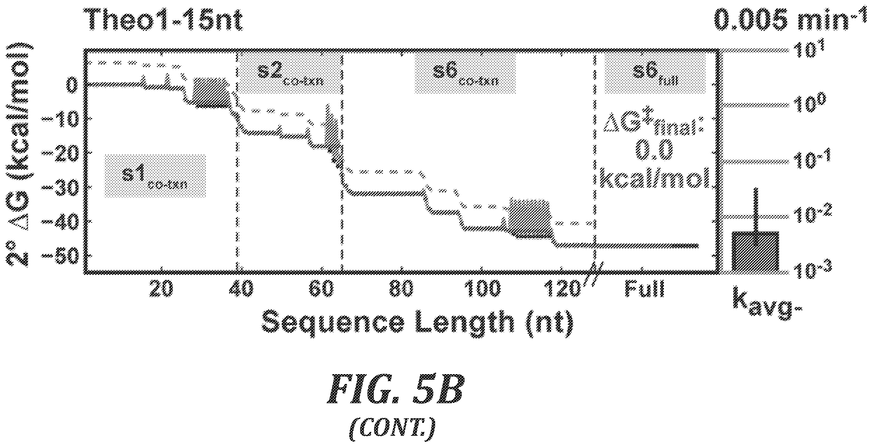

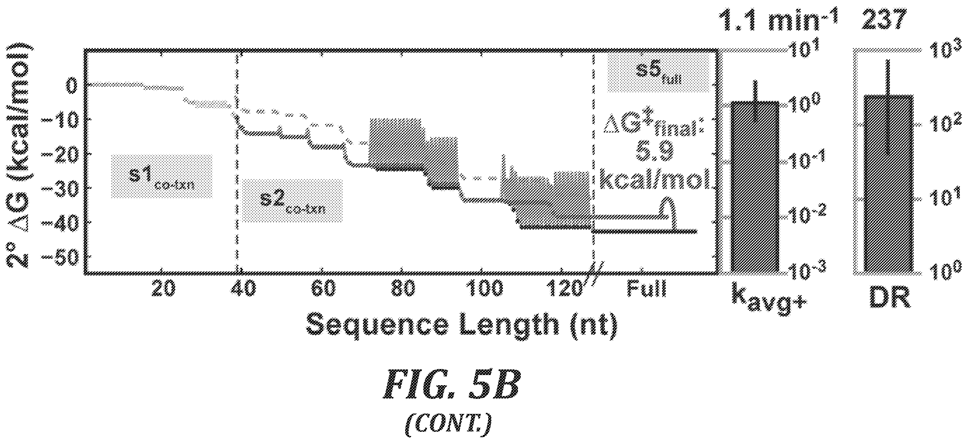

FIGURES 5A and 5B illustrate the reliable multi-state co-transcriptional folding design enables kinetically-controlled biosensor engineering. 5A) In order to predict coarse-grained folding trajectories, MFEpath predicts the time to rearrange to the next Minimum Free Energy (MFE) substructure from Arrhenius-like interconversion barriers. If rearrangement is expected to be faster than the addition of the next nucleotide, structural rearrangement is allowed. If slower, the transition is disallowed, and the next base is added, and the analysis is performed for the new substructures. After the final base is added, the last barrier height is used to predict the time needed for the kinetically-controlled biosensor to convert from the co-transcriptional structure to the post-transcriptional MFE structure. 5B) Selected kinetically-controlled biosensors demonstrate the significance of the final output barrier. In the absence of ligand, a low final barrier indicates a co-transcriptional interconversion to the OFF state, resulting in low background cleavage. A high barrier results in rapid cleavage and a loss of potential DR. In the presence of ligand, a high barrier indicates that the cleavage rate is limited by structural interconversion. A low barrier indicates that the interconversion occurs on a faster timescale than cleavage, and the intrinsic cleavage rate of the ribozyme becomes limiting. When both barriers are low, very large dynamic ranges become possible.

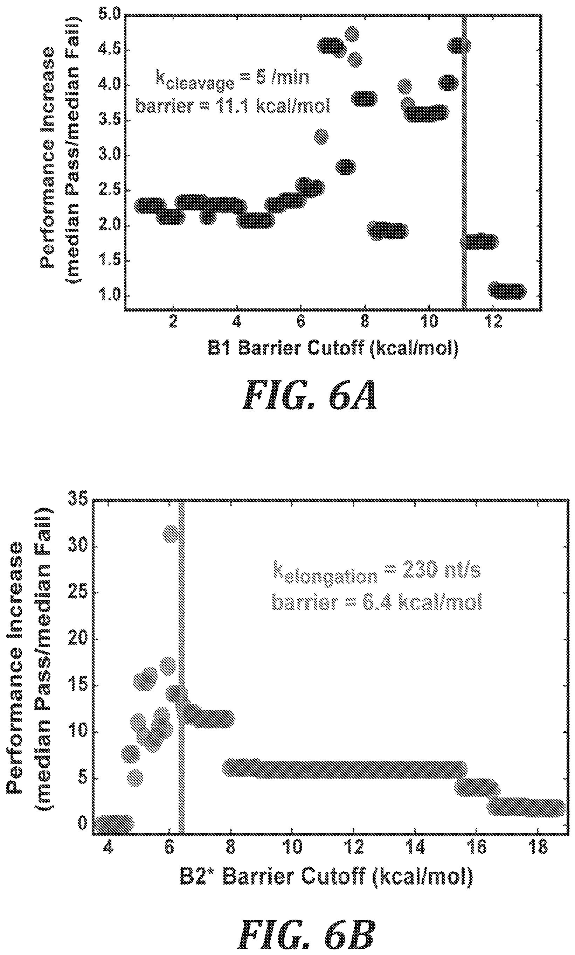

FIGURES 6A-6D illustrate the thresholds for structural rearrangement. 6A) Performance increase (the ratio of the median cleavage rate ($k_{avg}$ +) for devices that pass the threshold, to the median of those that fail) is at a maximum at our predicted value. implying our a priori predicted barrier height (11.1 kcal/mol) is ideal for screening kinetically-controlled biosensors. The vertical line represents the predicted barrier of the rate of nucleotide addition. 6B) Performance increase was defined as the inverse of part A, with respect to induced cleavage ($k_{avg}$ -). The maximum

is near our implemented elongation barrier (6.4 kcal/mol), though slightly below. This suggests the estimate is good, but that there may be additional factors to consider. 6C) Predicted values for the B2* of aptamer deformation partially explain observed uninduced burst fraction (UBF). 6D) Kinetically-controlled biosensors with Timers and low background (UBF< 0.1, kavg- < 0.01 min-1) only display significant burst phase kinetics when B1 is below the predicted threshold.

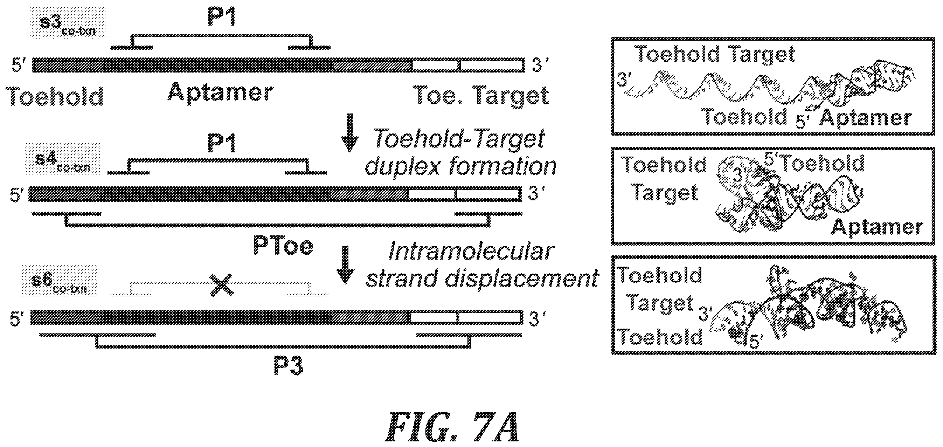

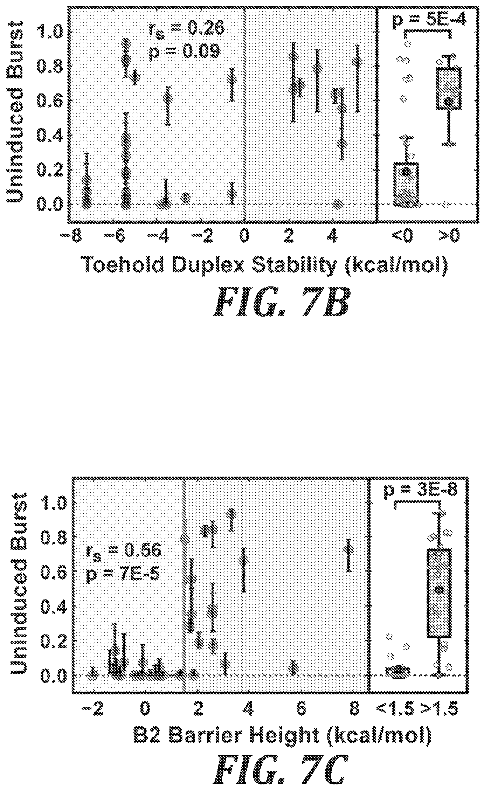

FIGURES 7A-7E illustrate that intramolecular toehold-mediated strand displacement (TMSD) reduces leak. 7A) Cartoon mechanism for intramolecular TMSD. 7B) Devices predicted to form stable toehold-target duplexes display significantly reduced uninduced burst fraction (UBF) relative to those not expected to form favorable contacts (not shown). 7C) The B2 barrier, which combines the barrier heights of all possible transitions from ON to OFF pathways after the branch point, displays a threshold below which devices have UBFs below 0.1. 7D) Representative 3-dimensional predictions of the toe-target distances for identical kinetically-controlled biosensors containing Timer domains with different amounts of predicted structure. Structures generated from MFEpath secondary -structures and 3-D structures produced by RNAcomposer (Purzycka. K. J. et al. Chapter One - Automated 3D RNA Structure Prediction Using the RNAComposer Method for Riboswitches, in Methods in Enzymology (eds. Chen. S.-J. & Burke-Aguero, D. H.) vol. 553 3-34 (Academic Press, 2015)).

7E) For devices below the threshold in part D. devices with small predicted toe-target distance display lower UBFs than those with larger predicted distances. 3-D images produced using PyMol.

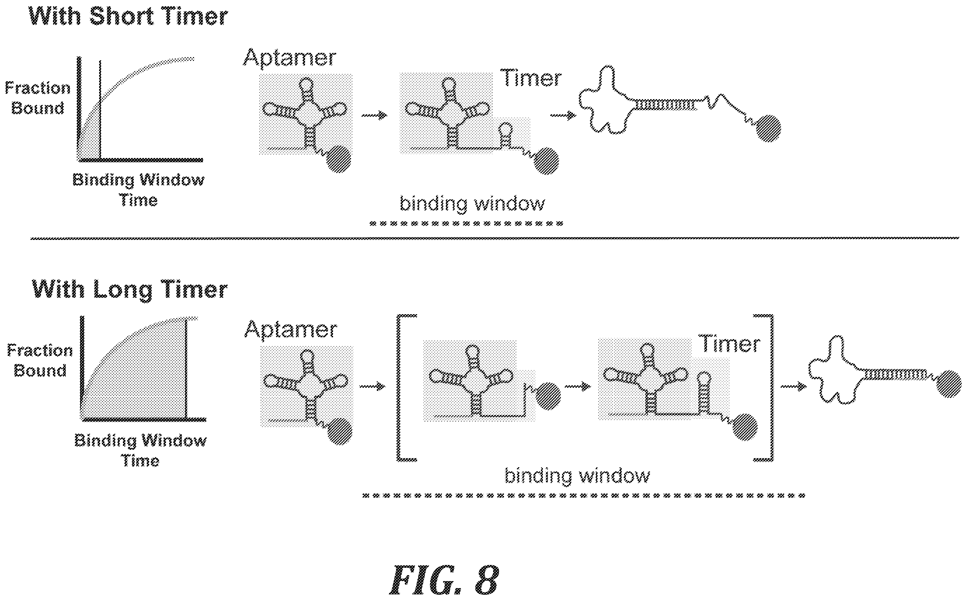

FIGURE 8 schematically illustrates that Timer domains increase the ligand binding window. As the length of the Timer domain increases, so too does the time it takes to transcribe the kinetically-controlled biosensor. This results in a greater fraction of the aptamers bound when the binding window closes, and therefore increased sensitivity to the target molecule

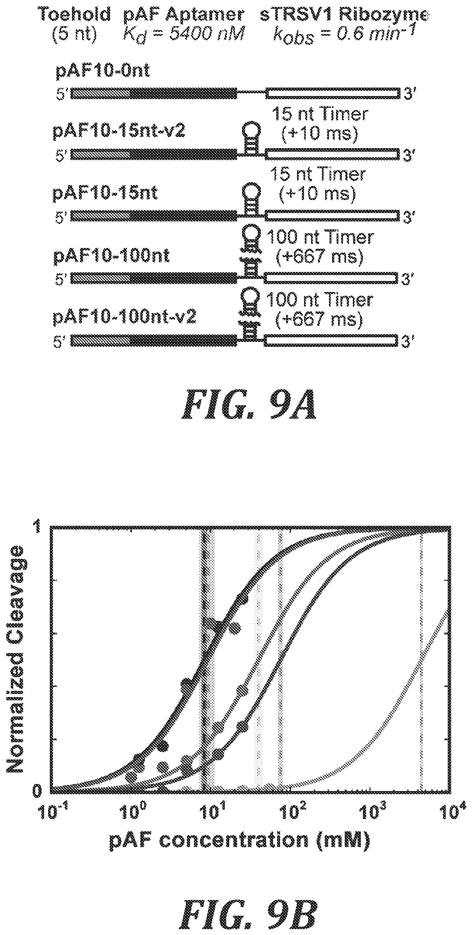

FIGURES 9A-9C illustrate that longer Timer domains increase the ligand sensitivity of kinetically-controlled biosensors. 9A) Five functional (DR > 9) kinetically-controlled biosensors were designed from one parental device (pAF10-0nt), varying only in the length and sequence of the Timer domain. 9B) Normalized output vs. ligand concentration is fit to a standard 1:1 binding curve. $EC_{50}$ values, indicated by vertical lines, decrease as Timer length increases. 9C) Fit and predicted $EC_{50}$ values for the five devices. Error bars represent the standard deviation of the fits shown in 9B). Predicted $EC_{50}$ values, derived from known rate constants and the length of the Timer domain agree well with observed values

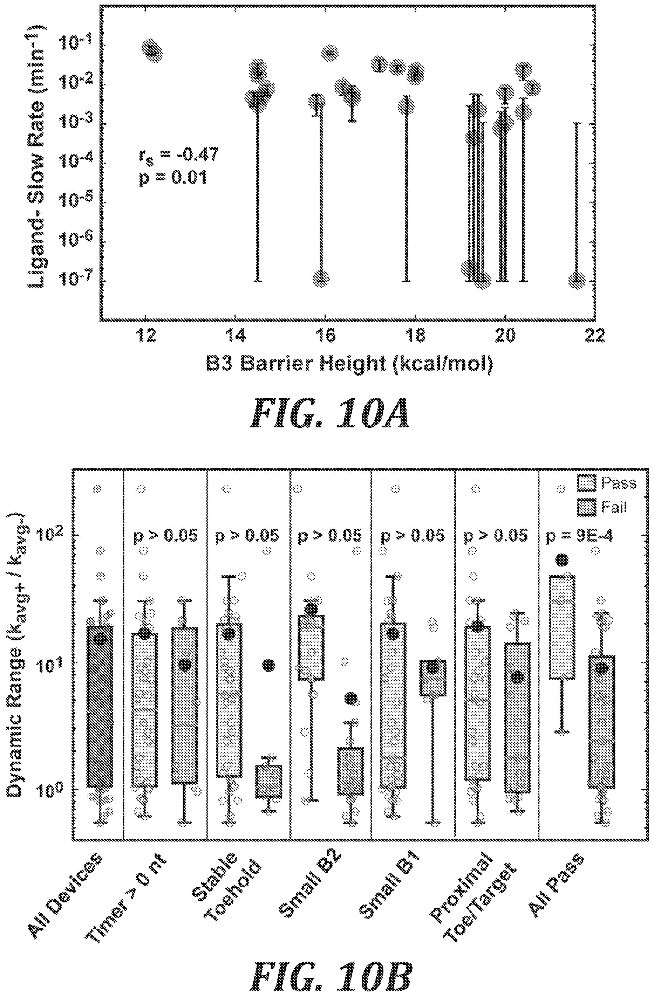

FIGURES 10A and 10B graphically illustrate that computational screening metrics predict kinetically-controlled biosensor activity. 10A) Ligand independent slow rate decreases as predicted B3 height increases. Error bars represent bootstrapped 95% confidence intervals of the fit in vitro data. Rate constants below $10^{-3}$ min$^{-1}$ cannot be determined with statistical significance, limiting prediction of extremely low rates. 10B) Impact of kinetically-controlled biosensor screening steps on the performance of the resulting populations. Red lines represent the median of the population, while green circles represent the means. Although all screening steps improve the mean dynamic range of the surviving sequences, p-values are > 0.05 for all means except when all screening steps are implemented (p = 0.02).

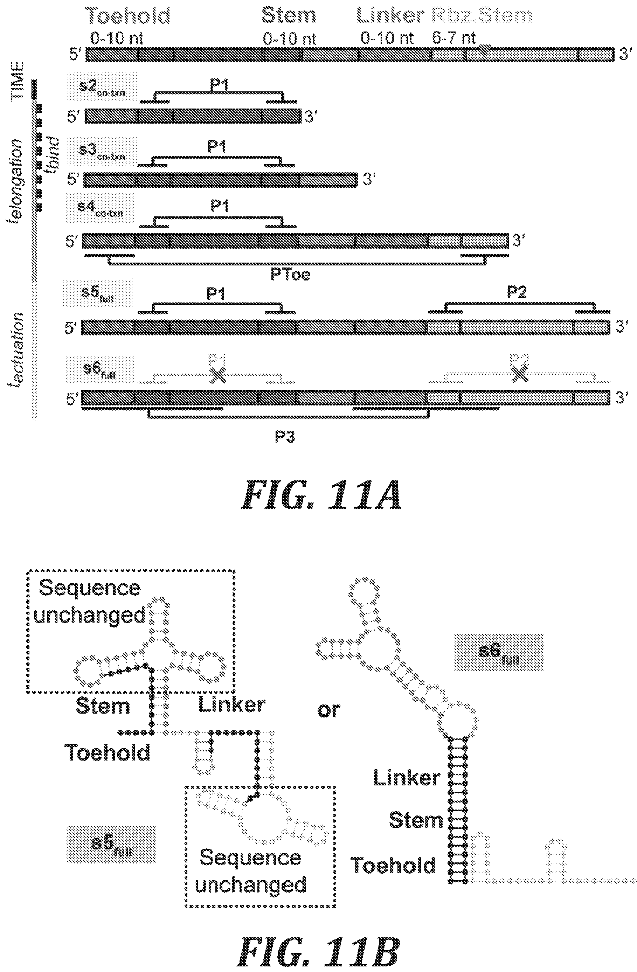

FIGURES 11A and 11B schematically illustrate the structure states analyzed in the screening of kinetically-controlled biosensor candidates. 11A) Analyzed during the thermodynamic stage of kinetically-controlled biosensor (e.g., K-A) design. Sub-sequences are screened for their ability to stably form target structure states, mimicking co-transcriptional folding. 11B) The three regions of complementary sequence (toehold, linker, and stem) utilized in the design process to create a selectively-inactive MFE structure.

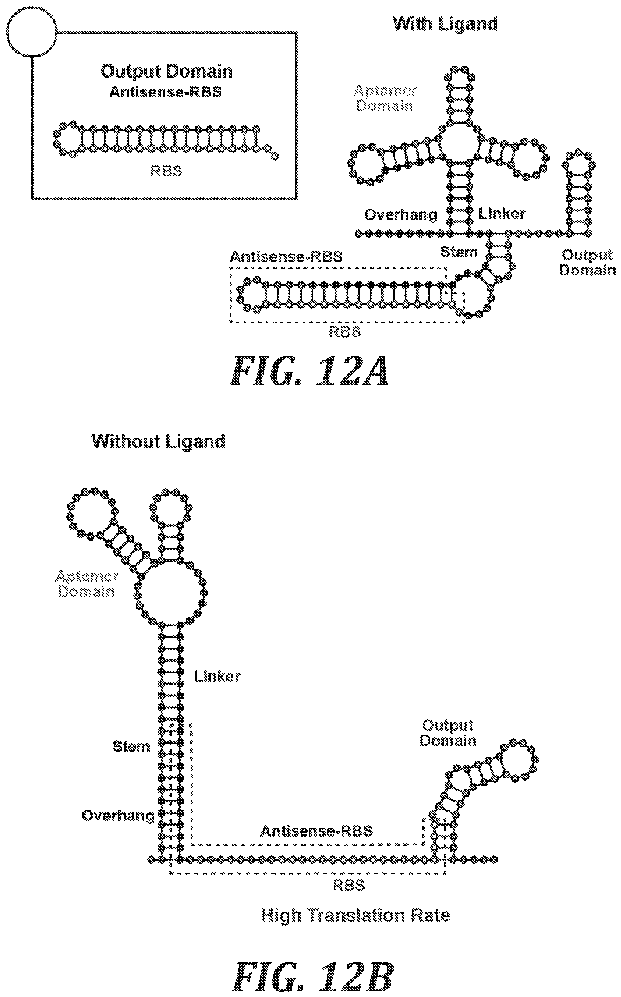

FIGURE 12A-12C schematically illustrate an AS-RBS riboswitch kinetically-controlled biosensor. 12A) In order to make a ribosome binding siteRBS) suitable for implementation within our kinetically-controlled biosensor molecular architecture, the RBS is combined with a 5' antisense sequence. 12B) When incorporated into a candidate AS-RBS riboswitch, it results in high translation in the absence of the target molecule, and low translation when the target molecule is bound. 12C) Incorporating a transcriptional pause into the Timer domain increases the duration of the binding window dramatically, resulting in increased sensitivity to the target molecule.

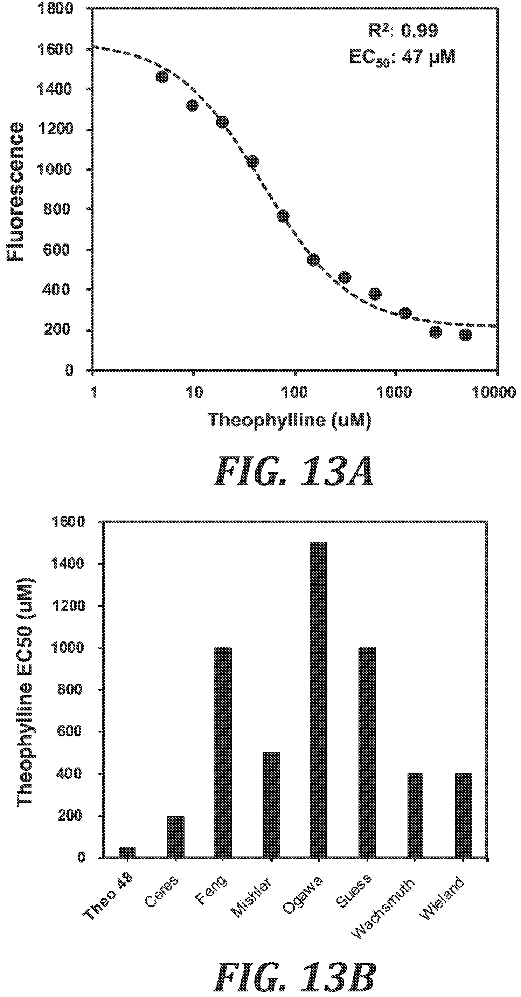

FIGURES 13A and 13B graphically illustrate the characterization of a high-sensitivity AS-RBS riboswitch kinetically-controlled biosensor. 13A) The Theo-48 AS-RBS riboswitch possesses a significant fold-change in response, which means that at least 90% of all transcribed biosensors are able to bind to the target molecule. In addition, it possesses an extremely low $EC_{50}$. 13B) The $EC_{50}$ of Theo-48 is substantially lower than all other cis-acting theophylline-responsive riboswitches in bacteria. It is noted that the $EC_{50}$ values were not explicitly presented for the riboswitches identified as Feng, Mishler, Ogawa, Suess, Wachsmuth, and Wieland, but rather was estimated from presented titration data.

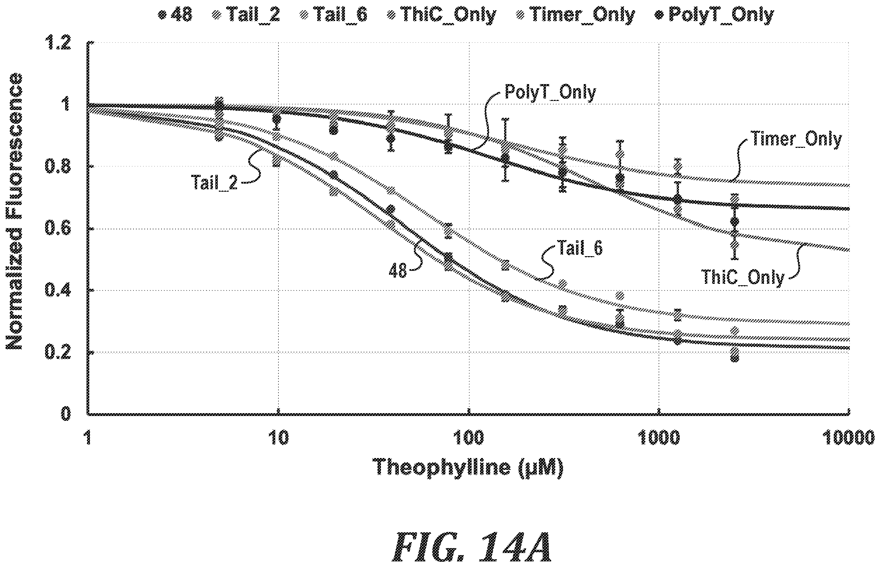

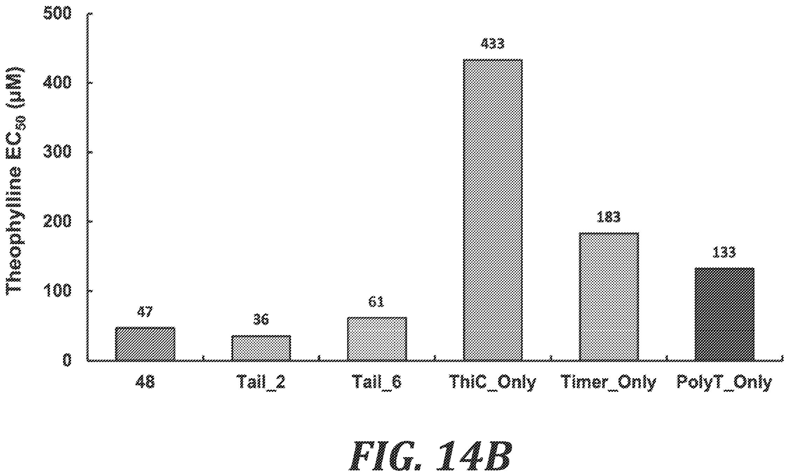

FIGURES 14A and 14B graphically illustrate the pause sequence and context are critical for pause site activity. 14A) Modifying a pause-containing Timer domain results in reductions in activation ratio and sensitivity to the target molecule. 14B) EC50 values increase for all mutations or deletions. Interestingly this is especially true for ThiC_Only, which contains the pause site, but none of the flanking sequence. This suggests that folding context is critical for effective transcriptional pausing.

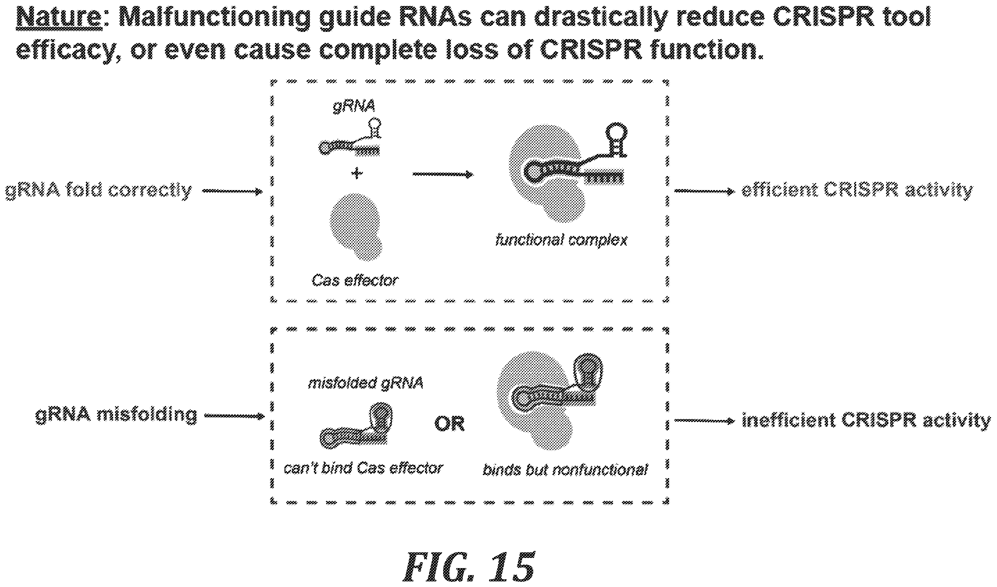

FIGURE 15 schematically illustrates the functional outcome of CRISPR activity based on proper folding versus misfolding of the guide RNAs (gRNA). Malfunctioning of the guide RNA can drastically reduce or prevent altogether CRISPR activity. Misfolding results from interactions between the ~20 base target sequence unique to each guide and the rest of the gRNA construct.

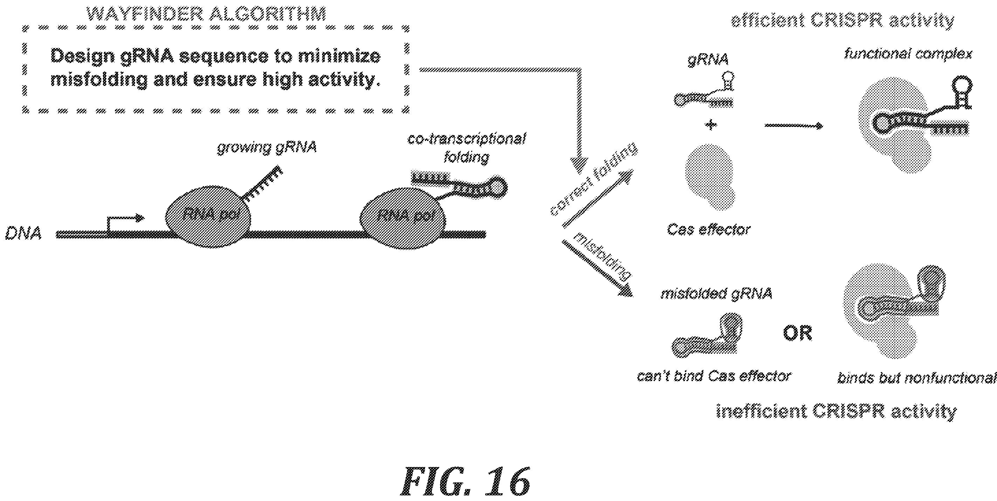

FIGURE 16 schematically illustrates application of the Wayfinder algorithm to predict and ensure accurate folding and, thus, high activity, of the expressed scRNA constructs.

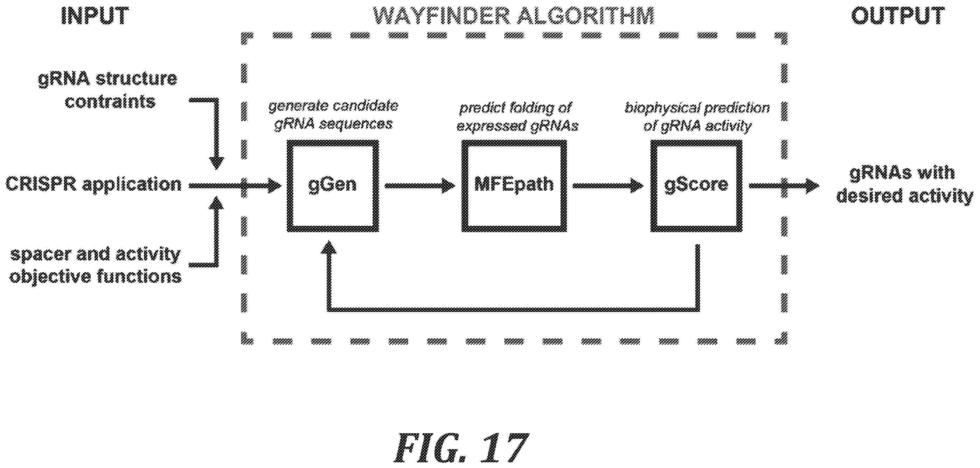

FIGURE 17 schematically illustrates an implementation of the Way finder algorithm to process input data including gRNA structure constraints. CRISPR application considerations, and spacer/activity objective functions to produce a prediction of gRNAs with desired activity.

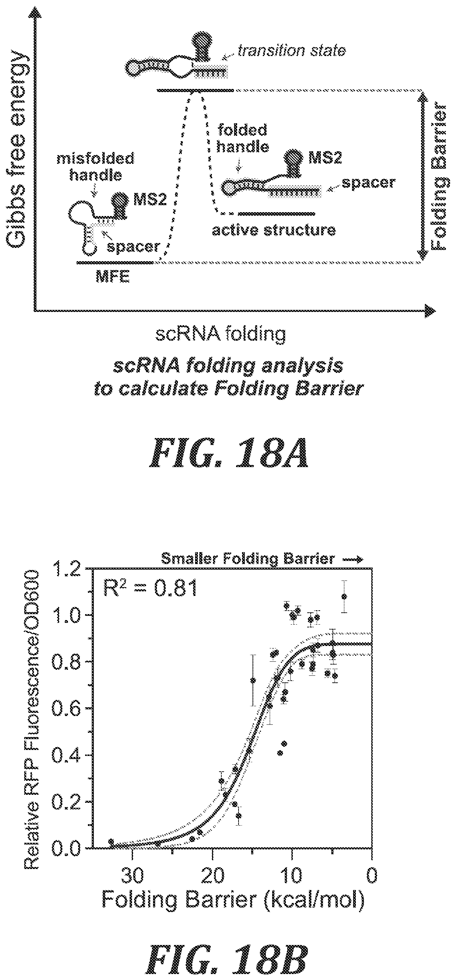

FIGURES 18A and 18B illustrate that the folding barrier predicts impact of spacer sequence on CRISPRa. 18A) As the folding barrier gets smaller, scRNAs can more rapidly fold into the Cas9-competent conformation, where they are able to participate in complex formation. 18B) 30 scRNAs were tested in which their 20 base spacer sequence were varied. The computationally-predicted folding barrier height accurately predicts scRNA activity. Dots representing scRNAs with smaller folding barriers were chosen to engineer synthetic CRISPRa promoters for subsequent applications.

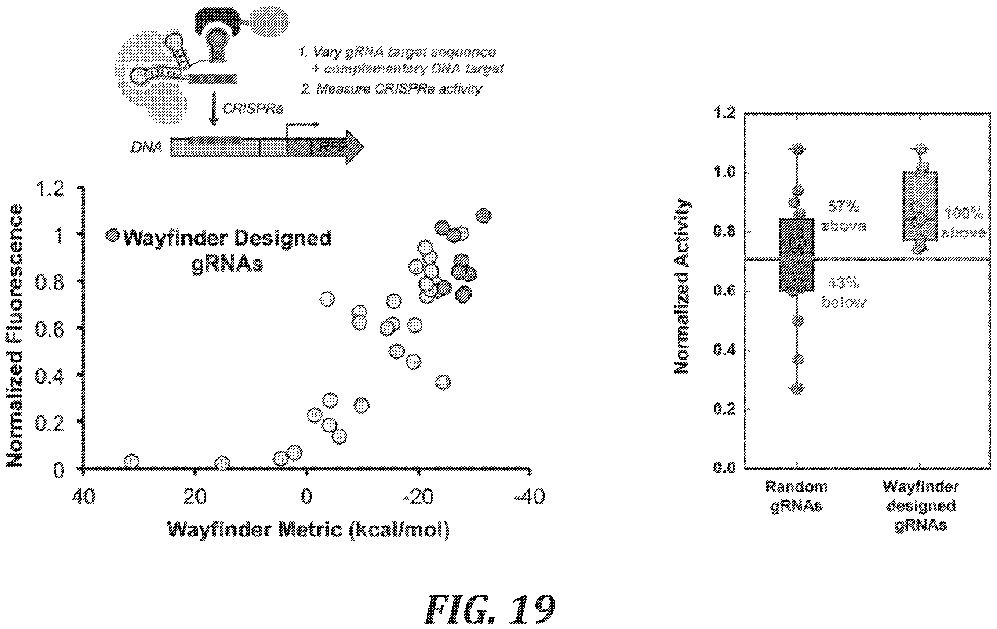

FIGURE 19 schematically and graphically illustrates an assay to test the ability of Way finder to predict functionality of gRNA with scRNA constructs. The assay design was used to generate the data illustrated in FIGURES 18A and 18B. The Wayfinder designed gRNAs exhibited significantly and consistently elevated activity versus constructs with random gRNA sequences.

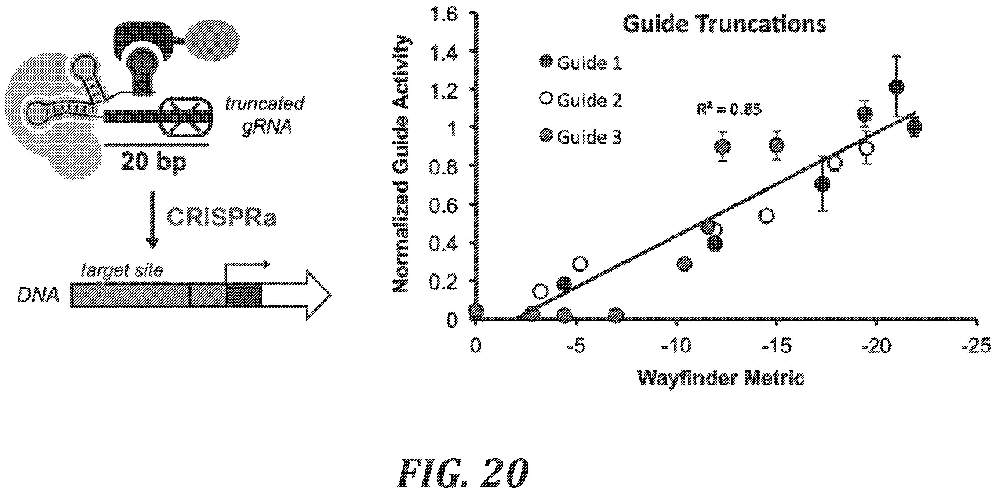

FIGURE 20 graphically illustrates that the Wayfinder algorithm can predict the activity of truncated scRNAs. Truncations of the 5' end of the spacer sequence result in decreased CRISPRa levels, by decreasing the stability of the CRISPRa complex binding to its target DNA (see cartoon depiction in left panel). By incorporating the net binding energy of the RNA binding to its target with the kinetic barrier, the Wayfinder algorithm is able to predict the activity of scRNAs with truncated spacer sequences

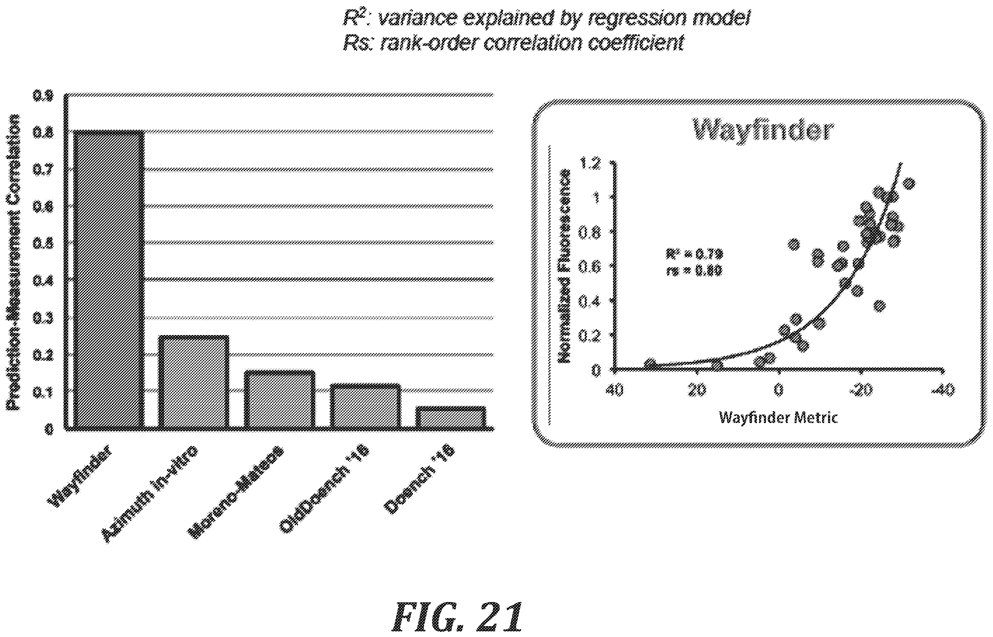

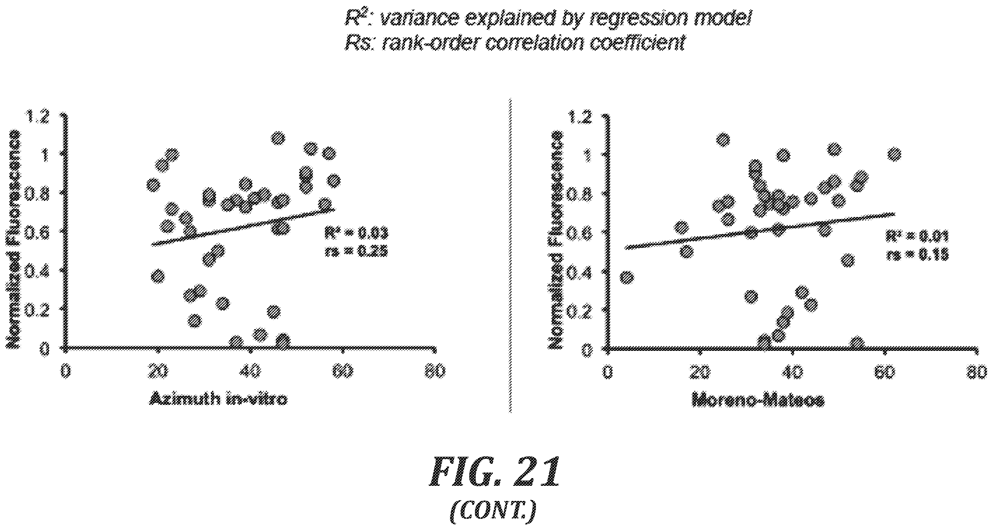

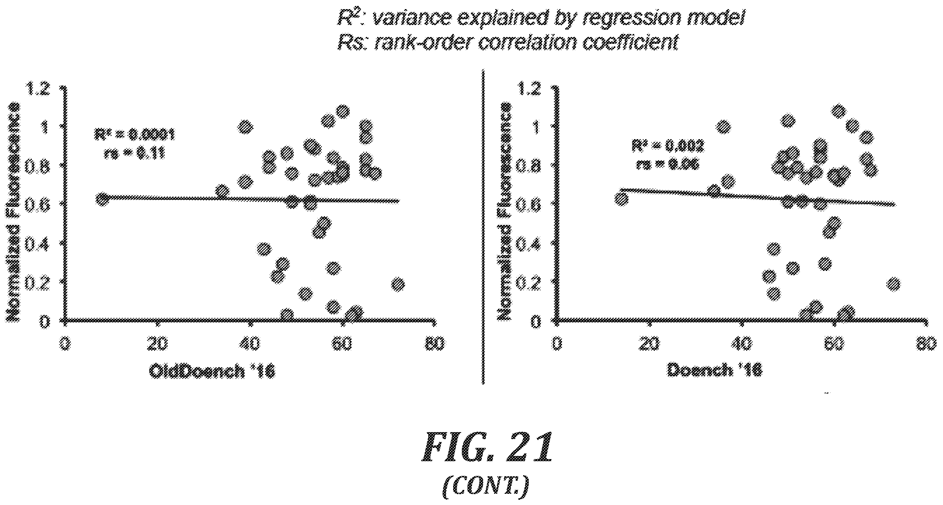

FIGURE 21 illustrates the comparison of Wayfinder algorithm to other common guide prediction tools. Wayfinder significantly outperforms the other common guide RNA activity prediction tools when applied to our CRISPRa dataset. Most other tools are trained on large eukaryotic gene editing datasets.

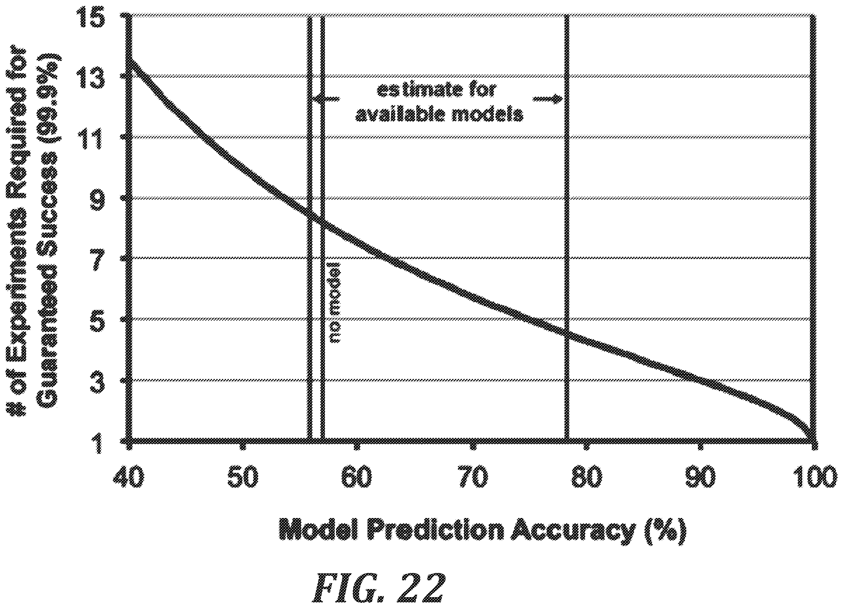

FIGURE 22 graphically illustrates the gained efficiency provided by Wayfinder-based predictions resulting from higher predictive quality and reduced validation experimentation to result in accurate implementation of CRISPR applications.

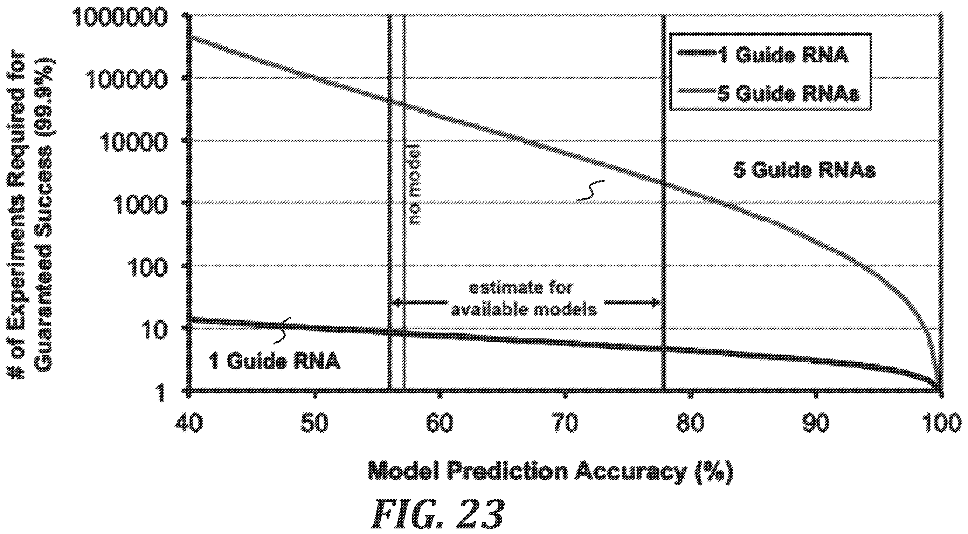

FIGURE 23 illustrates that accurate model predictions enable forward-engineering of complex systems. In order to forward-engineer complex systems in which multiple novel scRNAs are required to function without prior validation, highly accurate predictions become necessary. Wayfinder provides significantly enhanced predictive power so as to drastically reduce the experimentation required in multiplexed applications.

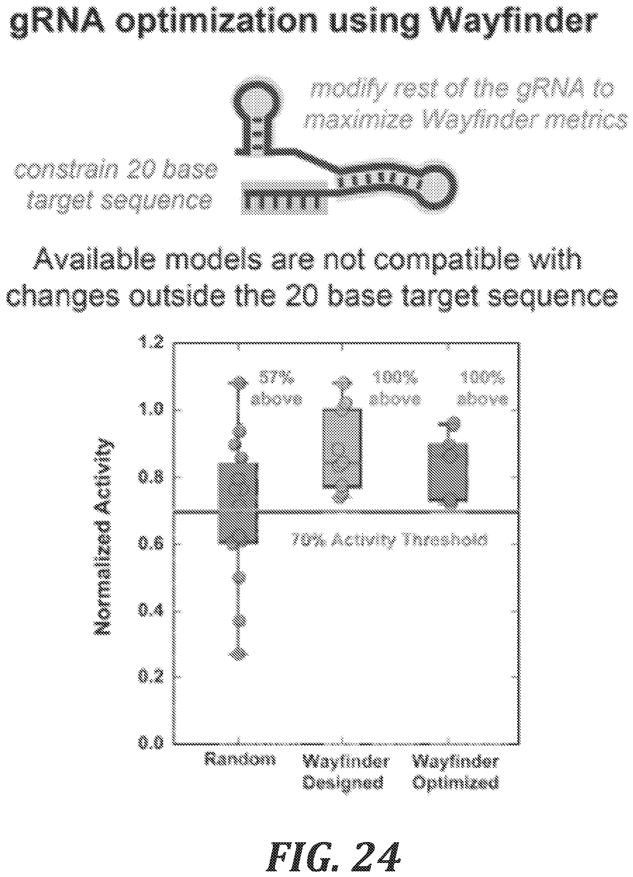

FIGURE 24 schematically and graphically illustrates that accurate model predictions enable forward-engineering of complex systems, similar to FIGURE 19. Here, scRNAs that were also optimized using the Wayfinder algorithm, in addition those initially screened or designed by Wayfinder, show consistently high CRISPRa activity. In comparison, random scRNAs frequently show activity falling below a 70% activity threshold.

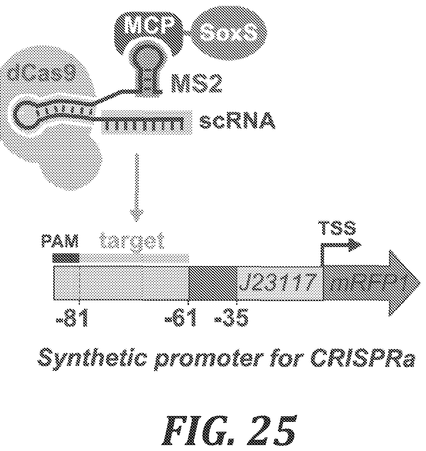

FIGURE 25 is a schematic illustration of an embodiments of a CRISPRa target, which comprises a gene expression cassette with a promoter that is optimized for targeting by an scRNA construct. An scRNA construct is a gRNA modified by adding a MS2 aptamer at its 3' end. Upon binding of the CRISPRa complex, the promoter is activated to initiate transcription, in this case of the reporter gene *mRFP1.*

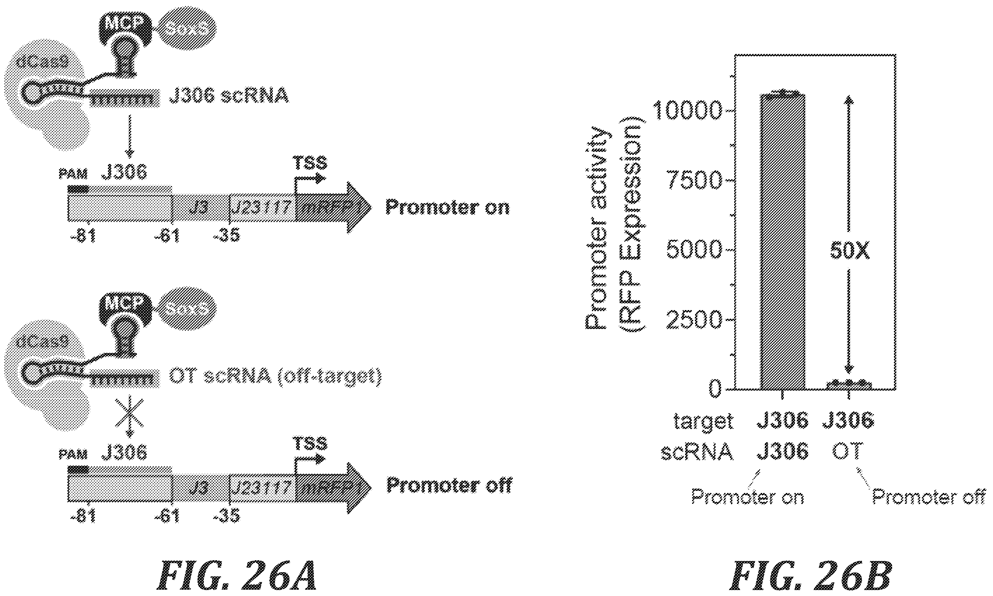

FIGURES 26A and 26B illustrate that guide RNA in scRNA constructs and corresponding sequence engineered into a target expression cassette result in highly specific induction of gene expression. 26A) Schematic illustration of assay design. 26B) graphical representation of on-target CRISPRa versus off-target CRISPRa resulting in specific induction of gene expression.

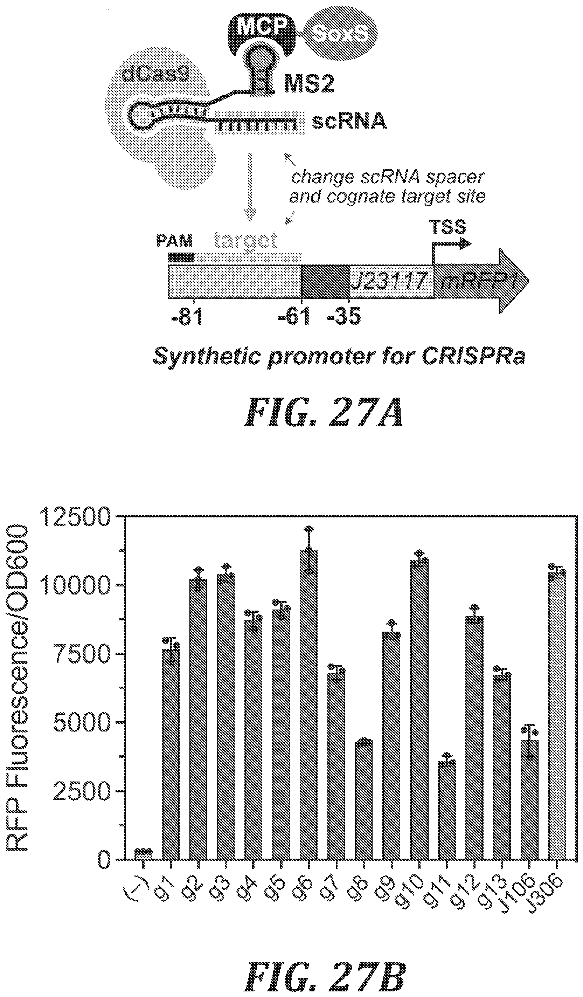

FIGURES 27A and 27B illustrate that CRISPRa promoter regions can be varied, i.e., programmed, to be responsive only to corresponding scRNA constructs, by changing the sequence of the scRNA target site.

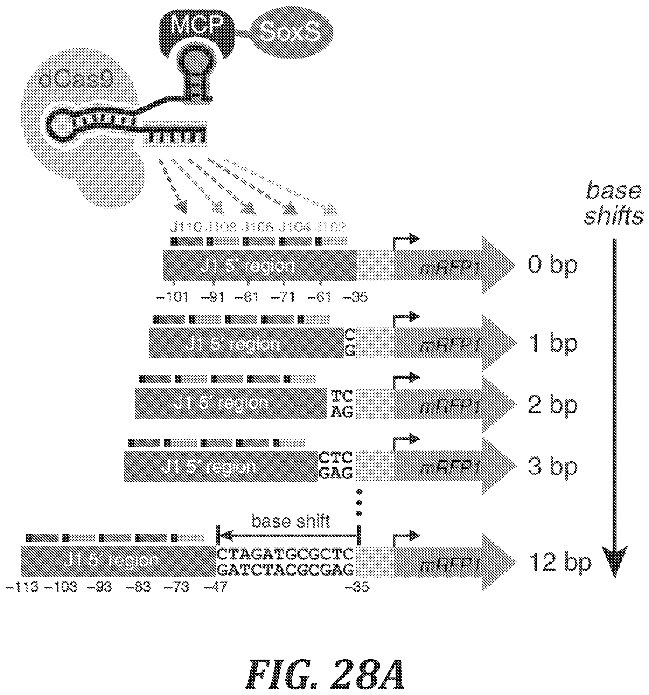

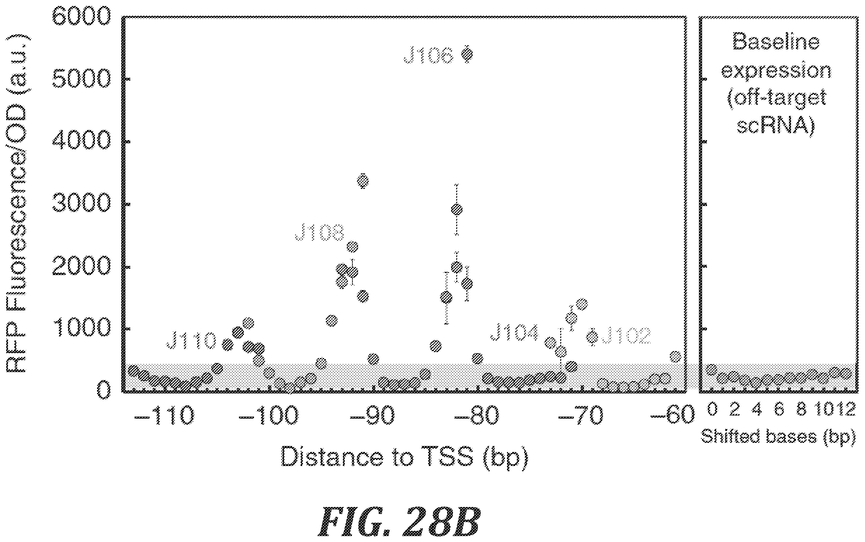

FIGURES 28A and 28B illustrate that CRISPRa is sensitive to the precise position of the scRNA target site. 28A) CRISPRa displays periodic positioning dependence with peak activities every 10-11 bases between -60 and -100 from the transcription start site (TSS). Reporter genes were constructed by inserting 0-12 bases upstream of the -35 region of the J1-J231 17-mRFP1 reporter Five scRNA sites (J102, J104, J106, J108, J110) with positions -61, -71, -81, -91, -101 from the TSS on the non-template strand of the original promoter were targeted. In this way, the complete -61 to -113 region can be covered at single base resolution. The color coding (shading) indicates data for the same target site shifted across a 12 base window. 28B) The baseline expression of reporters with shifted bases when an off-target scRNA was used (J206). The gray area represents the range of the baselines among the reporter series.

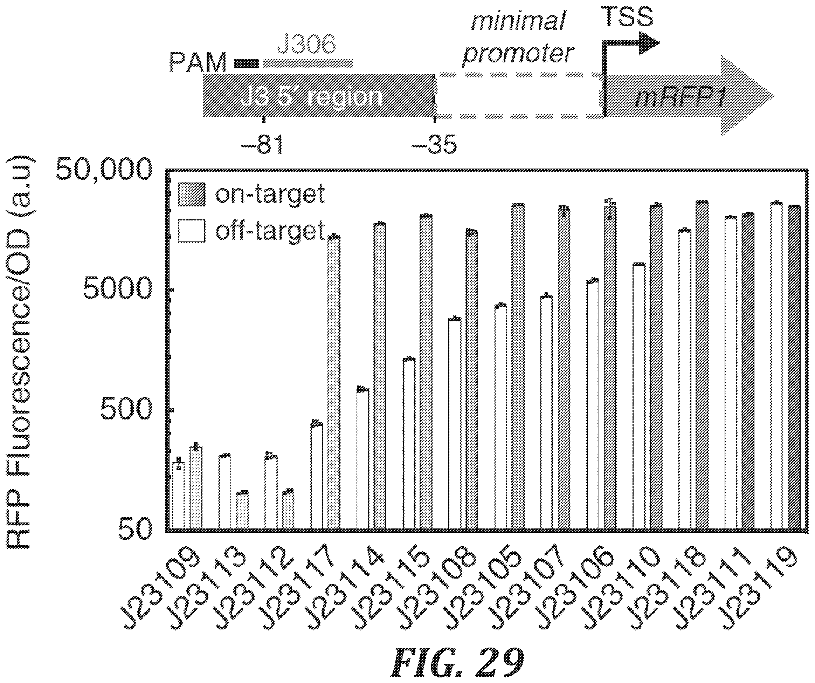

FIGURE 29 graphically illustrates that CRISPRa is sensitive to promoter strength. Promoters contain a scRNA target site at -81 from the TSS of the indicated J231NN minimal promoter, on the non-template strand3. The illustrated graph

shows the Fluorescence/OD$_{600}$ of strains expressing an on-target or off-target scRNA.

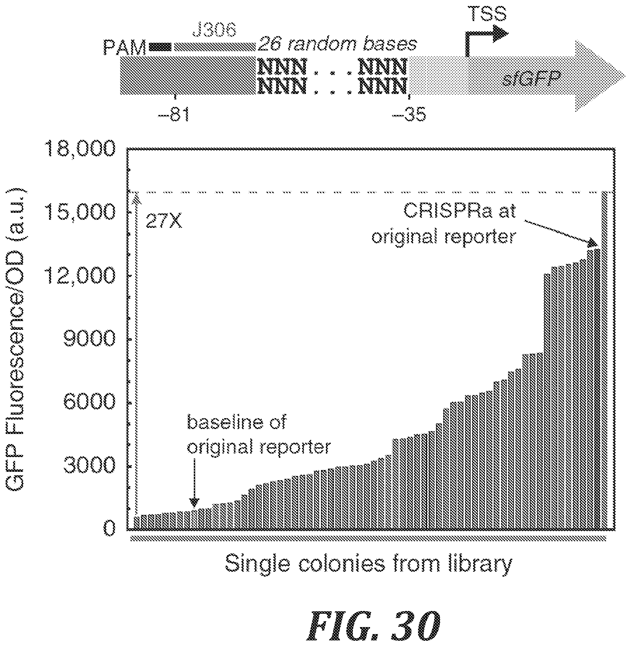

FIGURE 30 graphically illustrates that CRISPRa activity differs significantly among promoters with varying sequence composition between the scRNA target and the -35 region. Most bars represent the Fluorescence/OD$_{600}$ of overnight cultures from individual colonies. The dark bar (indicated by the right arrow) represents the Fluorescence/OD$_{600}$ of a strain expressing the J3-J23117-sfGFP reporter, activated by CRISPRa with the J306 scRNA. The bar indicated by the left arrow represents a negative control expressing the J3-J23117-sfGFP reporter plasmid with CRISPRa targeting an off-target site (J206).

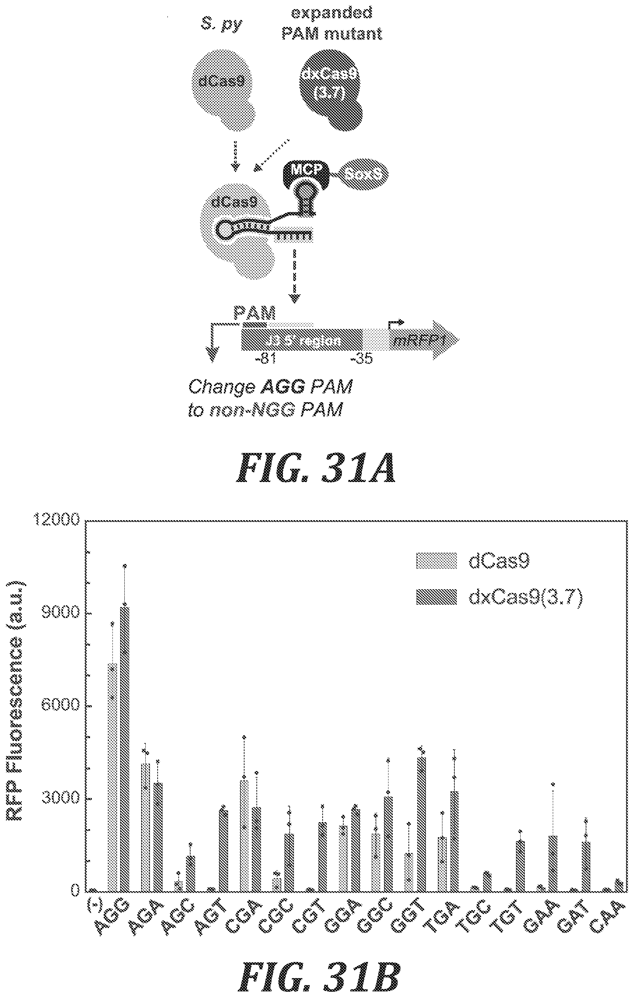

FIGURES 31A and 31B illustrate that dxCas9(3.7) expands the range of targetable scRNA target sites by recognizing alternative PAMs. CRISPRa with dxCas9(3.7) displayed activity on non-NGG PAM sites with AGA, AGC. AGT. CGA, CGC. CGT. GGA, GGC. GGT, TGA, TGC. TGT, GAA, GAT, CAA sequences. CRISPRa activity with dxCas9(3.7) on non-NGG PAM sites was generally lower (6-fold to 89-fold activation relative to a control without a scRNA) compared to the AGG PAM site (188-fold activation). Sp-dCas9 also displayed moderate CRISPRa activity at non-NGG PAM sites with AGA, CGA, GGA, GGC, GGT. TGA sequences. Reporter plasmids were constructed by replacing the AGG PAM site for the J306 target in the J3-J23117-mRFPI reporter with alternative PAM sequences that have been previously reported to be recognized by dxCas9(3.7) in human cells (Hu, J. H. et al. Evolved Cas9 variants with broad PAM compatibility and high DNA specificity. Nature 556, 57-63 (2018)). The (-) sign indicates a control expressing the original reporter with the AGG PAM and the CRISPRa components with Sp-dCas9, the activation domain and no scRNA.

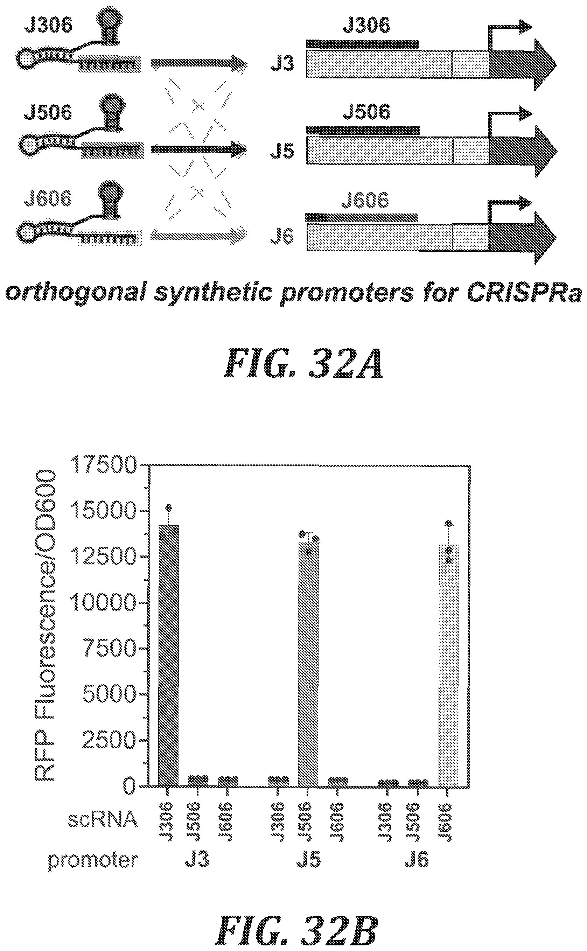

FIGURES 32A and 32B illustrate that CRISPRa targeting synthetic promoters with designed target sites exhibit independent and specific control. 32A) Schematic illustration of assay design. 32B) Graph demonstrating specific signal is only achieved when exposed to an scRNA with a gRNA specific for the engineered promoter target sequence.

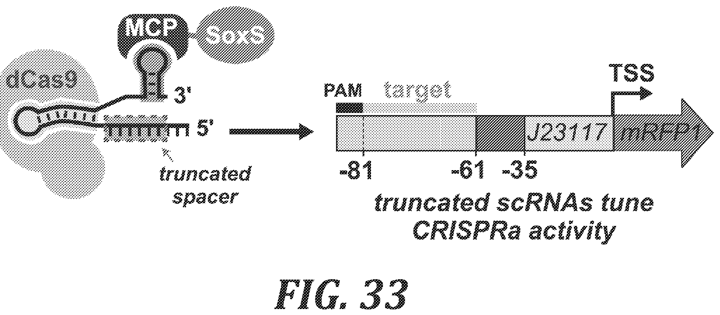

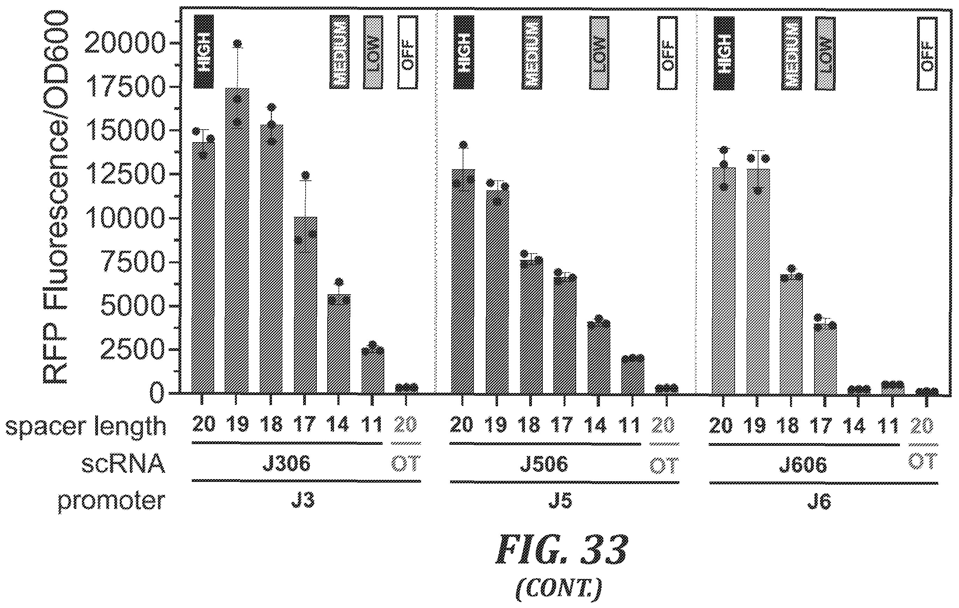

FIGURE 33 schematically and graphically illustrate that gene expression can be tuned for each promoter by modification of the matching scRNA from the 5' end.

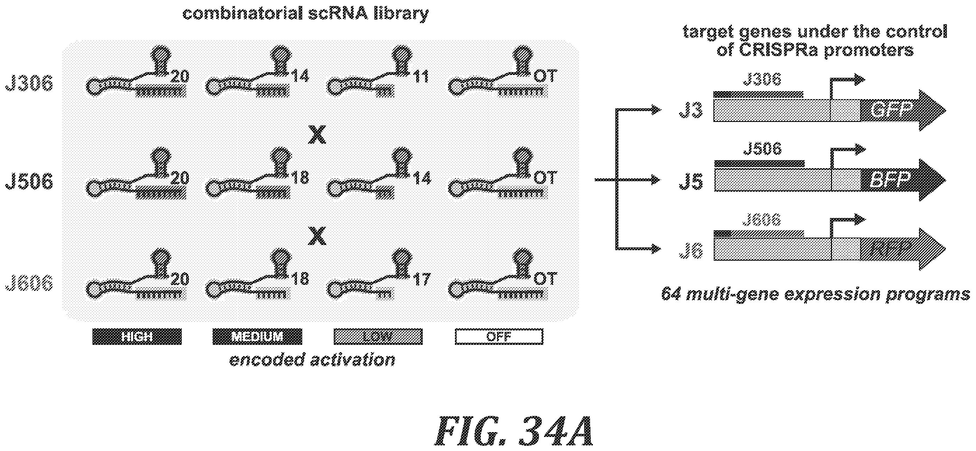

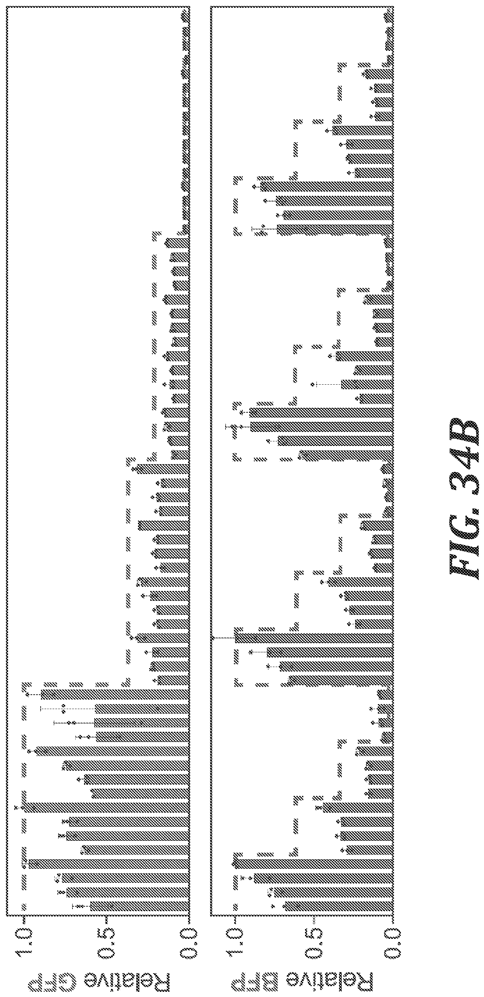

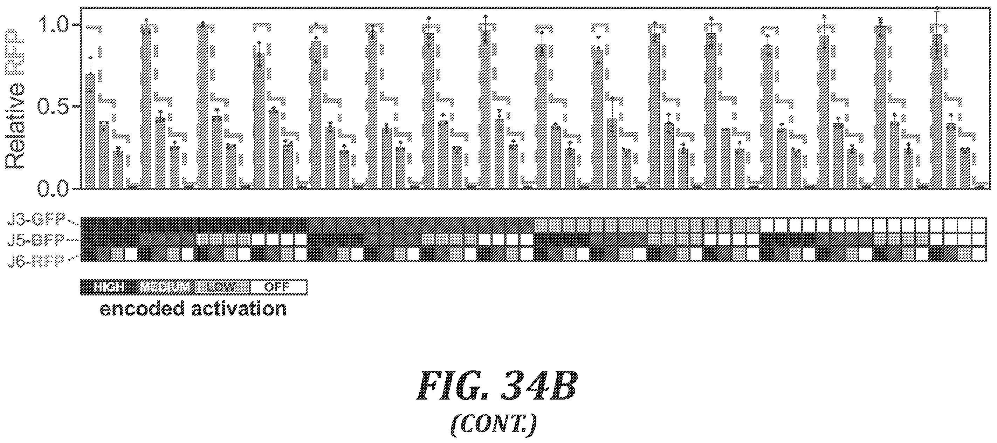

FIGURES 34A and 34B illustrate the independent tuning of gene expression for multiple CRISPRa target reporter genes. 34A) Schematic illustration for creation of library scRNAs with all possible combinations of four tuned expression levels for three different target reporter genes. 34B) Graphic illustration of the specific reporter fluorescence levels from all possible combinations of the four tuned expression levels for the three different reporter genes using the library of scRNAs illustrated in 34A.

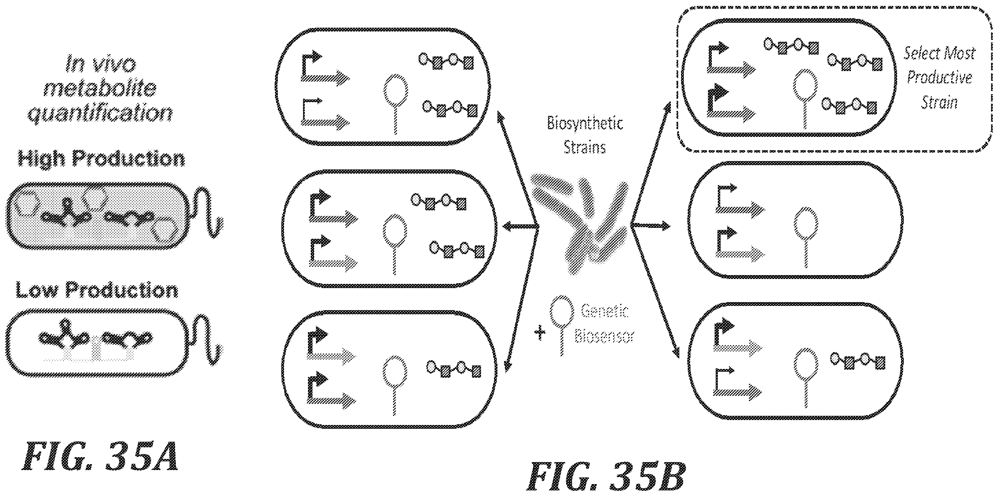

FIGURES 35A and 35B schematically illustrate the implementation of ligand-responsive biosensors and target expression cassettes for development of cells with enhance bioproduction of a metabolite of choice. 35A) Diagram showing difference between an engineered cell with high production of desired bioproduct versus an engineered cell with low production of the bioproduct. 35B) By utilizing a genetically-encoded biosensor for the desired bioproduct it is possible to rapidly identify the most productive biosynthetic pathway variants on the basis of an easily detectable signal such as cell color.

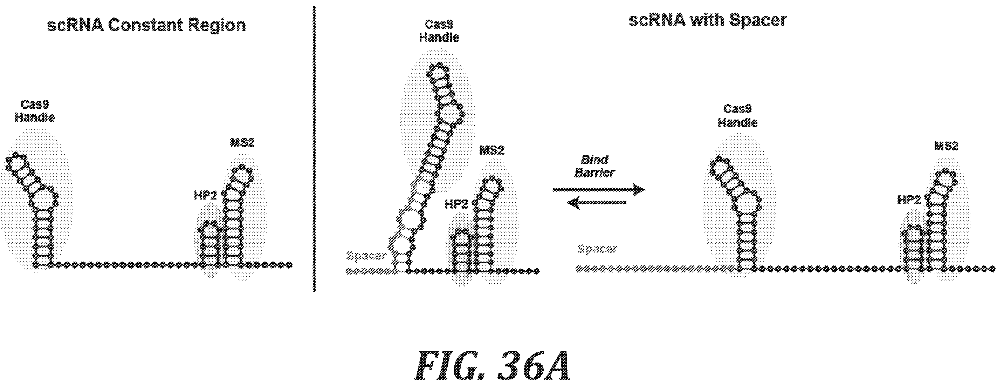

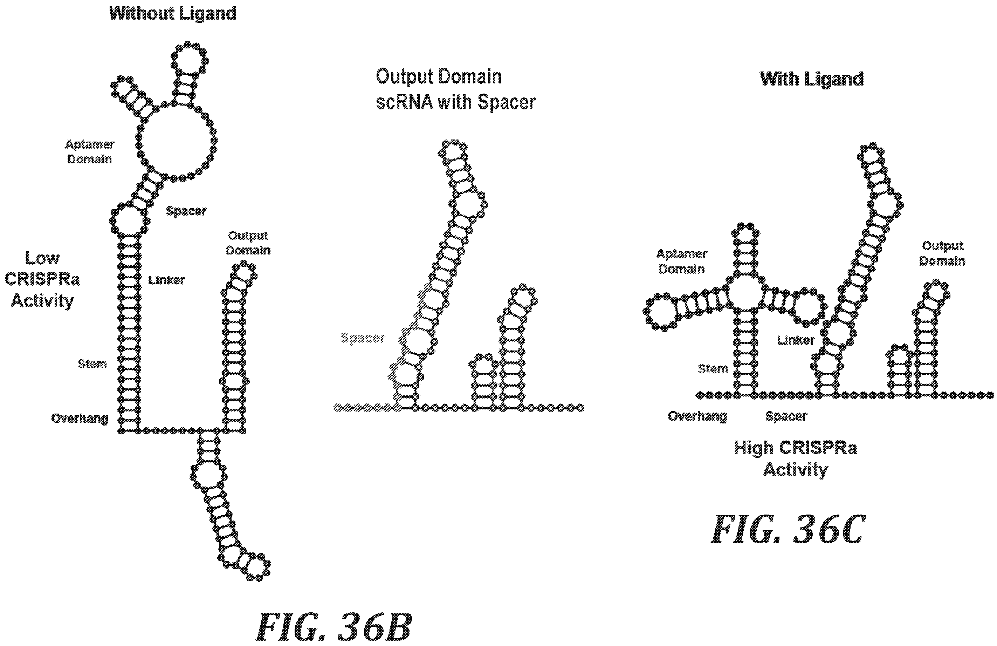

FIGURES 36A-36C illustrates the engineering kinetically-controlled ligand-responsive scRNAs (LR-scRNAs) to regulate bacterial CRISPRa. 36A) scRNA activity depends on the ability of the scRNA to rapidly adopt a binding-competent conformation. 36B) The scRNA conformation corresponding to the absence of 5' sequence is used as an objective for structure-based switch screening. 36C) LR-scRNA candidates are designed such that in the absence of the target molecule the scRNA adopts a conformation incapable of binding Cas9. In the presence of the target molecule, the scRNA component is able to fold as it would in isolation.

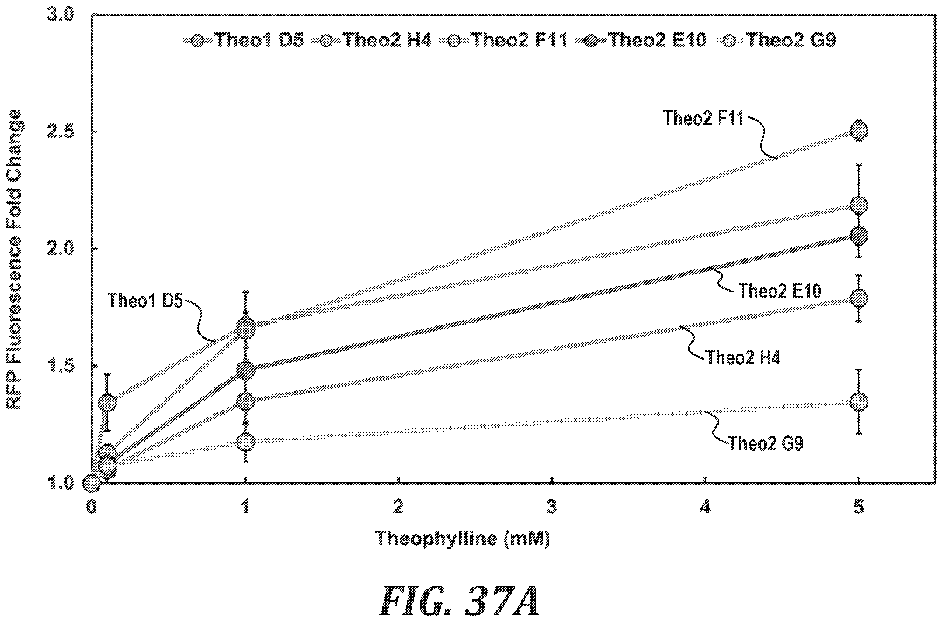

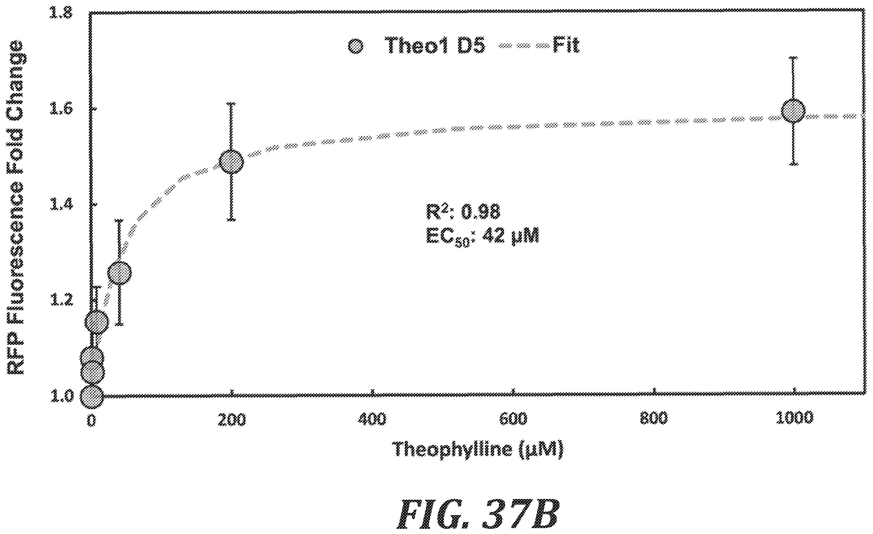

FIGURES 37A and 37B illustrate that engineered ligand-responsive scRNAs are able to respond to theophylline. 37A) Theophylline-responsive LR-scRNAs containing transcriptional pause sites show varied response to theophylline. 37B) Theo1 D5 shows displays the same low EC$_{50}$ as previously engineered AS-RBS riboswitch constructs.

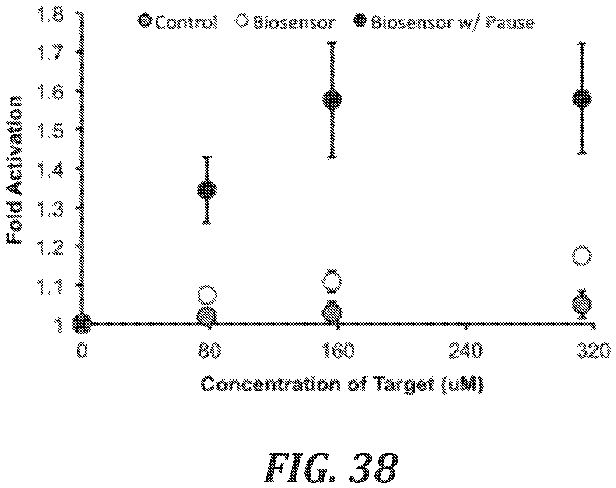

FIGURE 38 graphically illustrates that inclusion of a pause site in a ligand-responsive scRNA biosensor enhances sensitivity of the resulting signal, similar to the AS-RBS riboswitch constructs.

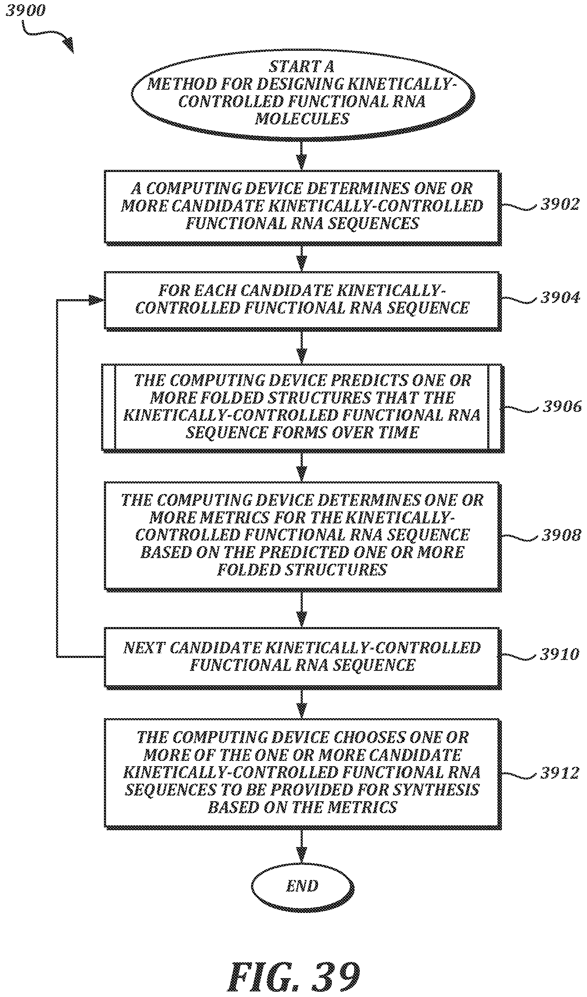

FIGURE 39 illustrates a non-limiting example embodiment of a method for designing kinetically-controlled functional RNA molecules according to various aspects of the present disclosure.

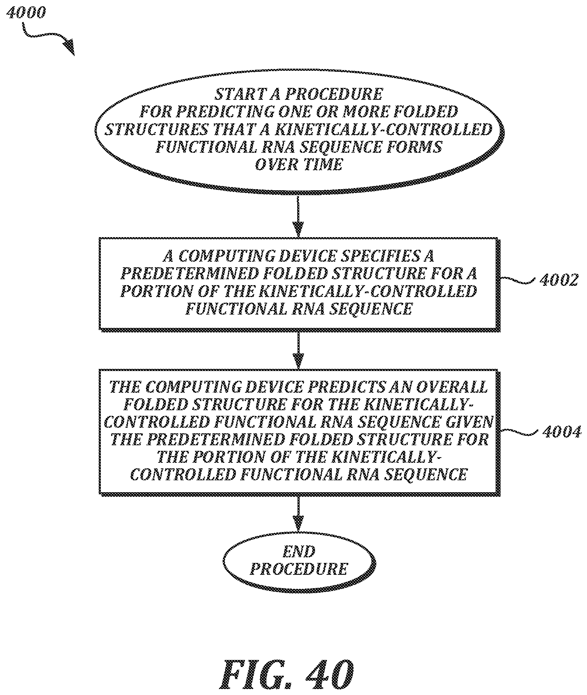

FIGURE 40 illustrates a non-limiting example embodiment of a procedure for predicting one or more folded structures that a kinetically-controlled functional RNA sequence forms over time. In the procedure 4000, a constraint folding analysis is conducted. The procedure 4000 is a non-limiting example of a procedure suitable for use at subroutine block 3906 in FIG. 39.

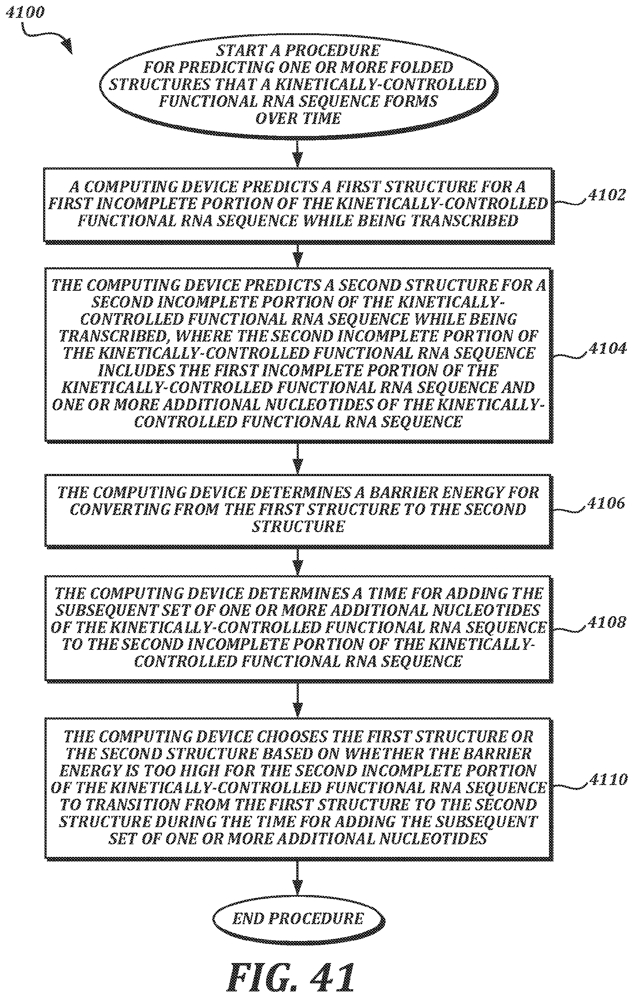

FIGURE 41 illustrates a non-limiting example embodiment of a procedure for predicting one or more folded structures that a kinetically-controlled functional RNA sequence forms over time. In the procedure 4100, two or more co-transcriptional folding pathways are compared. The procedure 4100 is a non-limiting example of a procedure suitable for use at subroutine block 3906 in FIG. 39.

DETAILED DESCRIPTION

The present disclosure is based on the inventors' development of a design platform for modular, tunable, and kinetically-controlled biosensors that can be implemented to produce a vast array of unique biosensors useful across molecular sensing applications. As part of the design, the inventors implemented approaches to design and optimize guide RNAs and synthetic promoters for reporter functionality, but which can be implemented in other CRISPR applications as well.

Kinetically-controlled RNA biosensors are disclosed herein. As used herein, the term "kinetically-controlled RNA biosensor" refers to RNA molecules that can sense (i.e., indicate detection of) a target ligand. The functional characteristics of the kinetically-controlled RNA biosensor are conferred by the three-dimensional folding conformations that are created during active transcription process. The conformations, and by extension the functionalities conferred thereby, are influenced by the presence or absence of the target ligand. Thus, the term "kinetically-controlled" refers to an element of the RNA molecule being actively transcribed (e.g., with the participation of RNA polymerase acting on an encoding template molecule) and elongated in a dynamic process, and the folding that specifically occurs during this process. The kinetically-controlled RNA biosensors are useful because they allow modular coupling of diverse sensing (aptamer) and output domains resulting in the ability to generate signal in response to the desired concentration of an innumerable number of potential target molecules. Furthermore, the sensitivity of a kinetically-controlled RNA biosensor is determined by the amount of time the sensing domain (aptamer) is available during transcription and before the transcription and folding of the output domain. Thus, the sensitivity of kinetically-controlled RNA biosensors can be quantitatively tuned by manipulation of the time required for transcription of the designed constructs. This is in sharp contrast for thermodynamically-controlled RNA biosensors, which rely on complex equilibria between a plethora of transient, ill-defined structure-states that make rational engineering difficult.

To implement reliable and reproducible designs of kinetically-controlled RNA biosensors, convergent expertise in *in vitro* RNA analysis, *in silico* prediction of RNA co-transcriptional folding trajectories, and *in vivo* implementation/analysis of biomolecular switch function inside of cells were required. As described in more detail below, the inventors developed a novel molecular architecture that allowed the disclosed biomolecular switches to take advantage of co-transcriptional RNA folding. This architecture, combined with the use of sequence complementarity to critical regions of the input domains, resulted in an efficient and elegant strategy to create *in silico* libraries of candidate switches with a probability of being functional much higher than through random sequence search. The inventors also developed an algorithm for predicting co-transcriptional folding in a way that allowed access to the underlying quantitative kinetic parameters, permitting the modular, tunable design.

FIGURE 2 schematically illustrates exemplary embodiments of the RNA biosensor design and resulting architecture for active ("ON") and inactive ("OFF") switch states depending on the presence of a ligand. To confer ligand-dependent activity, two different structures must be encoded: an "ON" state where both the sensor domain (including an aptamer domain) and output domain are correctly folded and able to function as they would in isolation, and an "OFF" state where neither domain is correctly folded and, therefore, are nonfunctional. The base-pairing interactions that provide stabilizing energy to the "OFF" state are encoded within the sensor domain, which comprises the overhang, stem, and linker sequences adjacent to the invariable aptamer sequence. The overhang, stem, and linker sequences can have variable lengths (e.g., from 0 to about 15 bases). Embodiments of the biosensor with different lengths for the overhang, stem, and linker sequences are illustrated in "Ex. 1" and Ex. 2". Their sequences allow hybridization with subregions within the aptamer sequence, and subsequences of the output domain, such that they form one continuous helix within the OFF state. For example, the sequences for the named regions (overhang, stem, linker) can be the reverse complement or nearly reverse complement to the sequence in the corresponding subregions within the aptamer sequence, and subsequences of the output domain. For example, the overhang and stem sequences can be the reverse complement to sequences within the 5' end of the output domain such that together they will bind to the 5'-most n bases of the output domain, where n is the sum of the lengths of the overhang and stem domains. The linker sequence can be the reverse complement to the 5' end of the invariable region of the aptamer domain (which is also referred to herein as the linker target sequence).

While the discussion presented herein is generally presented in the context of binary states of "ON" and "OFF" states resulting from the folding in the presence and absence of a ligand, respectively, it will be understood that the configuration can also be inverted. The features and elements described in more detail below can be configured to result in "ON" and "OFF" states based on the absence and presence of the ligand, respectively, or swapped, allowing for configurations of "ON"/"ON", "OFF"/"OFF". "ON"/"OFF", and "OFF"/"ON" dependent on ligand binding or absence of ligand binding. Such inverted embodiments are also encompassed by the present disclosure.

Referring to Figure 1 and the disclosure of Example 1, below, the disclosed modular molecular architecture allows a variety of output domains (ribozyme, ribosome binding site, CRISPR gRNA, etc.) to be controlled by the binding state of an RNA aptamer domain using the same rules and quantitative screening metrics. The overhang, stem, and linker sequences are targeted to specific parts of the sensor and output domains. Their sequences can be the reverse-complement of sequences in the aptamer and output domains, providing thermodynamic incentive for the two domains to cooperatively misfold in the absence of stabilization by the target molecule (ligand). Their lengths can be systematically varied to thermodynamically couple the structures of the two domains, while still allowing the energy from the target ligand to ensure the correct structure upon binding. This enables a very efficient computational search for switch candidates that satisfy the thermodynamic requirements of switching (kinetic requirements are screened later).

Unlike thermodynamic biosensors, which rely on rapid interconversion between two states, the disclosed kinetically-controlled sensors are designed to only sense the target ligand during a brief co-transcriptional binding window. As the RNA is transcribed from the 5' end, first the aptamer folds and opens the binding window by allowing the target ligand to associate. Then the timer domain (described below) is transcribed, increasing the duration of the binding window without providing thermodynamic incentive to misfold. Next, the linker sequence and a portion of the output domain are transcribed. In one illustrative embodiment, it is at this point that the RNA molecule structurally rearranges to an inactive conformation where neither the aptamer, nor output domain, are functional. This is the "OFF/OFF" configuration indicated above. If, however, the target ligand has bound during the binding window, it thermodynamically stabilizes the conformation containing the correctly-folded aptamer, allowing the rest of the output domain to fold into its functional conformation. This is the "ON/ON" configuration indicated above. As indicated above, other embodiment invert this configuration to provide "ON/OFF" or "OFF/ON" configurations. For example, if no ligand binds to the aptamer or sensor domain as it is being transcribed the output domain, once transcribed, will fold resulting in an active or functional (i.e., "ON") output domain. This is the "OFF/ON" configuration indicated above. If a ligand does bind to the aptamer or sensor domain prior to or during transcription of the output domain, there is a thermodynamic incentive to (mis)fold into a configuration that results in a non-functional output domain. This is the "ON/OFF" configuration indicated above. In any of these embodiments and configurations thereof, temporally decoupling the binding window from the output activity, large activation ratios, as well as high- and tunable-sensitivities to the target ligand, become accessible.

The kinetic character of the disclosed RNA biosensors defines a finite ligand binding window in which the aptamer is correctly folded and available to bind its target ligand. By lengthening the duration of the binding window, more of the target ligand will bind at a given concentration. Because the fraction of switches that will be bound to the target ligand at the close of the binding window is determined by the product of the intrinsic ligand-aptamer association rate, the concentration of the target ligand, and the duration of the binding window, an increase in the binding window duration will result in a proportionate increase in the sensitivity for the target ligand. This results in a quantitatively predictable increase in the sensitivity of the kinetically-controlled biosensor when the timer domain is lengthened. The longer the sequence of the timer domain, the longer the binding window. However, without being bound by any particular theory, as the timer domain gets longer it is possible that folding becomes less predictable. To avoid any detriment to aberrant folding of an overly long timer domain, other strategies to significantly increase the binding window without significantly increasing the sequence length of the timer domain can be implemented. For example, transcriptional pause sites, which have never been implemented in *de novo* RNA engineering efforts, can provide such an additional strategy to significantly increase the duration of the binding window while maintaining the length of the timer domain to remain manageable.

The described work provides a computational and experimental pipeline that takes a ligand binding aptamer domain as an input, and outputs a genetic RNA biosensor that can be used to detect the concentration of intracellular metabolites. First, combinatorial variation of three variable regions in the molecular architecture (e.g., overhang, stem, and linker sequences) creates a diverse *in silico* pool of candidate sequences that can be screened using RNA folding simulations. Second, one can perform thermodynamic simulations, utilizing constraint folding, to ensure the proper switch states exist with the desired energies. Next, one can perform co-transcriptional folding simulations, which ensure that the desired states will be accessible on relevant cellular timescales. Finally one can insert timer domains, either with or without a transcriptional pause site, and test the device within a cell to validate response.

In accordance with the foregoing, in one aspect the disclosure provides a kinetically-controlled RNA biosensor construct. Described from 5' to 3', the biosensor construct comprises: a sensor domain that specifically binds to a ligand of interest and an output domain configured to modulate a detectable output signal when folded into an active conformation. The output domain folds into either a functional ("ON") or non-functional "OFF") configuration depending on whether the sensor domain is bound to the ligand of interest. In some embodiments, the output domain folds into the active conformation when the sensor domain is bound to the ligand of interest ("ON/ON" configuration). In contrast, the output domain folds into an inactive conformation when the sensor domain is not bound to the ligand of interest ("OFF/OFF" configuration). In other instances the configurations are inverted where the output domain folds into the inactive conformation when the sensor domain is bound to the ligand of interest ("ON/OFF" configuration) or, alternatively, the output domain folds into an active conformation when the sensor domain is not bound to the ligand of interest ("OFF/ON" configuration).

In some embodiments, the sensor domain comprises, from 5' to 3', at least two of: an overhang sequence, an aptamer domain, and a linker sequence. The aptamer domain itself comprises a stem sequence, a linker target, an aptamer subsequence, and a stem target sequence. The output domain comprises, from 5' to 3': a stem target sequence, an overhang target sequence, and an output subsequence.

In some embodiments, the overhang sequence of the sensor domain is the reverse complement of at least a portion of the overhang target sequence of the output domain. In some embodiments, the overhang sequence of the sensor domain is the reverse complement of at least a substantial portion (e.g., at least about 60%. 65%, 70%, 75%, 80%, 85%. 90%, 95%, 98%) of the overhang target sequence of the output domain. In additional or alternative embodiments, the stem sequence of the sensor domain is the reverse complement of at least a portion of the stem target sequence of the sensor domain and is the reverse complement of at least a portion of the stem target sequence of the output domain. In some embodiments, the stem sequence of the sensor domain is the reverse complement of at least a substantial portion (e.g., at least about 60%, 65%. 70%. 75%, 80%, 85%, 90%, 95%, 98%) of the stem target sequence of the sensor domain and is the reverse complement of at least a substantial portion (e.g., at least about 60%, 65%. 70%, 75%, 80%, 85%, 90%, 95%. 98%) of the stem target sequence of the output domain. In additional or alternative embodiments, the linker target sequence is the reverse complement of at least a portion of the aptamer subsequence and is also the reverse complement of at least a portion of the linker sequence. In some embodiments, the linker target sequence is the reverse complement of at least a substantial portion (e.g., at least about 60%, 65%, 70%, 75%, 80%, 85%, 90%, 95%, 98%, 99%, or all) of the aptamer subsequence and is also the reverse complement of at least a substantial portion (e.g., at least about 60%. 65%. 70%, 75%, 80%. 85%, 90%, 95%, 98%, 99%, or all) of the linker sequence. In a further embodiment the overhang sequence of the sensor domain is the reverse complement of at least a portion (e.g., a substantial portion such as at least about 60%, 65%, 70%, 75%, 80%, 85%, 90%, 95%, 98%. 99%, or all) of the overhang target sequence; the stem sequence of the sensor domain is the reverse complement of at least a portion (e.g., a substantial portion such as at least about 60%, 65%, 70%, 75%, 80%, 85%, 90%, 95%, 98%, 99%, or all) of the stem target sequence of the sensor domain and is the reverse complement of at least a portion of the stem target sequence of the output domain; and the linker target sequence is the reverse complement of at least a portion (e.g., a substantial portion such as at least about 60%, 65%, 70%, 75%, 80%. 85%, 90%, 95%, 98%, 99%, or all) of the aptamer subsequence.

In some embodiments, the portion of the aptamer subsequence that is the reverse complement of at least a portion of the linker target sequence is a discontinuous portion of the aptamer subsequence. When in the "ON" or active folding conformation, e.g., due to the binding of the ligand of interest, the discontinuous portion of the aptamer subsequence can comprise two sequences with reverse complementarity to different portions of the linker target sequence, with an intervening sequence that forms a secondary structure, such as a hairpin domain that contributes to the ligand binding functionality within the aptamer subsequence.

In some embodiments, when the sensor domain is bound to the ligand of interest, the stem sequence of the sensor domain is hybridized to the stem target sequence of the sensor domain, thereby permitting folding of the output domain into the active conformation. Alternatively or in addition, in some embodiments the linker target sequence is hybridized to the portion of the aptamer subsequence, thereby permitting folding of the output domain into the active conformation.

In some embodiments, the hybridization events indicated above are facilitated by the conformation of the sensor domain that is stabilized when a ligand binds to the sensor domain as it is transcribed. This results in an active or "ON" conformation that results in the correct folding of the output domain. In absence of a ligand, the elongating transcript will adopt a different conformation, allowing components of the sensor domain to instead hybridize with the output domain, resulting in an inactive or "OFF" conformation. For example, when the sensor domain is not bound to the ligand of interest, in some embodiments the overhang sequence of the sensor domain hybridizes to the overhang target sequence of the output domain, thereby permitting folding of the output domain into the inactive conformation. Additionally or alternatively, when the sensor domain is not bound to the ligand of interest, the stem sequence of the sensor domain is hybridized to the stem target sequence of the output domain, thereby permitting folding of the output domain into the inactive conformation. Additionally or alternatively, when the sensor domain is not bound to the ligand of interest the linker target sequence of the sensor domain is hybridized to the linker sequence of the sensor domain, thereby permitting folding of the output domain into the inactive conformation. In some embodiments, when the sensor domain is not bound to the ligand of interest, the overhang sequence of the sensor domain hybridizes to the overhang target sequence of the output domain, the stem sequence of the sensor domain is hybridized to the stem target sequence of the output domain, and the linker target sequence of the sensor domain is hybridized to the linker sequence of the sensor domain, thereby permitting folding of the output domain into the inactive conformation. While this disclosure is generally presented to illustrate the "ON/ON" or "OFF/OFF" configurations, it will be understood that the relative configurations can be adjusted to invert the biosensor to result in an "ON/OFF" or "OFF/ON" configuration. In this inverted instance, the sensor domain is stabilized by the binding on the ligand during its transcription and initial folding This binding stabilizes the conformation in a manner that results in a non-functional output domain upon its transcription and resultant folding. Alternatively, when no ligand is present, the senor domain will assume a different conformation that provides thermodynamic incentive for the output domain to fold into a functional conformation upon its transcription.

In some embodiments, at least two of the overhang sequence of the sensor domain, the stem sequence of the sensor domain, and the linker target sequence form a continuous helix stem structure when hybridized to at least a portion of the overhang target sequence of the output domain, a portion of the stem target sequence of the output domain, and a portion of the linker sequence of the sensor domain, respectively, thereby permitting the output subsequence to fold into the inactive conformation.

In some embodiments, the overhang sequence of the sensor domain is between 0 and about 15 nucleotides in length. In some embodiments, the stem sequence of the sensor domain is between 0 and about 15 nucleotides in length. In some embodiments, the linker sequence of the sensor domain is between 0 and about 15 nucleotides in length. In some embodiments, each of the overhang sequence of the sensor domain, the stem sequence of the sensor domain, and the linker sequence of the sensor domain are all between 1 and about 15 nucleotides in length, e.g., each is independently 1, 2, 3, 4, 5, 6, 7, 8, 9, 10, 11, 12, 13, 14, or 15 nucleotides in length.

The biosensor can further comprise a timer domain disposed between the aptamer domain and the linker sequence of the sensor domain. The timer domain can result in additional time between the transcription of the sensor domain and the transcription of the output domain. The additional time provides an extended time window in which the ligand of interest can contact and bind to the sensor domain and, thus, direct the active or "ON" confirmation of the output domain. This additional time window allows additional sensitivity of the biosensor, thus permitting tuning of the biosensor designed to perform optimally at expected concentrations of the ligand of interest. The tuning is implemented by altering the relative length of the timer domain. In some illustrative embodiments, the timer domain has a length up to about 150 nucleotides in length (e.g., about 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, and 150 nucleotides in length). For example, the timer domain can have a length of between 1 and about 50 nucleotides, between about 25 and 75 nucleotides. between about 50 and 100 nucleotides, between about 75 and 125 nucleotides, and between about 100 and 150 nucleotides. In some embodiments, the timer domain is configured to fold into a secondary structure. For example, the timer domain can be configured to form a stem-and-loop structure with each end of the timer domain (i.e., the regions at the 5' end and the 3' end) forming part of a stem of the stem-and-loop structure. The loop structure is comprised of a middle section of the timer domain.

The timer domain can further comprise at least one transcriptional pause sequence. A transcriptional pause sequence is a particular sequence that causes the polymerase to slow its catalytic activity or stop temporarily at the site during transcription. The incorporation of one or more pause sites in the timer domain provides additional time between the transcription of (and folding of) the aptamer domain and the transcription of (and folding of) the output domain. Functionally, the pause in transcription conferred by the transcriptional pause site provides additional time for a ligand of interest to bind to the already transcribed aptamer domain (e.g., binding to the part of the sensor domain formed by the aptamer domain) and, thus, to influence folding of the output domain into an active ("ON") versus inactive ("OFF") conformation. This increased time further increases the sensitivity of the kinetically-controlled RNA biosensor construct for ligands, and allows detection even at low concentrations. Accordingly, the transcriptional pause sequence(s) can be a further tool to "tune" the designed kinetically-controlled RNA biosensor construct to optimize performance at various ligand concentrations. The disclosure encompasses embodiments of multiple transcriptional pause sequences throughout the timer domain. The multiple transcriptional pause sequences can be the same or different. A non-limiting example of a transcriptional pause sequence is a sequence derived from a thiamine pyrophosphate (TPP)-sensing *thiC* riboswitch from *Escherichia coli.* Other transcription pause sequences are known and are encompassed by this disclosure. See, e.g., Chauvier, A., et al., (2019) Role of a hairpin-stabilized pause in the Escherichia coli thiC riboswitch function, RNA Biology, 16:8, 1066-1073, and Kingston, R. E., and Chamberlin, M. J., (1981) Pausing and attenuation of in vitro transcription in the rrnB operon of E. coli. Cell, 27:3, 523-531 .

The output domain can be any RNA-based construct that can be configured to modulate, directly or indirectly, a detectable signal when folded properly. The modulation can be the induction or increase of a detectable signal. In alternative embodiments, the modulation can be the reduction of a detectable output signal when the output domain is folded into the active conformation. In either approach, the change is detected and used to inform the presence and/or concentration of the ligand of interest.

To illustrate, in some embodiments, when the output domain is folded into an active conformation the output domain is or comprises a functional ribozyme, a functional nuclease guide RNA (gRNA), a ribosome binding site, a transcriptional terminator, or RNA aptamer. In an exemplary embodiment, when the output domain is folded into an active conformation the output domain is or comprises a functional gRNA. wherein the functional gRNA is between about 40 and about 400 nucleotides in length. The gRNA can be designed using the computational method, described in more detail below, that enhances the interaction of the gRNA with its cognate target sequence.

In some embodiments, the functional gRNA associates with a nuclease, such as a nuclease selected from Cas9, Cas12a, Cas13, derivatives thereof, and the like. In this regard, the gRNA can integrate into a CRISPR-based reporting system. In some embodiments, the nuclease has ablated nuclease function. In some embodiments, the nuclease confers CRISPR activation (CRISPRa) function (see, e.g., Example 4). In some embodiments, the nuclease confers CRISPR inhibition (CRISPRi) function. In some embodiments, the nuclease has nuclease function.

The ligand of interest can be any compound or moiety that is capable of binding by an RNA aptamer. Non-limiting examples of ligands of interest include chemicals, metabolites, proteins, peptides, small molecules (e.g., drug molecules, drug precursor molecules, drug metabolites, etc.), cells, and the like. Persons of ordinary skill in the art can readily identify other ligands that are encompassed by this disclosure.

The kinetically-controlled RNA biosensor construct can be designed using computational approaches that ensure that the different domains (e.g., the sensor domain and the output domain) are independently functional, but can operate in a binary "switch" manner when fused into a single construct with the sensor domain being transcribed before the output domain. For example, the computer-implemented design method described in more detail below in the context of guide RNA design is configured and applied to the design of the overall kinetically-controlled RNA biosensor construct. A person of ordinary skill in the art can make the requisite adjustments to the described method to ensure design and output of function RNA biosensors constructs, as described above.

In another aspect, the disclosure provides a polynucleotide molecule comprising a sequence encoding the kinetically-controlled RNA biosensor construct described herein. The polynucleotide can comprise or consist of DNA or RNA. In some embodiments, the polynucleotide is a DNA molecule, which also comprises a promoter operatively linked to the encoding sequence. The term "promoter" refers to a regulatory nucleotide sequence that can activate transcription (expression) of encoding DNA. A promoter is typically located upstream of the encoding DNA, but can be located at other regions proximal to the encoding DNA. The promoter typically contains binding sites for RNA polymerase and one or more transcription factors, which participate in the assembly of the transcriptional complex. As used herein, the term "operatively linked" indicates that the promoter and the encoding DNA are configured and positioned relative to each other in a manner such that the promoter can activate transcription of the encoding nucleic acid by the transcriptional machinery of the cell. The promoter can be constitutive or inducible. Constitutive promoters can be determined based on the character of the target cell or transcription environment and the particular transcription factors available therein. A person of ordinary skill in the art can select an appropriate promoter based on the intended purpose, as various promoters are known and commonly used in the art.

In another aspect, the disclosure provides a vector comprising the DNA molecule described herein. The vector can be any construct that facilitates the delivery of the nucleic acid to the target cell or transcription environment (e.g., acellular environment) and/or expression of the nucleic acid within the cell or environment. The vectors can be viral vectors, circular nucleic acid constructs (e.g., plasmids), or nanoparticles. Various viral vectors are known in the art and are encompassed by the present disclosure. See, e.g.. Machida. C. A. (ed.), Viral Vectors for Gene Therapy: Methods and Protocols, Humana Press, Totowa, New Jersey (2003); Muzyczka, N., (ed.), Current Topics in Microbiology and Immunology: Viral Expression Vectors, Springer-Verlag. Berlin, Germany (2012).

In some embodiments, the viral vector is an adeno associated virus (AAV) vector, an adenovirus vector, a retrovirus vector, or a lentivirus vector. A specific embodiment of an AAV vector includes the AAV2.5 serotype.

In another aspect, the disclosure provides a cell comprising the nucleic acid or the vector described above. The cell is capable of transcribing the kinetically-controlled RNA biosensor construct from the DNA molecule. For example, a promoter operatively linked to the encoding DNA can be appropriately configured to allow binding of the cell's RNA polymerase and one or more transcription factors to permit assembly of the transcriptional complex.

The disclosure encompasses any type of cell for this aspect. For example, the cell can be prokaryotic or eukaryotic, without limitation.

The cell can be engineered to further comprise an expression construct comprising a reporter gene operatively linked to a promoter, wherein the promoter is targeted by the output domain when folded into the active ("ON") conformation. For example, the promoter can be a synthetic promoter optimized to be bound by a functional gRNA. In some embodiments, the output domain of the kinetically-controlled RNA biosensor construct is or comprises a functional gRNA when folded into an active conformation, and the functional gRNA hybridizes to the synthetic promoter. In some embodiments, the synthetic promoter contains a Protospacer Adjacent Motif (PAM) positioned between about 40 and about 120 bases 5' of the transcription start site. The synthetic promoter design and its use in CRISPR activation (CRISPRa) is described in more detail in Fontana, J., et al., "Effective CRISPRa-mediated control of gene expression in bacteria must overcome strict target site requirements," Nature Communications (2020) 11:1618.

In some embodiments, the cell is engineered to further express a nuclease with ablated nuclease functionality and a transcription factor, wherein the expressed nuclease and transcription factor associate with the functional gRNA and are configured to facilitate transcription of the reporter gene when the gRNA hybridizes with the synthetic promoter of the expression construct. See, e.g., Mali P, Esvelt KM. and Church GM. Cas9 as a versatile tool for engineering biology. Nat Methods. 2013 Oct,10(10):957-63, and Dominguez AA, Lim WA, and Qi LS. Beyond editing: repurposing CRISPR-Cas9 for precision genome regulation and interrogation. Nat Rev Mol Cell Biol. 2016 Jan;17(1):5-15.

In some embodiments, the cell is engineered or treated to modify expression or production of the ligand which the biosensor binds to. For example, the cell can be engineered to increase or decrease biosynthesis of a particular metabolite or bioproduct. The expression of the kinetically-controlled RNA biosensor construct can facilitate the identification of cells that biosynthesize the metabolite or bioproduct, e.g., to screen for the success of the genetic manipulations.

The disclosed RNA biosensor is not limited to use in cells. Thus, in another aspect, the disclosure provides a biosensor system that comprises:

a first DNA expression cassette comprising sequence encoding the kinetically-controlled RNA biosensor construct as described herein; and

an RNA polymerase and NTPs sufficient to facilitate synthesis of the kinetically-controlled RNA biosensor construct.

The biosensor system can be configured to perform *in vitro,* cell-free transcription (IVT). In further embodiments, the biosensor is configured to perform cell-free synthesis (CFS) of protein from transcribed RNA templates. Accordingly, in such embodiments, the biosensor system further comprises protein translation elements selected from ribosomes, tRNAs, aminoacyl-tRNA synthetase, initiation factors, elongation factors, termination factors, amino acids, ATP, GTP, and/or translation co-factors, in any combination.

In some embodiments, the biosensor system further comprises an expression construct comprising a reporter gene operatively linked to a synthetic promoter, wherein the output domain of the kinetically-controlled RNA biosensor construct is or comprises a functional gRNA when folded into an active conformation, and the functional gRNA hybridizes to the synthetic promoter.

In some embodiments, the biosensor system further comprises a nuclease with ablated nuclease functionality, and a transcription factor. The nuclease and transcription factor associate with the functional gRNA and are configured to facilitate transcription of the reporter gene when the associated gRNA hybridizes with the synthetic promoter of the expression construct.

Described herein is a method of detecting a ligand of interest. The method comprises synthesizing the kinetically-controlled RNA biosensor construct, as described herein, in an environment that may contain the ligand of interest, and detecting an output signal. A detected output signal or modulation of an output signal indicates binding of the ligand of interest to the sensor domain.

In some instances, the environment is an *in vitro* environment capable of facilitating transcription of the kinetically-controlled RNA biosensor construct. For example, the *in vitro* environment can comprise an RNA polymerase, NTPs, and a template DNA molecule as described herein to facilitate synthesis of the kinetically-controlled RNA biosensor construct. In some instances, the environment is a cell-free synthesis (CFS) environment. For example, the CFS environment can comprise protein translation elements selected from ribosomes, tRNAs, aminoacyl-tRNA synthetase, initiation factors, elongation factors, termination factors, amino acids, ATP, GTP, and/or translation co-factors, in any combination. In some instances, the environment comprises a cell lysate.

In other instances, the environment is in a cell. The cell can be engineered, such as in a manner described above. For example, the cell can be engineered to further comprise an expression construct comprising a reporter gene operatively linked to a synthetic promoter, wherein the output domain of the kinetically-controlled RNA biosensor construct is or comprises a functional gRNA when folded into an active conformation, and the functional gRNA hybridizes to the synthetic promoter. In some instances, the cell is engineered to further express a nuclease with ablated nuclease functionality and a transcription factor, wherein the expressed nuclease and transcription factor associate with the functional gRNA and are configured to modulate transcription of the reporter gene when the gRNA hybridizes with the synthetic promoter of the expression construct. In some instances, the nuclease with ablated nuclease functionality confers CRISPR activation (CRISPRa) function, and wherein the reporter gene encodes a fluorescent protein, an antibiotic resistance protein, beta-galactosidase, and the like. In some instances, the nuclease with ablated nuclease functionality confers CRISPR inhibition (CRISPRi) function. For example, in some instances, the nuclease with ablated nuclease functionality confers CRISPR inhibition (CRISPRi) function and the reporter gene encodes a fluorescent protein, an antibiotic resistance protein, beta-galactosidase, and the like, or an endogenous gene. A change is detectable as a result of CRISPRi activity, indicating the status of ligand binding to the biosensor construct.

In some instances, the output domain of the kinetically-controlled RNA biosensor construct is or comprises a functional gRNA when folded into an active conformation, and the functional gRNA hybridizes to a target sequence of interest, and wherein the cell is engineered to further express a nuclease that has nuclease function.

In some instances, the cell is engineered to modify production of a ligand of interest in the cell, as described above. In other embodiments, the method further comprises subjecting the cell to experimental conditions suspected to modify production of the ligand of interest in the cell. In other instances, the ligand of interest is a compound contacted to the cell or a metabolite thereof. In such instances, the method can be a method of detecting whether the compound is transported into or out of the cell, or is metabolized by the cell to produce the metabolite.

The disclosure provides an RNA molecule that comprises an RNA sequence encoded by a set forth in one of SEQ ID NOS:44~49. In some instances, the RNA sequence is encoded by a sequence set forth in one of SEQ ID NOS: 1-43. As described in more detail below, these sequences can serve as variant Cas9-binding handles that retain at least 90% functionality of the reference handle sequence Accordingly, the RNA molecule can be a guide RNA (gRNA) or scaffold RNA construct comprising the gRNA domain. The gRNA or gRNA domain comprises the disclosed sequences and can bind to Cas9

The disclosure provides a computer-implemented method for designing kinetically-controlled functional RNA molecules. As used in this context, the term "kinetically-controlled functional RNA" refers to an RNA molecule that has a functional capacity conferred by the three dimensional conformation that is assumed during active transcription. In some instances, the functional conformation is distinct from a later-assumed conformation of the same RNA molecule (e.g., after prolonged storage). Thus, the kinetic control refers to dynamics culminating in the transcription process. In some embodiments the kinetically-controlled functional RNA molecule is or comprises a guide RNA (gRNA) molecule. In other instances, the kinetically-controlled functional RNA molecule is a kinetically-controlled RNA biosensor molecule. Each of these instances are described in more detail elsewhere herein.

This method comprises:

determining, by a computing device, one or more candidate kinetically-controlled functional RNA sequences, for each of the one or more candidate kinetically-controlled functional RNA sequences:

predicting, by the computing device, one or more folded structures that the kinetically-controlled functional RNA sequence forms over time.
determining, by the computing device, one or more metrics for the kinetically-controlled functional RNA sequence based on the predicted one or more folded structures; and
choosing, by the computing device, one or more of the one or more candidate kinetically-controlled functional RNA sequences to be provided for synthesis based on the scores.

In some instances, the step of determining the one or metrics for the kinetically-controlled functional RNA sequence based on the predicted one or more folded structures includes at least one of:

determining an energy of a predicted folded structure for the kinetically-controlled functional RNA sequence;
comparing an energy of a predicted folded structure for the kinetically-controlled functional RNA sequence to energies of other predicted folded structures for the kinetically-controlled functional RNA sequence; and
determining a barrier energy for converting a predicted folded structure for the kinetically-controlled functional RNA sequence to a target folded structure.

In some instances, predicting one or more folded structures that the kinetically-controlled functional RNA sequence forms over time includes conducting a constraint folding analysis.

In some instances, conducting a constraint folding analysis includes:

specifying a predetermined folded structure for a portion of the kinetically-controlled functional RNA sequence; and
predicting an overall folded structure for the kinetically-controlled functional RNA sequence given the predetermined folded structure for the portion of the kinetically-controlled functional RNA sequence

In some instances, predicting one or more folded structures that the kinetically-controlled functional RNA sequence forms over time includes:
comparing two or more co-transcriptional folding pathways.

In some instances, comparing two or more co-transcriptional folding pathways includes:
predicting a first structure for a first incomplete portion of the kinetically-controlled functional RNA sequence while being transcribed:

predicting a second structure for a second incomplete portion of the kinetically-controlled functional RNA sequence while being transcribed, where the second incomplete portion of the kinetically-controlled functional RNA sequence includes the first incomplete portion of the kinetically-controlled functional RNA sequence and one or more additional nucleotides of the kinetically-controlled functional RNA sequence; and
choosing the first structure or the second structure for use in a subsequent structural prediction for a third incomplete portion of the kinetically-controlled functional RNA sequence that includes a subsequent set of one or more additional nucleotides of the kinetically-controlled functional RNA sequence.

In some instances, choosing the first structure or the second structure includes:

determining a barrier energy for converting from the first structure to the second structure;
determining a time for adding the subsequent set of one or more additional nucleotides of the kinetically-controlled functional RNA sequence to the second incomplete portion of the kinetically-controlled functional RNA sequence,
choosing the first structure in response to determining that the barrier energy is too high for the second incomplete portion of the kinetically-controlled functional RNA sequence to transition from the first structure to the second structure during the time for adding the subsequent set of one or more additional nucleotides: and

choosing the second structure in response to determining that the barrier energy is not too high for the second incomplete portion of the kinetically-controlled functional RNA sequence to transition from the first structure to the second structure during the time for adding the subsequent set of one or more additional nucleotides.

FIGURE 39 illustrates a non-limiting example embodiment of a method for designing kinetically-controlled functional RNA molecules according to various aspects of the present disclosure.

From a start block, the method 3900 proceeds to block 3902. where a computing device determines one or more candidate kinetically-controlled functional RNA sequences. The method 3900 then proceeds to a for-loop defined between a for-loop start block 3904 and a for-loop end block 3910, wherein each of the candidate kinetically-controlled functional RNA sequences is processed to predict folded structures and determine metrics for the kinetically-controlled functional RNA sequences based on the predicted structures.

From the for-loop start block 3904, the method 3900 proceeds to subroutine block 3906, where a subroutine is executed wherein the computing device predicts one or more folded structures that the kinetically-controlled functional RNA sequence forms over time. Any suitable technique for predicting the folded structures may be used, including but not limited to the procedure 4000 or the procedure 4100 described below.

At block 3908, the computing device determines one or more metrics for the kinetically-controlled functional RNA sequence based on the predicted one or more folded structures. Any suitable metrics may be used, including but not limited to one or more of determining an energy of a predicted folded structure for the kinetically-controlled functional RNA sequence, comparing an energy of a predicted folded structure for the kinetically-controlled functional RNA sequence to energies of other predicted folded structures for the kinetically-controlled functional RNA sequence, and determining a barrier energy for converting a predicted folded structure for the kinetically-controlled functional RNA sequence to a target folded structure.

The method 3900 then proceeds to the for-loop end block 3910. If further candidate gRNA sequences remain to be processed, then the method 3900 returns to for-loop start block 3904 to process the next candidate kinetically-controlled functional RNA sequence. Otherwise, the method 3900 proceeds to block 3912.

At block 3912. the computing device chooses one or more of the one or more candidate kinetically-controlled functional RNA sequences to be provided for synthesis based on the metrics. The method 3900 then proceeds to an end block and terminates.

FIGURE 40 illustrates a non-limiting example embodiment of a procedure for predicting one or more folded structures that a kinetically-controlled functional RNA sequence forms over time. In the procedure 4000, a constraint folding analysis is conducted. The procedure 4000 is a non-limiting example of a procedure suitable for use at subroutine block 3906 in FIGURE 39.

From a start block, the procedure 4000 advances to block 4002, where a computing device specifies a predetermined folded structure for a portion of the kinetically-controlled functional RNA sequence. At block 4004, the computing device predicts an overall folded structure for the kinetically-controlled functional RNA sequence given the predetermined folded structure for the portion of the kinetically-controlled functional RNA sequence. Further details of each of these actions are included above.

The procedure 4000 then proceeds to an end block and terminates.

FIGURE 41 illustrates a non-limiting example embodiment of a procedure for predicting one or more folded structures that a kinetically-controlled functional RNA sequence forms over time. In the procedure 4100, two or more co-transcriptional folding pathways are compared. The procedure 4100 is a non-limiting example of a procedure suitable for use at subroutine block 3906 in FIGURE 39.

From a start block, the procedure 4100 advances to block 4102, where a computing device predicts a first structure for a first incomplete portion of the kinetically-controlled functional RNA sequence while being transcribed.

At block 4104, the computing device predicts a second structure for a second incomplete portion of the kinetically-controlled functional RNA sequence while being transcribed, where the second incomplete portion of the kinetically-controlled functional RNA sequence includes the first incomplete portion of the kinetically-controlled functional RNA sequence and one or more additional nucleotides of the kinetically-controlled functional RNA sequence.

At block 4106, the computing device determines a barrier energy for converting from the first structure to the second structure.

At block 4108, the computing device determines a time for adding the subsequent set of one or more additional nucleotides of the kinetically-controlled functional RNA sequence to the second incomplete portion of the gRNA sequence.

At block 4110, the computing device chooses the first structure or the second structure based on whether the barrier energy is too high for the second incomplete portion of the kinetically-controlled functional RNA sequence to transition from the first structure to the second structure during the time for adding the subsequent set of one or more additional nucleotides.

Further description of the actions of each of the blocks 4102 - 4110 is provided above.

The procedure 4100 then proceeds to an end block and terminates.

As indicated above, in some instances, the kinetically-controlled functional RNA is a guide RNA (gRNA) molecule. In some instances, the kinetically-controlled functional RNA is a kinetically-controlled RNA biosensor. In some specific instances, the kinetically-controlled functional RNA of this is a kinetically-controlled RNA biosensor that comprises guide RNA (gRNA) molecule as an output domain. In other instances, the output domain does not comprise a gRNA but rather an alternative kinetically-controlled functional RNA domain. For example, the kinetically controlled RNA biosensor has an output domain that is or comprises a riboswitch domain, as described herein.

The disclosure provides a non-transitory computer-readable medium having computer-executable instructions stored thereon that, in response to execution by one or more processors of a computing device, cause the computing device to perform actions of the computer-implemented method as described above.

The disclosure provides a computing device configured to perform actions of the computer-implemented method as described above.

The disclosure provides computer-implemented methods, non-transitory computer-readable media, and computing devices, as described above, but implemented for the design and implementation of kinetically-controlled RNA sensor constructs, also as described above. The above elements of the computer-implemented methods and related media and devices can be modified and implemented to the design of the disclosed kinetically-controlled RNA biosensor construct to ensure that the different domains (e.g., the sensor domain and the output domain) are independently functional, but can operate in a binary "switch" manner when fused into a single construct with the sensor domain being transcribed before the output domain. Accordingly, for brevity the above elements are encompassed by the present disclosure and are not repeated.

General Definitions

Unless specifically defined herein, all terms used herein have the same meaning as they would to one skilled in the art of the present disclosure. Practitioners are particularly directed to Ausubel, F.M., et al. (eds.), Current Protocols in Molecular Biology, John Wiley & Sons, New York (2010); Coligan, J.E., et al. (eds.), Current Protocols in immunology, John Wiley & Sons, New York (2010); Mirzaei. H. and Carrasco, M. (eds.), Modem Proteomics - Sample Preparation, Analysis and Practical Applications in Advances in Experimental Medicine and Biology. Springer International Publishing. 2016; Mali P. Esvelt KM, and Church GM. Cas9 as a versatile tool for engineering biology. Nat Methods. 2013 Oct;10(10):957-63. and Dominguez AA, Lim WA, and Qi LS. Beyond editing: repurposing CRISPR-Cas9 for precision genome regulation and interrogation. Nat Rev Mol Cell Biol. 2016 Jan;17(1):5-15, for definitions and terms of art.

For convenience, definitions for certain terms employed in this disclosure are provided here. The definitions are provided to aid in describing particular embodiments and are not intended to limit the claimed invention, because the scope of the invention is limited only by the claims.

The use of the term "or" in the claims is used to mean "and/or" unless explicitly indicated to refer to alternatives only or the alternatives are mutually exclusive, although the disclosure supports a definition that refers to only alternatives and "and/or."

The words "a" and "an," when used in conjunction with the word "comprising" in the claims or specification, denotes one or more, unless specifically noted.

Unless the context clearly requires otherwise, throughout the description and the claims, the words "comprise," "comprising," and the like, are to be construed in an inclusive sense as opposed to an exclusive or exhaustive sense, which is to indicate, in the sense of "including, but not limited to." Words using the singular or plural number also include the plural and singular number, respectively. Additionally, the words "herein," "above," and "below," and words of similar import, when used in this application, shall refer to this application as a whole and not to any particular portions of the application. The word "about" indicates a number within range of minor variation above or below the stated reference number. For example, in some embodiments, the term "about" refers to a number within a range of 10%, 9%, 8%, 7%. 6%, 5%, 4%, 3%. 2%, or 1% above and/or below the indicated reference number.

A nucleic acid is a polymer of monomer units or "residues". The monomer subunits, or residues, of the nucleic acids each contain a nitrogenous base (i.e., nucleobase) a five-carbon sugar, and a phosphate group. The identity of each residue is typically indicated herein with reference to the identity of the nucleobase (or nitrogenous base) structure of each residue. Canonical nucleobases include adenine (A), guanine (G), thymine (T), uracil (U) (in RNA instead of thymine (T) residues) and cytosine (C). However, the nucleic acids of the present disclosure can include any modified nucleobase, nucleobase analogs, and/or non-canonical nucleobase, as are well-known in the art. Modifications to the nucleic acid monomers, or residues, encompass any chemical change in the structure of the nucleic acid monomer, or residue, that results in a noncanonical subunit structure. Such chemical changes can result from, for example, epigenetic modifications (such as to genomic DNA or RNA), or damage resulting from radiation, chemical, or other means. Illustrative and nonlimiting examples of noncanonical subunits, which can result from a modification, include uracil (for DNA), 5-methylcytosine, 5-hydroxymethylcytosine, 5-formethylcytosine, 5-carboxycytosine b-glucosyl-5-hydroxy-methylcytosine, 8-oxoguanine, 2-amino-adenosine, 2-amino-deoxyadenosine, 2-thiothymidine, pyrrolo-pyrimidine, 2-thiocytidine, or an abasic lesion. An abasic lesion is a location along the deoxyribose backbone but lacking a base. Known analogs of natural nucleotides hybridize to nucleic acids in a manner similar to naturally occurring nucleotides, such as peptide nucleic acids (PNAs) and phosphorothioate DNA.

The five-carbon sugar to which the nucleobases are attached can vary depending on the type of nucleic acid. For example, the sugar is deoxyribose in DNA and is ribose in RNA. In some instances herein, the nucleic acid residues can also be referred with respect to the nucleoside structure, such as adenosine, guanosine, 5-methyluridine, uridine, and cytidine. Moreover, alternative nomenclature for the nucleoside also includes indicating a "ribo" or deoxyribo" prefix before the nucleobase to infer the type of five-carbon sugar. For example, "ribocytosine" as occasionally used herein is equivalent to a cytidine residue because it indicates the presence of a ribose sugar in the RNA molecule at that residue. A nucleic acid polymer can be or comprise a deoxyribonucleotide (DNA) polymer, a ribonucleotide (RNA) polymer. The nucleic acids can also be or comprise a PNA polymer, or a combination of any of the polymer types described herein (e.g., contain residues with different sugars)

As used herein, the term "polypeptide" or "protein" refers to a polymer in which the monomers are amino acid residues that are joined together through amide bonds. When the amino acids are alpha-amino acids, either the L-optical isomer or the D-optical isomer can be used, the L-isomers being preferred. The term polypeptide or protein as used herein encompasses any amino acid sequence and includes modified sequences such as glycoproteins. The term polypeptide is specifically intended to cover naturally occurring proteins, as well as those that are recombinantly or synthetically produced.

"Percent sequence identity" or grammatical equivalents means that a particular sequence has at least a certain percentage of nucleic acid or amino acid residues identical to those in a specified reference sequence using an alignment algorithm. An example of an algorithm that is suitable for determining sequence similarity is the BLAST algorithm which is described in Altschul, et al., J. Mol. Biol. 215:403-410 (1990). Software for performing BLAST analyses is publicly available through the National Center for Biotechnology Information (NCBI) website.

Reverse complement refers to the sequences of corresponding sequences that can mutually hybridize according to Watson-Crick base pairing rules. The term refers to each of the corresponding sequence (i.e., the sense and the anti-sense) hybridizing in reverse orientations with respect to the 5' to 3' directionalities. For example, a sense strand will have a sequence from 5' to 3' that is the complement of a sequence in the corresponding anti-sense strand when the anti-sense strand is aligned in the 3' to 5'. As used herein, it is contemplated that sequences indicated as being the reverse complement of a reference sequence does not have to have perfect, i.e., 100% complementation, but can have some residues that do no complement so long as the corresponding sequences still mutually hybridize under normal operating conditions.

Kinetically-controlled RNA biosensors are defined as RNA biomolecules that sense a target ligand and regulate the folding of an output RNA domain in response, while actively being transcribed by a RNA polymerase enzyme. As indicated above, the RNA molecule will fold into three-dimensional conformations during the active transcription process and which can confer discrete functionalities. The conformations may be distinct from conformations that might occur in a fully formed (i.e., fully transcribed) RNA molecule if it is allowed to fold at a later time. Furthermore, the conformations that develop during transcription are influenced by molecules in the environments, e.g., by the presence or absence of a target ligand that can specifically bind to a domain of the RNA molecule during its transcription.

Disclosed are materials, compositions, and components that can be used for, can be used in conjunction with, can be used in preparation for, or are products of the disclosed methods and compositions. It is understood that, when combinations, subsets, interactions, groups, etc., of these materials are disclosed, each of various individual and collective combinations is specifically contemplated, even though specific reference to each and every single combination and permutation of these compounds may not be explicitly disclosed. This concept applies to all aspects of this disclosure including, but not limited to, steps in the described methods. Thus, specific elements of any foregoing embodiments can be combined or substituted for elements in other embodiments. For example, if there are a variety of additional steps that can be performed, it is understood that each of these additional steps can be performed with any specific method steps or combination of method steps of the disclosed methods, and that each such combination or subset of combinations is specifically contemplated and should be considered disclosed. Additionally, it is understood that the embodiments described herein can be implemented using any suitable material such as those described elsewhere herein or as known in the art.

EXAMPLES

The following examples are provided to illustrate certain features and/or embodiments of the disclosure. This example should not be construed to limit the invention to the particular features or embodiments described.

Example 1

This example discloses the development of a molecular architecture and computational workflow for the *in silico* engineering of representative modular, kinetically-controlled aptamer-based biosensors according to some embodiments of the disclosure. Through *in vitro* co-transcriptional cleavage assays, a set of computational design parameters are identified that enable the robust *in silico* identification of functional RNA switches. High performance switches and tunable ligand sensitives are demonstrated. The biosensors can be used, e.g., for screening applications in industrial biotechnology, medical biotechnology, pharmaceutical development, diagnostics, etc. The described biosensors are kinetically-controlled and the sensitivity can be tuned by implementation of transcription delays and pauses to lengthen the time window available for ligand binding. The modular design allows implementation and optimization of biosensors for any ligand of interest using a variety of output signals.

Introduction

Aptazymes are a class of synthetic RNA switch combining a ligand-binding aptamer domain with an autocatalytic self-cleaving ribozyme domain. As they do not rely on other biomolecules to function, they are attractive for their utility as multi-host genetic controllers, where RNA backbone cleavage can be utilized by the host biochemistry in a variety of ways. Aptazymes have been implemented to dynamically regulate gene expression levels in bacteria, yeast, mammalian cells, and viruses. Another beneficial result of aptazymes' independence from other biomolecules is that their kinetic properties can be readily assayed *in vitro* through changes in the length of the RNA molecules, as characterized by denaturing Urea-PAGE.

Based on ribozyme cleavage rates, and the background hydrolysis rate of RNA, aptazymes can theoretically possess dynamic ranges of greater than $10^7$-fold. However, despite numerous design and selection methods, the best dynamic ranges identified to date within physiological buffer conditions are $< 10^2$-fold, due to high background cleavage. low induced cleavage, or both. One potential reason for this shortcoming of conventionally-designed thermodynamic aptazymes is their fundamental competition between aptazyme dynamic range and their concentration sensitivity. This means it is only possible to generate thermodynamic aptazymes that are sensitive to their target, or possess large dynamic ranges, but not both. This becomes especially problematic when trying to design switches responsive to ligands with low affinities for their cognate aptamer, where the concentration necessary for significant actuation lies above the limit of solubility (or toxicity for a host organism). Another potential reason for the shortcoming of conventional aptazymes is the modification of the component parts. In order to couple the functions of binding and cleavage, the aptamer domain is inserted in place of one of the ribozyme's interaction loops in the hopes of causing the ribozyme domain to reversibly misfold. As these loops are critical for forming tertiary contacts known to dramatically speed ribozyme cleavage, the maximum cleavage rate, and therefore aptazyme performance, is degraded even when the target ligand is bound. Additionally, as the sequences are overlapping, there is no guarantee that the 3D structures of the two domains will be compatible with each other, adding additional unknown variables in the design process.

Numerous strategies have been employed to design aptazymes to date, including semi-rational design, thermodynamic secondary-structure design, *in vitro* selection, and in *vivo* screening. The most successful strategy to date has been *in vivo* selection, wherein libraries containing thousands of variants are screened for their ability to provide ligand-responsive genetic output (usually fluorescence) within a desired genetic context. While computational design holds perhaps the greatest promise long-term, computationally designed aptazymes suffer from poor predictability, with only a small fraction of designed devices proving functional, and possessing small dynamic ranges when they do. When computational design strategies are employed, they are rarely generalizable or systematic, and usually involve varying the sequence composition of a single stem. An additional problem with the rational/computational design of aptazymes for genetic control is their integration into a novel context. Even aptazymes possessing excellent *in vitro* performance are often rendered non-functional when placed into a genetic context, as the surrounding RNA sequence can interact with the aptazyme and hinder its function. Identifying molecular design rules that apply just as well to an aptazyme within a larger RNA molecule, as they do to an isolated aptazyme, would represent a tremendous step forward for RNA design as a whole. A final, significant limitation of conventional aptazymes is the lack of control over the ligand concentration to which the aptazyme responds. For nearly any application, if the aptazyme sensitivity and desired ligand concentration are mismatched, the aptazyme becomes useless. Thus, to fully realize the utility of ligand-responsive switches to the extent that nature has, it will be necessary to discover a route to rationally tune the ligand sensitivity of an aptazyme without breaking the switching capability.

One critical aspect of the cellular production of RNA molecules is that of co-transcriptional folding As the RNA polymerase produces an elongating transcript, that transcript can begin folding before the entire RNA molecule has been produced. Despite being an integral feature of naturally-occurring RNA switches, co-transcriptional folding has long been viewed as an impediment to synthetic RNA design, as the resulting kinetic traps often invalidate thermodynamic structural predictions on relevant biological timescales. A kinetic trap occurs when an RNA molecule becomes stuck in an energetically-suboptimal conformation, due to a slow transition rate to the minimum free-energy (MFE) structure. However, despite this perceived impediment to RNA design, co-transcriptional folding allows the free-energy landscape to evolve, and be programmed, as a function of time and sequence length, resulting in tunable kinetic responses in addition to those dictated solely by thermodynamic ensemble behaviors.

As a general principle, increasing the length of an RNA molecule dictates an increased stability of its minimum free energy (MFE) structure, as well as decreased rates of interconversion between its various global folds. Thus, an elongating transcript provides a unique opportunity to utilize small temporally-resolved inputs early in transcription to dictate large changes in the global RNA structures present on biologically-relevant timescales. While thermodynamic RNA structure predictions have been successfully used to design numerous types of short functional RNAs, longer RNA sequences such as mRNA transcripts are known to possess kinetic traps that prevent those sequences from reaching equilibrium on relevant timescales. Thus, to predict the function of genetically-encoded RNA switches it is necessary to predict what structures those molecules adopt on the short and intermediate timescales dictated by co-transcriptional folding.

To date, there have been numerous efforts to utilize computational algorithms to predict the secondary-structures that an RNA molecule will adopt along its co-transcriptional folding trajectory. Each has its own strengths and weaknesses, which have limited their application to RNA switch design. Problematically, many published algorithms are trained on a data set in order to get good predictions, limiting the kinetic information that can be extracted from individual transcription and refolding steps. Many are difficult to implement, and even more difficult to modify to suit one's own purposes.

To this end, the inventors developed MFEpath, an algorithm for the course-grained screening for sequences able to rapidly fold during transcription into their minimum free-energy (MFE) structure.

<u>Methods</u>

<u>MFEpath algorithm</u>

Algorithmically. MFEpath breaks down coarse grained co-transcriptional RNA folding into a series of binary operations. For each 5' subsequence of the RNA molecule, the previous structure can either transition to the MFE substructure, or the next base is added and the structure remains unchanged (except for rapid local structural rearrangements). To determine which of these two events happens faster, their rates (approximated using structural rearrangement barriers as Arrhenius-like activation energies) are calculated, in one example, using the ViennaRNA algorithm Findpath and a previously described relationship between these barriers and the interconversion rate (Equation 2; Geis, M. et al. Folding Kinetics of Large RNAs. J. Mol. Biol. 379, 160-173 (2008)). Upon addition of the final base, the height of the rearrangement barrier between the penultimate structure and the MFE structure of the full sequence is used to determine the rate of folding into the desired structure. This rate can then be compared to the rate of actuation to determine whether or not the device is expected to be functional on a relevant timescale. As MFEpath does not depend on any specific algorithm it remains applicable as new and more efficient RNA structure/barrier prediction algorithms become available, allowing MFEpath to remain relevant into the foreseeable future.

Preliminary implementations of the MFEpath algorithm possessed a significant limitation with respect to the total evaluation time for long, kinetically-trapped, sequences. The barrier-height analysis algorithm currently employed (Findpath) increases the execution time dramatically as sequence length increases, and as the barriers between the evaluated states increase (a hallmark of sequences that do not follow the MFE structures). As co-transcriptional RNA folding is a pathway-dependent process, a dead end elimination algorithm, that terminates as soon as the pathway deviates from the desired one, is especially well suited for this application, as it saves significant computational time. Therefore, MFEpath has been implemented with a number of checkpoints (some for general properties, and some specific to the correct folding of the kinetically-controlled biosensor structure states) that will terminate the simulation if failed.

<u>Computational kinetically-controlled biosensor</u> design.

In order to identify sequences capable of adopting the desired co-transcriptional structures the population is initially screened to ensure that the desired structure states exist and possess the appropriate relative free energies (FIGURES 11A and 11B). First, everything 5' through the aptamer (s2) is screened for the ability to form the proper aptamer fold necessary for binding the ligand in the MFE structure. Next, each nucleotide of the Timer is added (s3) and screened for the presence of the aptamer structure in the MFE to ensure that once the aptamer domain folds, that it remains folded. Next, the sequence through the toehold target (s4) is considered, and screened for the ability to form a stable toehold-target duplex. Next, the entire sequence (s5) is considered and screened such that when the aptamer is present, the ribozyme is too (and vice versa). Then the entire sequence is again considered (s6) and folded to make sure that neither the aptamer nor ribozyme domain active structures appears in the MFE. This is important, as the presence of the ribozyme domain in the MFE would indicate that the kinetically-controlled biosensor is active regardless of aptamer binding state, and because the presence of the aptamer in the MFE would indicate that binding the target ligand will not selectively stabilize the ribozyme. Finally, the ligand-binding stabilization energy, as calculated from the aptamer dissociation constant, is compared to the difference in free energies of the s5 and s6 states to ensure that if the ligand binds, that the ligand stabilization is sufficient to make s5 the most stable state, instead of s6. Co-transcriptional folding analysis is then performed using MFEpath to ensure that the cutoffs described are passed.

*In vitro* co-transcriptional cleavage analysis

In order to characterize the relevant kinetics of designed kinetically-controlled biosensors it was necessary to develop an assay that mimicked cellular RNA production. The two critical components of cellular production are co-transcriptional folding and low free magnesium concentration. Details of the assay are provided in Sparkman-Yager, D.. et al. Chapter Sixteen - Kinetic Folding Design of Aptazyme-Regulated Expression Devices as Riboswitches for Metabolic Engineering, in Methods in Enzymology (ed. Burke-Aguero. D. H.) vol. 550 321-340 (Academic Press. 2015).
In brief. DNA templates containing the kmetically-controlled biosensor downstream of a bacteriophage T7 RNA polymerase were incubated with T7 RNA polymerase for up to 45 minutes. By matching the concentration of $MgCl_2$ to the concentration of NTPs, the free concentration of $Mg^{2+}$ is expected to be between 0.5 and 1 mM, comparable to what is observed in nature. Reaction aliquots were quenched at four time points by mixing the reaction with a formamide-EDTA solution. Reaction time points were analyzed on an 8% denaturing 7.5 M Urea-PAGE gel. Gel bands were quantified using ImageJ with rolling ball background subtraction of radius 50 (Schneider, C. A., et al. NIH Image to ImageJ: 25 years of image analysis, Nature Method 9.7 (2012):671-675..

Biphasic cleavage fitting

To capture the contributions of the two putative reaction pathways, co-transcriptional cleavage data was fit using a biphasic cleavage function (Equation 7). This 3-parameter function assumes that the rapidly-cleaving burst fraction ($f_{burst}$) cleaves at rate $k_{burst}$, and the slow fraction ($f_{slow}=1-f_{bursrt}$) cleaves with rate $k_{slow}$. All fitting was performed using weighted least-squared regression using the Scipy package in Python. As assay signal (and therefore certainty in $f_{clv.}$) increases as a function of time, time point duration was used for weighting of residuals. To determine the appropriate threshold for distinguishing rapid and slow cleavage, an F-test was performed for selecting either a monophasic or biphasic model for each (alpha= 0.05) of the no-, and max-ligand assays for all 50 K-A devices. For each of the 38 conditions (out of 100) for which the biphasic model was statistically superior, the $k_{burst}$ rate was > 0.2 $min^{-1}$, and the $k_{slow}$ rate was < 0.2 $min^{-1}$. For this reason, the cleavage rate 0.2 $min^{-1}$ was used as a lower cutoff for $k_{burst}$, and an upper cutoff for $k_{slow}$, for all subsequent fitting and analysis for the entire set of 100.
In order to prevent fitting/experimental error to return non-physiological rate constants, all fitting was performed with a lower bound on cleavage rate of $1e^{-7}$ $min^{-1}$ (rate of spontaneous RNA cleavage in buffer), and an upper bound of 5 $min^{-1}$ (maximal cleavage rate of hammerhead ribozymes). To estimate confidence intervals for the calculated variables, percentile bootstrapping was used to yield 95% confidence intervals from 1000 re-sampled data points. Estimation of experimental error yielded a value of 1.15 % $f_{clv}$, by comparing the four timepoints of the 0 mM pAF condition of the pAF10-100nt device assayed on separate days.

$$\text{Equation 7.} \quad f_{unclv.} = f_{burst} * \left(\frac{1-e^{-k_{burst}*t}}{k_{burst}*t}\right) + (1 - f_{burst}) * \left(\frac{1-e^{-k_{slow}*t}}{k_{slow}*t}\right)$$

Results and Discussion

In order to attain the tunable ligand sensitivity and large dynamic ranges (DRs) necessary for future applications, implementation of the kinetic control observed in natural riboswitches was sought in the computational design of aptazymes. To do so, a molecular architecture able to provide robust access to a co-transcriptional ligand-binding window was first required. Inspired by natural riboswitches, the aptamer domain was placed upstream of the ribozyme domain. In doing so it becomes possible to bind the ligand before the ribozyme domain is transcribed, providing a temporal window in which ligand-binding and cleavage are not competing. Unlike conventional aptazymes, which combine the aptamer and ribozyme domains with a randomized communication module, the kinetically-controlled biosensors utilize variable sequence upstream of the aptamer, and between the aptamer and ribozyme domains, to encode the desired structural transitions (FIGURE 1). This has a number of potential advantages: The first is the preservation of the tertiary structure of the component parts. Because none of the internal positions of either aptamer or ribozyme are mutated in order to encode structural transitions, the parts should maintain their optimal parental characteristics such as aptamer $K_d$ and ribozyme cleavage rate. This should increase the likelihood of identifying solutions to the aptazyme design problem, by eliminating cases where the 3D structures of the two domains are incompatible.

If implemented correctly, the kinetically-controlled biosensor molecular architecture will allow the desired structure states to be accessed co-transcriptionally, and the relative population of the active and inactive pathways to be determined by the concentration of ligand present during the co-transcriptional ligand binding window. In the absence of ligand, the elongating kinetically-controlled biosensor undergoes rapid structural rearrangement into an inactive state lacking correctly-folded aptamer and ribozyme domains (FIGURE 3A). In the presence of ligand, however, the aptamer domain is thermodynamically stabilized and kinetically trapped, allowing the rapid folding, and cleavage, of the ribozyme domain. Unlike conventional aptazymes, in which the background cleavage rate is dictated by the relative stability of rapidly equilibrating ON and OFF states, the background rate of kinetically-controlled biosensor cleavage should be dictated by the slow kinetics of conversion from the S6 to S5 states post-transcriptionally. Thus, if the height of the B3 barrier is large, and the rate of no-ligand structural rearrangement (B2) is fast relative to transcription, kinetically-controlled biosensors should be able to attain extremely low background cleavage. Combined with a small B1 barrier, which should allow the rapid formation of the catalytic ribozyme, large DRs should be attainable.

To create the diversity necessary for screening, the kinetically-controlled biosensor architecture divides the mutable nucleotide positions into three regions of variable length, containing complementary sequences to the functional domains (not shown). This complementary sequence provides thermodynamic incentive for the kinetically-controlled biosensor to fold into an alternative more stable structure in which both the aptamer and ribozyme domains are misfolded. Each of the three domains targets a different region, and tuning their respective length modulates the thermodynamic incentive to complex with their target. By combinatorially screening all possible lengths of the three regions it is possible to generate a *in silico* pool containing such solutions for any aptamer or ribozyme domain (FIGURE 3B).

In order to ensure that the designed kinetically-controlled biosensor candidates fold along the desired trajectories the screening is broken into two stages (described in greater detail in the methods provided herein): multi-state thermodynamic screening, and kinetic co-transcriptional screening using our novel MFEpath algorithm. By screening for the presence, and relative stability, of multiple target states within the thermodynamic ensemble we are able to ensure that the device predominantly remains inactive at equilibrium, and that the thermodynamic stability provided by ligand-binding will bias the ensemble towards a catalytically-active fold. By subsequently screening the devices for their ability to rapidly reach the target structure states during transcription, it is ensured that the general thermodynamic switch properties screened previously are accessible within an elongating transcript.

Containing up to 90 variable nucleotide positions, the kinetically-controlled biosensor architecture contains >$10^{54}$ sequences for each aptamer/output pair, dramatically higher than RNA pools that can be commercially synthesized (~$10^{17}$). Based on the successful identification of aptazymes from similarly designed pools containing $10^6$ sequences, the kinetically-controlled biosensor molecular architecture likely contains many sequences that would perform as desired. However, when the kinetically-controlled biosensor candidate pool is generated with these semi-rational complementary sequences, as opposed to completely random sequences, the search space is reduced to a more manageable size (~$10^6$), while dramatically increasing the odds of finding a solution. For a single combination of aptamer and ribozyme domain we determined the odds of identifying a solution to our thermodynamic objective functions is ~1 in 57,000 within the random pool, as opposed to ~1 in 6 for the complementary pool. This ~10,000-fold increase in search efficiency allows a computational pool of $10^6$ sequences to potentially contain as many solutions as a randomly-generated pool of $10^{10}$ sequences, which is significantly greater than what is screenable with current *in vivo* methods.

In order to test the viability of the molecular architecture for designing diverse aptazymes, two different aptamer domains were utilized: the well-studied theophylline aptamer. which binds the methylated xanthine derivative theophylline, and the pAF4z1d3 aptamer, which binds the functionalized amino acid *p*-aminophenylalanine (pAF) (Zimmermann, G. R., et al. Molecular interactions and metal binding in the theophylline-binding core of an RNA aptamer. RNA 6, 659-667 (2000); Carothers, J. M.. et al. Selecting RNA aptamers for synthetic biology: investigating magnesium dependence and predicting binding affinity. Nucleic Acids Res. 38, 2736-2747 (2010)).

Three different ribozymes were also used: The S. *mansoni,* sTRSV1. and PLMVd hammerhead ribozymes (Carothers, J. M., eta I. Model-Driven Engineering of RNA Devices to Quantitatively Program Gene Expression. Science 334, 1716 1719 (2011)). The devices utilizing an *in vitro* co-transcriptional cleavage assay designed to mimic cellular production were analyzed, characterizing the cleavage kinetics at various concentrations of ligand. Fifty different kinetically-controlled biosensors containing combinations of the various aptamer and ribozyme domains were designed. built, and characterized. In doing so it was possible to identify devices, incorporating each of the 5 domains, with dynamic ranges greater than 29, and as high as 240. This demonstrates that the kinetically-controlled biosensor molecular architecture (and the disclosed automated computational design algorithms) can utilize diverse input components to robustly identify functional aptazymes.

Timer domain creates 'binding window'

One important feature of the kinetically-controlled biosensor molecular architecture is the co-transcriptional ligand binding window. The binding window is the period of time after the transcription and folding of the aptamer domain, but before the kinetically-controlled biosensor has made a fate decision. This window closes when the kinetically-controlled biosensor, if unbound by its target ligand, structurally rearranges into a state that is neither able to bind its target nor able to cleave (S6). To ensure that the designed kinetically-controlled biosensors have time for the aptamer domain to properly fold, and the target ligand to associate, additional sequence referred to as the Timer' domain was incorporated. The Timer domain, placed between the aptamer and ribozyme, is designed to be an orthogonal sequence element that does not contribute to the relative energetics of the designed states. However, by providing additional sequence between the two other domains, it extends the binding window by the length of time it takes the Timer to be transcribed.

So long as the ribozyme domain is able to rapidly adopt the active conformation during transcription, on a faster timescale than that of the intrinsic ribozyme cleavage rate, it is expected that kinetically-controlled biosensors that bind their target co-transcriptionally would display rapid cleavage at the rate of their parental ribozyme. Thus, it is expected that kinetically-controlled biosensor molecules that bind their target ligand will be able to cleave at a rapid 'burst' rate, while those that do not will undergo fast structural rearrangement to the inactive S6 state, and only cleave at a 'slow' rate limited by large-scale structural rearrangement to S5 post-transcriptionally. In order to capture the contributions of the two expected reaction pathways, *in vitro* co-transcriptional cleavage data was fit using a biphasic cleavage function (see below for more details), wherein the burst fraction describes the relative abundance of the population of RNA molecules cleaving rapidly.

It was hypothesized that increasing the interdomain separation between the aptamer and ribozyme with a Timer domain would aid in co-transcriptional ligand binding, and observed this to be the case. In fact, Timer domains appear to be important for achieving rapid co-transcriptional actuation, as only kinetically-controlled biosensors containing Timer domains demonstrated ligand-inducible burst phase cleavage. For example, the Theo1-0nt kinetically-controlled biosensor, which has no Timer domain, displays low background cleavage rate, no burst phase kinetics, and a moderate DR (FIGURES 4A-4C). However, the Theo1-15nt kinetically-controlled biosensor, which is an identical sequence with only a 15 nt Timer domain added, displays burst phase kinetics while maintaining the low background cleavage, resulting in a significantly increased DR of 237. DR is calculated as the ratio of $k_{avg}+$ over $k_{avg}$, according to Equation 1 (Long, D. M. & Uhlenbeck, O. C. Kinetic characterization of intramolecular and intermolecular hammerhead RNAs with stem II deletions. Proc. Natl. Acad. Sci. 91, 6977-6981 (1994))).

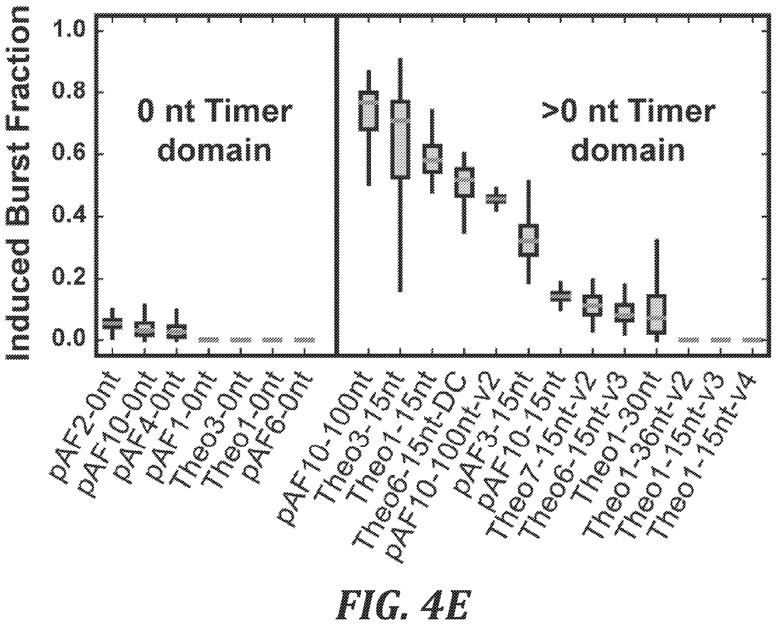

This holds true broadly, as for the twenty kinetically-controlled biosensors with low background cleavage, only those kinetically-controlled biosensors containing Timer domains had significant burst fractions in the presence of the ligand (FIGURE 4E). It is unclear why the differences are so stark, though it is possible that the additional time is necessary for the aptamer to fold into the ligand-competent state, or that there are specific geometric constraints on an elongating RNA molecule that require a minimal interdomain separation to allow the aptamer domain to be solvent accessible prior to structural rearrangement.

$$\text{Equation 1.} \qquad k_{avg.} = (f_{burst} * k_{burst}) + ((1 - f_{burst}) * k_{slow})$$

It is important to note that the presence of the Timer domain alone does not appear sufficient to gain access to co-transcriptional ligand binding and burst phase kinetics. Three of the devices containing Timers displayed no significant induced burst, suggesting that other factors, such as the rate of folding of the ribozyme domain likely play a role in kinetically-controlled biosensor function. For example, a long-lived folding intermediate between s4 and s5 could slow the effective cleavage rate of the kinetically-controlled biosensor below the threshold for burst cleavage, despite binding the target ligand co-transcriptionally.

MFEpath for screening RNA co-transcriptional folding

In order to design RNA aptamer-based switches that can function when produced *in situ,* or *in vivo,* it is necessary to be able to predict the relevant three dimensional structures that an elongating RNA molecule will adopt co-transcriptionally. As direct time-resolved prediction of three-dimensional structures of macromolecules (on the seconds scale) is currently computationally infeasible, it is necessary to abstract RNA three-dimensional structures to rapidly computable secondary structures. Due to the hierarchical folding of RNA, the secondary structure that an RNA molecule adopts dictates its accessible 3D folds, and it follows that a lack of the functional 2D structure precludes the formation of the functional 3D structure. This allows the use of 2D objective functions to drive the screening for functional 3D structures.

Although the directional transcription of RNA molecules complicates structure prediction, it also provides an opportunity to encode kinetic control through a series of rapid nucleotide addition and structural rearrangement steps. This enables the exciting engineering prospect that the ligand-binding and actuation reactions can be separated and tuned independently. Although there have been several algorithms developed to predict the co-transcriptional folding trajectories of RNA molecules, they predominantly are either unable to produce quantitatively accurate folding timescales, cannot be applied to long sequences, or are insufficiently transparent to allow for the type of quantitative analysis desired for our design-build-test-learn cycle. To fill this need, the MFEpath algorithm and computational framework for the predictable design of functional multi-state RNA devices was created. MFEpath works by screening RNA sequences for rapid co-transcriptional folding trajectories using secondary-structure prediction and Arrhenius-like interconversion kinetics (FIGURE 5A). The Arrhenius equation relates the activation energy of a reaction to exponential changes in reaction rate, and has been previously applied to the rates of RNA secondary-structural transitions. While empirical relationships between RNA structural rearrangement rate and barrier height have been described, and numerous barrier-prediction algorithms exist in the literature, there is a noted lack of broader computational tools for designing kinetically-functioning RNA devices using those calculated rate constants. While there are more algorithmically complex tools for the prediction of ensemble co-transcriptional folding, it is observed that the described MFEpath finds the ideal balance of computational efficiency and output granularity to be optimal for screening rapidly-folding kinetic RNAs.

Once kinetically-controlled biosensor candidates satisfying the thermodynamic objective functions were identified, the were screened for the ability to rapidly transition between the desired states during transcription. The candidate sequences possessed diverse *in vitro* co-transcriptional cleavage kinetics. as well as diverse MFEpath-predicted co-transcriptional folding characteristics. In order to predict coarse-grained folding trajectories during RNA transcription, MFEpath predicts the time to rearrange to the next MFE substructure using Arrhenius-like interconversion barrier heights ($\Delta G^{\ddagger}$), which correspond to the $\Delta\Delta G$ between the starting structure and the least stable structure along their refolding pathway. If rearrangement is calculated to be faster than the addition of the next nucleotide, structural rearrangement is allowed. If calculated to be slower, the transition is disallowed. the next base is added, and the analysis is performed for the new substructures. After the final base is added, the last barrier height ($\Delta G^{\ddagger}_{final}$) is used to predict the time needed for the RNA to convert from the co-transcriptional structure to the post-transcriptional structure. This analysis is performed both for the folding trajectory in the presence and absence of the target ligand.

In order to determine functionally-relevant screening cutoffs for the $\Delta G^{\ddagger}$ values, the apparent $\Delta G^{\ddagger}$ that would result in structural rearrangement kinetics of the same rate using Equation 2 was calculated. Assuming an elongation rate for T7 RNA polymerase of 230 nt/s. it was calculated that $\Delta G^{\ddagger}$ values of < 6.4 kcal/mol would occur faster than the addition of the next nucleotide. Assuming an upper limit on hammerhead ribozyme cleavage rate of 5 min$^{-1}$, it was calculated that $\Delta G^{\ddagger}$ values < 11.1 kcal/mol would result in structural rearrangements faster than ribozyme cleavage. The calculated $\Delta G^{\ddagger}_{final}$ values were then utilized to predict function within our kinetically-characterized kinetically-controlled biosensors (FIGURE 5B), as they should indicate how long a transcribed RNA will take to fold into its MFE structure post-transcription, and therefore whether the kinetically-controlled biosensors will undergo the rapid structural rearrangements necessary to display high DRs. For example, MFEpath predicts the Theo5-0nt kinetically-controlled biosensor to become kinetically trapped into the catalytically-active state regardless of the presence of the ligand, due to a large $\Delta G^{\ddagger}_{final}$. As expected, this device displays extremely high background cleavage, and therefore a negligible DR of 1.1. In contrast, in the absence of ligand, MFEpath predicts the Theo1-30nt kinetically-controlled biosensor will rapidly rearrange into the inactive conformation co-transcriptionally and, as predicted, the kinetically-controlled biosensor displays low background cleavage of 0.01 min$^{-1}$. However, in the presence of ligand. MFEpath predicts that the formation of the ribozyme domain will occur more slowly than the rate of ribozyme cleavage, and thus no rapid cleavage will be observed. This too holds true, as the device demonstrates a modest increase in the rate of non-burst cleavage, resulting in a DR of 5.6. Finally, MFEpath predicts that the Theol-15nt K-A will rapidly reach the desired structure states for both the ligand-dependent and -independent pathways. The device displays a low background cleavage rate, as well as rapid, ligand-dependent, burst phase cleavage resulting in an unprecedented DR of 237. These results illustrate how in order to attain large DRs, kinetically-controlled biosensor devices must possess small folding barriers for both the ligand-dependent and -independent folding trajectories.

$$\text{Equation 2.} \qquad \tau = 10^{\left(\frac{8}{11}*\Delta G^{\ddagger}\right)-7}$$

To validate that the calculated threshold values are accurate for the experimental conditions, all potential $\Delta G^{\ddagger}_{final}$ cutoffs were analyzed for the ability to split K-As into 'pass' and 'fail' categories. Kinetically-controlled biosensors possessing $\Delta G^{\ddagger}_{final}$ values below the cutoff should refold more rapidly than the competing reaction (i.e. transcript elongation or ribozyme cleavage). As the optimal barrier thresholds for screening kinetically-controlled biosensors are expected to be the barrier heights corresponding to the rate of the competing reaction, one should be able to map the best-performing barrier cutoff to the most physiologically-accurate barrier height. In order to compare cutoff values, 'performance increase' is defined as the ratio of the median cleavage rate ($k_{avg+}$ or $k_{avg-}$) for devices that pass the threshold, to the median of those that fail. For the plus-ligand screening, the one of the main peaks in performance increase occurs precisely at the *a priori* prediction (FIGURE 6A). For the minus-ligand screening, the maximum performance enhancement occurs slightly below our chosen value, which may either indicate a slight inaccuracy of our chosen threshold, or may suggest that there are additional factors that must be accounted for when predicting rearrangement from the s4 to s6 states co-transcriptionally (FIGURE 6B).

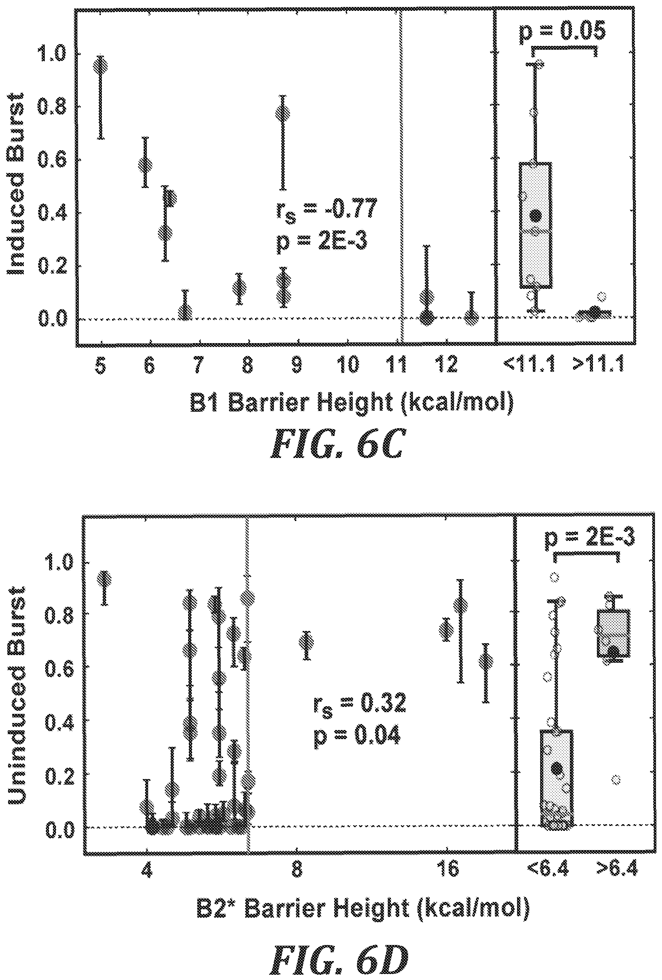

The calculated thresholds hold true when applied to the devices in aggregate as well. The kinetically-controlled biosensors that possess smaller -ligand $\Delta G^{\ddagger}_{final}$ values (B2* barrier) for aptamer deformation have smaller uninduced burst fractions (UBFs) (Spearman rho=0.34 , p=0.02), and all kinetically-controlled biosensors whose values lie above 6.4 possess large UBFs (FIGURE 6D). For kinetically-controlled biosensors with low background cleavage (to remove devices that attain burst cleavage due to very small B3 barriers), the devices that possess smaller +ligand $\Delta G^{\ddagger}_{final}$ values (B1 barrier) have larger IBFs (Spearman rho= -0.74 , p=0.004), and all whose values lie above 11.1 possess negligible IBF values compared to those with values lying below (FIGURE 6C). It is important to note that although many of the kinetically-controlled biosensors that pass MFEpath's -ligand screening possess small burst fractions in the absence of ligand, there remain a sizable number of such kinetically-controlled biosensors that possess large UBF values. This suggests that there are additional factors that dictate the B2 barrier that should describe the disclosed system.

Toehold-mediated strand displacement to reduce leakage

The most significant impediment to large-DR aptazymes is undesired cleavage in the absence of target ligand. More specifically, rapid, undesired cleavage is virtually incompatible with functional kinetically-controlled biosensors. As seen above, although MFEpath's barrier height predictions allow for identification of kinetically-controlled biosensors with a lower probability of possessing large uninduced burst fractions (UBF), they alone clearly do not explain the UBF in all cases. One likely reason for this is that even though a threshold is used as a pass-fail criterion for structural rearrangement, a predicted barrier height (and therefore reaction rate) nearly identical to the threshold, would result in a PASS within the MFEpath algorithm, but would result in an ~50/50 split between molecules that structurally rearranged, versus those that became kinetically trapped. Thus, in order to ensure extremely low UBF values, a structural rearrangement significantly faster than nucleotide addition is likely necessary.

Toehold mediated strand displacement (TMSD) is a well-known molecular mechanism in the field of DNA nanotechnology that can accelerate the rate of intermolecular strand exchange by up to $10^6$-fold (Zhang, D. Y. & Seelig, G. Dynamic DNA nanotechnology using strand-displacement reactions. Nat. Chem. 3, 103 (2011))

It has recently been utilized, with great success, to increase the effectiveness of trans-acting genetic RNA 'toehold switches'. By implementing the TMSD mechanism to accelerate the intramolecular structural rearrangement from state s4 to state s6 it may be possible to achieve extremely low background signal, and therefore unprecedented dynamic ranges. The kinetically-controlled biosensor molecular architecture is capable of utilizing the TMSD mechanism, as the P1 aptamer stem is analogous to the initial duplex, and the 5' end of the ribozyme domain acts as the invading strand (FIGURE 7A). Thus, the kinetically-controlled biosensor's rearrangement toehold initiates structural rearrangement by binding to its target.

There are three quantitative predictors of traditional intermolecular TMSD: 1. Stability of the toehold-target duplex. 2. The barrier height of the steps of the displacement reaction. 3. The concentration of the two species. By analogy, the effectiveness of intramolecular TMSD to enhance the rate of structural rearrangement in our system should be predictable from the stability of the toehold-target duplex, the free energy barrier height for the structural rearrangement, and relative volume that the toehold and target domains can explore.

To investigate whether kinetically-controlled biosensor UBF could indeed be predicted from the analogous TMSD parameters, the expected stability of the toehold-target duplex was calculated (FIGURE 7B). To do so, the ViennaRNA folding package's RNAeval algorithm with constraint folding to evaluate the stability of the toe-target duplex was utilized (Lorenz, R. et al. ViennaRNA Package 2.0. Algorithms Mol. Biol. 6, 26 (2011)). In order to account for frustrating structure formed internally to the toehold, any bases predicted to be base-paired prior to duplex formation were considered unable to contribute to the toe-target duplex. Toehold-target duplex stability displays a significant correlation with kinetically-controlled biosensor UBF (Spearman rho = 0.3 p = 0.05), but as expected does not satisfactorily explain all of the data.

Next, the barrier heights for the structural rearrangement that occurs post-duplex formation were characterized. As structural rearrangement from the active to inactive folding trajectory can occur at more than one transcriptional step, the B2 rearrangement barrier is the combination of all such possible barriers. Rearrangement barriers are only considered for steps in which the toehold and target are not engaged in frustrating structure, and therefore have a duplex stability less than zero kcal/mol. Barriers were combined utilizing Equation 3. This composite B2 barrier has a highly significant rank correlation with the observed UBF (Spearman rho = 0.57, p = 5E-5), and displays a stark threshold response just above 1.5 kcal/mol (Figure 6C). Although all kinetically-controlled biosensors with a B2 barrier smaller than 1.5 kcal/mol display UBF values less than 0.1, there remains some unexplained variation.

$$\text{Equation 3.} \qquad B2 = ln\left(\sum\nolimits_{n=branch}^{end} e^{-B2n}\right)$$

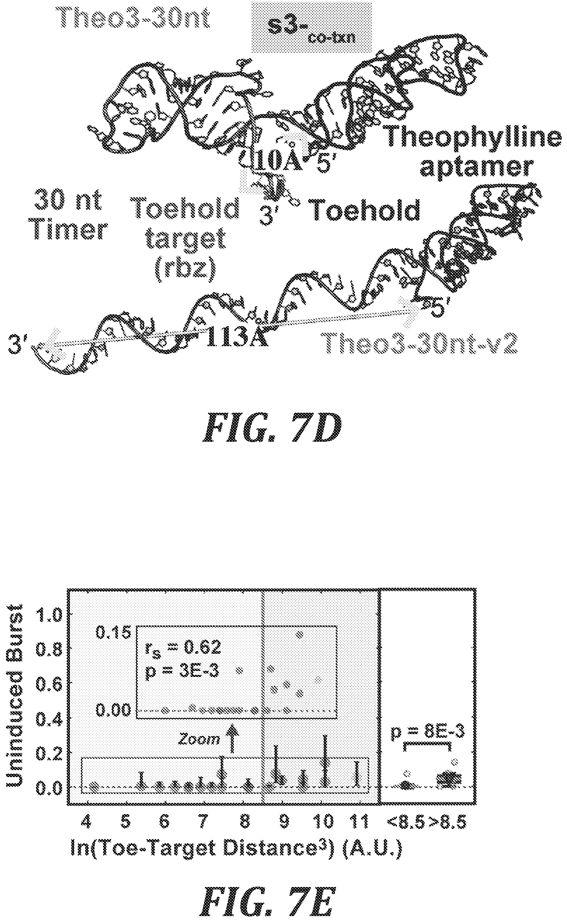

It was reasoned that if the kinetically-controlled biosensor structural rearrangement was proceeding via TMSD, that its rate would be proportional to the effective concentration of the toehold and target domains. As the TMSD reaction is intramolecular, the effective concentration should be inversely proportional to the 3-dimensional volume that the two domains can explore. Assuming that single-stranded RNA acts as a flexible linker, this volume should be proportional to the cube of the length of single-stranded RNA linker between the toehold and target. To predict this length, a simple algorithm for the coarse-grained estimation of the maximum linear distance between two nucleotides within a structured RNA was implemented. In effect, the algorithm counts the number of unstructured bases between the two, while skipping over any self-contained helical elements. For the kinetically-controlled biosensors possessing small B2 values, the remaining variation in UBF value correlated with this predicted distance (Spearman rho = 0.58 p = 0.006) (FIGURE 7E). For example, the Theo3-30nt K-A and the Theo3-30nt-v2 K-A are identical except for the sequence and structure of their 30 nt Timer domain. Theo3-30nt is expected to form a hairpin within its Timer domain that brings the toehold and toehold target into close proximity prior to structural rearrangement (FIGURE 7D). By contrast, the Timer domain of Theo3-30nt-v2 is expected to remain unstructured, allowing the toehold and target to explore a large volume, and decreasing the effective concentration of the two species. Thus, it appears that intramolecular TMSD is a viable and predictable mechanism for the enhancement of structural rearrangement, and reduction of ligand-independent burst phase cleavage.

Predictable K-A design

Ultimately any computational methodology to design aptazymes will only be useful if it can be relied upon to consistently produce devices with the large DRs necessary for downstream applications. To that end, the impact that the various design metrics described above have on the identification of high-DR kinetically-controlled biosensors was examined (FIGURE 10B). Interestingly, despite providing access to ligand-dependent burst phase cleavage, the addition of a Timer domain does little for the overall success of a device within our data set. This may be in part because the increased toehold-target distance that often accompanies a Timer domain, thus increasing the UBF observed. As expected, toehold stability (< 0 kcal/mol) plays a very large role in predicting the function of a candidate K-A. This effect becomes even more significant when B2 (which incorporates toehold stability) is utilized for screening (B2 < 1.5 kcal/mol). Interestingly, despite increasing the mean of the screened populations, through increased burst phase cleavage, a small B1 barrier (< 11.1 kcal/mol) decreases the median DR observed. This result may be somewhat idiosyncratic, as many of the devices with large B1 barriers also happened to have very low B2 barriers, and no measured UBF. Kinetically-controlled biosensors possessing toehold-target *natural log(linear distance$^3$)* (unitless) have improved mean and median values, though the differences are not overwhelming without first ensuring that the K-A possesses a small B2 barrier. When all of the screens are implemented simultaneously, the population possesses a median DR of ~10, and an extremely high mean DR of ~40. These are dramatic improvements over the population of K-As that fails even one of the criteria (p = 0.02). This seems to suggest that the screens are indeed synergistic, and will aid in the identification of high-DR K-As in the future.

The main area in which the prediction of kinetically-controlled biosensor behavior could improve is in the description of the B3 barrier height (FIGURE 10A). As the upper limit on cleavage is ~5 min$^{-1}$, a value only slightly faster than +ligand cleavage of the fastest K-As, the main available avenue for accessing DRs approaching the theoretical limit is to dramatically reduce the ligand-independent cleavage rate. As shown in Equation 1, $k_{avg}$ is the weighted average of the burst and slow rates multiplied by their relative fractions. As it appears that TMSD has allowed the design of kinetically-controlled biosensors with extremely low UBFs, the only avenue remaining to decrease $k_{avg}$ is to decrease $k_{slow-}$. Within the interpretation of the kinetically-controlled biosensor system, the height of the B3 barrier should dictate this $k_{slow-}$ rate constant. Indeed, it is observed that there is a statistically significant rank correlation between the computationally predicted B3 barrier height and the $k_{slow-}$ rate. However, it is apparent that the current calculations for B3 barrier will not be sufficient, as kinetically-controlled biosensors with predicted B3 heights varying by 6 kcal/mol display the same $k_{slow-}$.

The limitations of B3 prediction likely arise from two main issues: barrier height algorithm limitations, and structure state selection difficulty. As Findpath, the algorithm implemented in MFEpath, only considers direct refolding pathways (those that only contain base pairs in either the initial or final structure) it is likely to overestimate the barrier heights for real pathways, which usually undergo indirect refolding. Also, although the initial K-A design identifies the most thermodynamically stable structure that contains the ribozyme (s5), it is possible that the correct B3 barrier height would be one from s6 to a less-stable. but more rapidly accessed, structure that also contains the ribozyme. An additional current limitation is that the *in vitro* cleavage assay cannot statistically differentiate between cleavage rates slower than 10$^{-3}$ min$^{-1}$. Thus, to validate extremely low $k_{slow-}$ values, the duration of the assay itself will have to be extended. However, the ability to consistently design kinetically-controlled biosensors with DRs of 1000 would represent a significant improvement to the state of the art for most biosensing applications.

Kinetically-controlled biosensor ligand sensitivity tuning

One critical aspect of producing ligand-responsive switches is ensuring that they respond at concentrations that are relevant for subsequent applications. Designing switches that show switching behavior below cellularly-toxic, or insoluble, ligand concentrations has proved problematic in the past, and may be a principal reason that more aptazymes have not been identified to date. As such, it is critical that any methodology for the design of such switches allows the sensitivity to be rationally tuned. It is suggested that the variable-length hairpins observed between the aptamer domain and expression platform in natural riboswitches may exist in order to serve this purpose. By increasing the amount of time that the aptamer is available co-transcriptionally, through additional time the polymerase spends transcribing the hairpin, they may in turn increase the riboswitch's sensitivity to ligand.

To interrogate the impact of Timer domain length, and therefore the duration of the binding window, on kinetically-controlled biosensor ligand-sensitivity four additional kinetically-controlled biosensors were designed based on the pAF10-0nt device (FIGURE 9A). The kinetically-controlled biosensors contain identical sequences to pAF-10-0nt except for their Timer domains, which vary in both their length and sequence, and all possess DR values greater than 9. Two devices containing different 15 nt-long Timers were designed, as well as two with different Timers 100 nt-long. To determine the impact that these Timers had on device sensitivity, the described co-transcriptional cleavage assays were performed at six different concentrations of pAF for each device. To evaluate the ligand sensitivities of multiple devices the half maximal effective concentration ($EC_{50}$) was compared with respect to pAF. The *a priori* $EC_{50}$ value predictions were calculated from Equation 4, which assumes that ligand binding is a pseudo-first order irreversible process, that halts when the ligand binding window closes. In Equation 4. $k_{on}$ is the experimentally-determined association rate constant for the independently assayed aptamer-ligand pair, [L] is the concentration of ligand, $k_{pol}$ is the literature value for the *in vitro* elongation rate of T7 RNA polymerase at 37C, and nt is the nucleotide length of the inserted Timer domain.

$$\text{Equation 4.} \qquad EC_{50} = -\frac{\ln\ln\,(0.5)*k_{pol}}{k_{on}*nt_{timer}}$$

With the exception of the parental device, which does not have a Timer domain and therefore is not expected to bind co-transcriptionally, all of the kinetically-controlled biosensors possess B1 barriers well below the cutoff of 11.1 and are therefore expected to display ligand-dependent burst phase kinetics. As not all of the pAF10 kinetically-controlled biosensors displayed burst phase kinetics, and as very high $k_{burst}$ values can possess large errors due to manual pipetting limitations, it was decided to agnostically select whichever parameter ($k_{avg+}$, or IBF) provided the best $r^2$ value for each kinetically-controlled biosensor when fit to the 2-parameter binding Equation 5. The fit value for the maximum signal was used to normalize the data, which was subsequently fit to a 1-parameter binding Equation 6.

$$\text{Equation 5.} \qquad signal = \frac{signal_{max}*[L]}{[L]+EC_{50}}$$

$$\text{Equation 6.} \qquad f_{bound} = \frac{[L]}{[L]+EC_{50}}$$

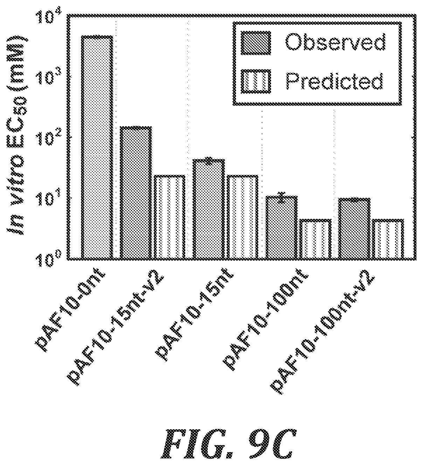

It was observed that as the length of the inserted Timer increased, so too did the measured $EC_{50}$ for the kinetically-controlled biosensor, resulting in fit $EC_{50}$ values spanning more than two orders of magnitude (8.4 mM to 4.4 M) (Figure 7B). Excitingly, the $EC_{50}$ values observed for pAF10-100nt and pAF10-100nt-v2 of 9.3 mM and 8.4 mM both fall within three-fold of our *a priori* expectation of 3.3 mM (FIGURE 9C). While somewhat less accurate, the $EC_{30}$ values observed for pAF10-15nt and pAF10-15nt-v2 of 40 mM and 142 mM fall near the prediction of 22 mM. No prediction for the $EC_{50}$ of the pAF10-0nt kinetically-controlled biosensor was made as, without a Timer domain, it is expected to bind pAF through post-transcriptional thermodynamic routes for which no straightforward $EC_{50}$ estimation methods exist.

It is relevant that the *a priori* $EC_{50}$ predictions were lower than observed for the four kinetically-controlled biosensors displaying burst phase kinetics. This suggests one of three main possibilities: 1. The characterized aptamer-ligand association rate measured in isolation is higher than within a K-A. This is certainly possible as weak, transient interactions with the rest of the nucleotides may reduce the availability of the aptamer for binding. 2. The literature value for T7 elongation rate is lower than the actual elongation rate in the experiment. While this is possible, the literature value utilized is already on the high end of those reported. 3. The binding window is shorter than the transcription time of the Timer domain. This again is very likely, as MFEpath predicts that the Timer domain is already partially transcribed by the time the aptamer domain becomes properly folded.

Although the idea of 'controlling any gene with any molecule' is an extremely ambitious and likely unattainable goal, it is believed that the work done to date illustrates the potential RNA switches hold in approaching that aim. It is believed that the performed and proposed research will provide significant advances to RNA design in a number of critical ways. First, any broadly-applicable computational strategy to design RNA switches is a major advance not only for the ability to design high performance RNA devices, but additionally for the lack of experimental expertise it demands from the end users. By moving the design labor from researchers to ever-cheaper computational resources. kinetically-controlled biosensors will be available to scientists who lack the technical proficiency (or resources) to perform the otherwise-necessary cellular screening experiments. Additionally, the design rules provide an excellent starting place for the computational design of other types of RNA switches. The extremely low background cleavage rates enabled by the intramolecular TMSD mechanism should enable applications in which leaky background signal cannot be tolerated, as in the described dCas9-based system. The demonstrated ability to tune the $EC_{50}$ of kinetically-controlled biosensors utilizing Timer domains provides another significant step forward for the field of RNA design, as it provides a framework for both the quantitative *a priori* predictions of switch sensitivity, as well as the rational tuning of switches custom-tailored to their application.

One class of genetic controller that has previously demonstrated in *E. coli* is that of an aptazyme-regulated expression device (aRED) (Carothers, J. M., et al. Model-Driven Engineering of RNA Devices to Quantitatively Program Gene Expression. Science 334, 1716-1719 (2011)). The mechanism utilized in an aRED is that of variable RNA degradation rates. When a ribozyme (or aptazyme) cleaves in the 5'-UTR of bacteria, the downstream gene is then terminated with a 5'-hydroxyl group, instead of a 5'-triphosphate. This has implications for the degradation rate, as an exonuclease recognizes and removes 5'-triphosphate groups. In the absence of such a group, the mRNA is degraded instead through endonucleolytic pathways instead. This slower degradation results in up to a 6-fold increase in the half-life of the RNA, and therefore steady-state protein expression level. While preliminary efforts to incorporate kinetically-controlled biosensors into an aRED showed promising results, subsequent analysis yielded unsatisfying and contradictory responses. One significant confounding factor is the adjacency of the aptazyme to the ribosome binding site. As ribosome binding site structure is known to be one of the primary factors in determining prokaryotic translation rates, any changes in ribosome binding site (RBS) structure that occur as a result of ribozyme cleavage or structural rearrangement are likely to have additional, unintended, impact on protein expression levels. Considering that the fold change of protein levels in response to an aRED is ~6, and the fold change of protein levels in response to changes in RBS structure are several orders of magnitude, it is very possible that the unintended effect will have a greater impact than the intended one.

Another piece of evidence that aptazymes may not be the ideal biochemical mechanism to utilize for genetic control is their surprising absence in nature. While riboswitches that dynamically regulate gene expression levels are ubiquitous in natural bacteria, and a natural ribozyme that uses a small molecule as a co-factor has been characterized, aptazymes where the self-cleaving ribozyme's cleavage activity is controlled by the binding state of an RNA aptamer domain have yet to be found. While there are several hypotheses as to why they are not more common, it appears that natural systems have found that other mechanisms are preferable, such as riboswitches that control the folding of a transcriptional terminator or RBS.


Example 2

This Example describes the engineering of antisense-ribosome binding site (AS-RBS) riboswitches, wherein the presence of the small molecule theophylline controls the expression of a fluorescent protein in *E. coli.* Borrowing from nature, transcriptional pause sites are incorporated into the switches to achieve unprecedented levels of sensitivity for their target molecule.


Introduction

Given the shortcomings of aptazymes as genetic controllers in bacteria, we decided to apply our molecular architecture and computational screening workflow to a new output domain better suited to regulating gene expression levels in *E. coli.* One of the most common mechanisms employed by natural riboswitches to control gene expression levels is to regulate the accessibility of the ribosome binding site (RBS) to incoming ribosomes. The 16S ribosomal subunit, which is composed of RNA, binds to the RBS through the base-pairing of complementary sequences. As a result, the occlusion of the RBS by competing base-pairs within its mRNA molecule is known to have a dramatic impact on how effectively the RBS and ribosome are able to associate. In turn, this regulates the rate at which the associated mRNA molecule is translated into protein, and the resulting steady-state protein concentration. This mechanism results in translation initiation rates that vary by several orders of magnitude, making them attractive for high performance biosensors.

While RBSs are an attractive output domain for synthetic aptamer regulation, they are not readily compatible with the described molecular architecture for the engineering of kinetically-controlled RNA biosensors. While the molecular architecture is designed to regulate the folding of structured RNA output domains that possess a closing stem, RBSs are nearly completely unstructured in their most active form. This represents a significant incompatibility, as the previously characterized objective functions and quantitative design metrics identified in the kinetically-controlled biosensor system would no longer apply. To address this issue, the RBS sequence was converted into an antisense-RBS sequence by appending a 5' extension to the RBS that is the reverse-complement of the wild-type sequence (FIGURE 12A). This creates an RNA domain with a closing stem, suitable for use in our molecular architecture, while also inverting the signal of the riboswitch, such that translation levels are maximized when the aptamer does not bind its target and minimized when it does bind its target (FIGURE 12B).

Due to a complex interplay of thermodynamic structure ensembles, and refolding kinetics, one particular struggle with the design of RNA biosensors acting under thermodynamic control is the difficulty of predicting the concentration at which one would expect the switch to respond. For this reason, it is possible that many functional RNA-based biosensors have been deemed non-functional due to a mismatch between the biosensor's actual $EC_{50}$, and the concentration that the researcher is able to assay the biosensor's performance under, whether due to solubility or other mechanistic incompatibility. This lack of predictability of response is exacerbated when the candidate biosensor is being expressed within a cell where cellular uptake of a molecule added extracellularly, and cellular metabolism of said molecule, create additional confounding factors that make the validation of novel biosensor design strategies increasingly difficult. For this reason, the theophylline aptamer has been a popular choice for the validation of new genetically-encoded biosensors. Although there is uncertainty regarding the quantitative relationship between extracellular and intracellular theophylline concentrations, it has been validated that theophylline can enter the cell and is not readily degraded by bacterial metabolism. However, despite these advantages, the biosensors that have been reported in the literature routinely have $EC_{50}$ values only slightly below the concentration where theophylline becomes toxic. In order to identify high performance biosensors, attaining high sensitivity to theophylline is therefore a high priority.

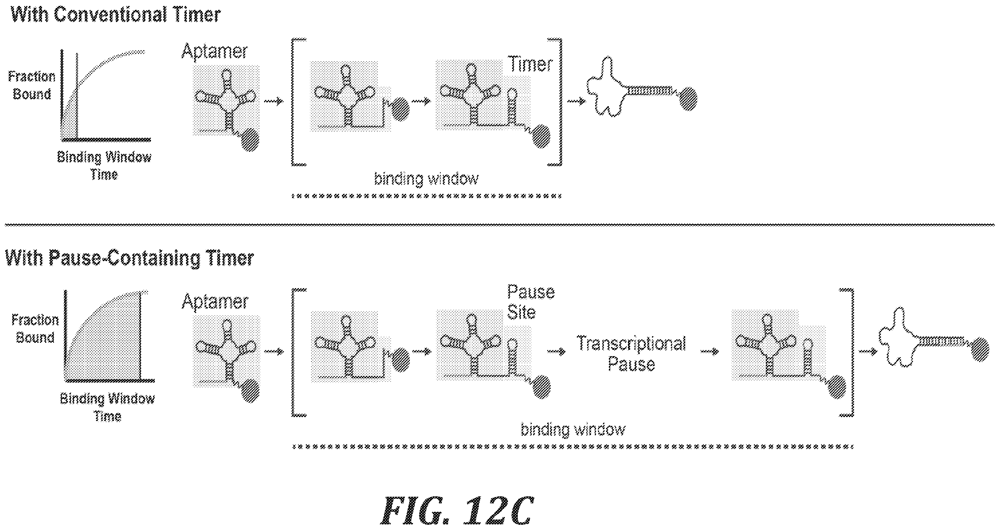

To validate this new application of the disclosed molecular architecture, and to benchmark the resulting biosensors against those reported in the literature, a theophylline-responsive AS-RBS riboswitch was engineered. While the kinetically-controlled biosensor molecular architecture allows one to make *a priori* predictions about biosensor sensitivity prior to being experimentally characterized, it gives no additional insight into the concentration of theophylline present intracellularly. Therefore, in order to give the disclosed biosensors the best chance to sense the potentially very low concentrations of theophylline within the cell, it was decided to take a lesson from nature and implement a transcriptional pause site within the Timer domain of the candidate biosensor. The TPP riboswitch family possesses a range of sensitivities to TPP, with $EC_{50}$ values ranging by over an order of magnitude. As in the AS-RBS riboswitches, the TPP riboswitch from the *ThiC* gene in *E. coli* regulates RBS accessibility such that translation levels are maximized when the aptamer does not bind its target and minimized when it does bind its target. Interestingly, between the aptamer and RBS, in the region analogous to the Timer domain in the kinetically-controlled biosensors, the riboswitch contains a hairpin that has been validated as a transcriptional pause site that causes the RNA polymerase to stall, with a half-life of nearly a minute, before continuing to transcribe the rest of the mRNA. As would be expected from a kinetically-controlled biosensor, this transcriptional pause activity has been demonstrated to increase the sensitivity of the biosensor to its target molecule. It was reasoned that in the correct folding context, this *ThiC* transcriptional pause site could be incorporated into the Timer domain of the AS-RBS riboswitches to significantly increase their sensitivity to theophylline and increase their overall performance (FIGURE 12C).

It is demonstrated here that the kinetically-controlled biosensor molecular architecture can also be applied to the design of translation-controlling AS-RBS riboswitch constructs that function within *E. coli*. Furthermore, it is demonstrated that a natural transcriptional pause site from *E. coli* can be incorporated into the Timer domain of an AS-RBS riboswitch, resulting in unprecedented sensitivity to theophylline. It was then demonstrated that the biosensor's high sensitivity and ligand activation ratio depend on the specific sequence of the Timer domain. Finally, it is shown that by screening synonymous codon variants of the 5' end of the output gene, increase of the expression levels was achieved without impacting the ligand activation ratio of the biosensor.

Methods

AS-RBS switch design

First the conventional RBS derived from the BglBrick vectors was combined with its reverse-complement appended 5' in order to create a hairpin expected to dramatically reduce translation initiation rate. A mutational operator was applied so that the AS-RBS itself was not a perfect hairpin in order to increase in silico pool diversity, and to prevent the 5' end of the switch from being identical to the RBS sequence itself, resulting in another site for translation initiation. The RBS calculator was used to ensure that the predicted translation initiation rate for the AS-RBS sequence was much lower than for the RBS without the antisense sequence appended (Salis, H. M. Chapter two - The Ribosome Binding Site Calculator, in Methods in Enzymology (ed. Voigt, C.) vol. 498 19-42 (Academic Press, 2011)). Screening for B1 barrier heights <= 7.8 kcal/mol was implemented, corresponding to the decreased rate of *E. coli* RNA polymerase nucleotide addition, relative to T7. Screening for B2 barrier heights <= 2.9 kcal/mol was implemented, corresponding to the same increase in barrier height as in B1. Toe-target distances were screened for values <= 8.5 arbitrary units, as for K-As previously. Screening for pathway convergence >= 0.7 was implemented.

*In vivo* Timer pool screening

In order to generate a pool of plasmids containing diverse Timer domains, a destination vector was constructed with two outward-facing SapI restriction sites placed into the computationally-designed switch candidate in the location where the Timer domain would be. Golden Gate plasmid assembly was used to insert a pool of short, double-stranded DNA fragments, generated through primer extension PCR. The Timer domain pool contained the *ThiC* transcriptional pause site flanked by variable positions.

The pools of plasmids were transformed into *E. coli* strain DH10B cells and plated onto plates containing the relevant antibiotic and grown at 37C for 16-24 hours. At that point, the brightest green colonies were picked and grown up in MOPS EZ-Rich defined media containing the appropriate antibiotics for 24 hours. The liquid cultures were then diluted 1:1000 into 400 uL of fresh media containing 0 mM or 1 mM theophylline. After an additional 16-24 hours, 150 uL of culture was read in a 96-well plate format in a Synergy HTX plate reader (BioTek) with gain 35.

Titration of modified pause-containing Timers

At least 2 biological replicates were grown up in 400 ul of MOPS EZ-Rich defined media containing the appropriate antibiotics for 24 hours. The liquid cultures were then diluted 1:1000 into 400 uL of fresh media containing a 2-fold dilution series of Theophylline starting at 2.5 mM. Cultures were grown for an additional 24 hours, and then 150 uL of culture was read in a 96-well plate format in a Synergy HTX plate reader (BioTek) with gain 35.

Synonymous codon pool screening

A destination vector containing outward-facing SapI restriction enzyme sites was assembled using standard molecular cloning techniques. A pool of short fragments, flanked by internal-facing SapI restriction enzyme sites was assembled, containing DNA containing the first 11 codons of the sfGFP gene, with positions varied such that synonymous codons, coding for the same 11 N-terminal amino acids of sfGFP could be identified (not shown). Partial doping was used to keep the pool to as many synonymous codon replacements as possible. The doped oligo to perform the assembly was ordered from IDT and assembled into a double-stranded fragment using primer extension. The assembled pool was transformed onto LB-Agar plates containing no theophylline. 48 of the brightest green colonies were picked, and grown in 400 μL of MOPS EZ-Rich defined media containing the appropriate antibiotics for 24 hours. The liquid cultures were then diluted 1:1000 into 400 μL of fresh media containing 0 mM or 1 mM theophylline. After an additional 16-24 hours, 150 μL of culture was read in a 96-well plate format in a Synergy HTX plate reader (BioTek) with gain 35.

Results and Discussion

Identification and characterization of a theophylline-responsive AS-RBS riboswitch

Utilizing the computational approach outlined in Example 1, a candidate AS-RBS riboswitch engineered to respond to theophylline was generated. The primary difference was that the barrier heights used for screening were increased corresponding to the slower rate of elongation for *E. coli* RNA polymerase compared to T7 RNA polymerase used in Example 1. In order to increase the sensitivity of candidate AS-RBS riboswitch constructs within a bacterial cell, a natural transcriptional pause site from *E. coli* was incorporated into the Timer domain of the AS-RBS riboswitches. Bacterial transcriptional pause sites resemble rho-independent transcriptional terminators, wherein a hairpin is followed by a 3' poly-T stretch. However, unlike transcriptional terminators, this poly-T stretch is not continuous, and is interrupted by other bases. As transcriptional terminators are known to have very rapid and specific co-transcriptional folding trajectories that enable them to function, it was reasoned that transcriptional pause sites would as well, and would therefore only function under very specific folding contexts. Without knowing what these precise folding contexts should look like, it was decided to screen for functional switches *in vivo,* as opposed to *in silico.* In the future, folding analysis of functional switch variants is believed to allow determination of the folding rules to predict pause site function purely computationally.

To perform the *in vivo* screening, scarless Golden Gate plasmid assembly was used to generate a pool of plasmids containing variable sequence within the Timer domain adjacent to the transcriptional pause site from the *ThiC* gene in *E. coli.* Individual colonies were picked into liquid media and grown in the presence and absence of theophylline. Colonies that showed the largest fold change in normalized GFP fluorescence were then isolated and sequenced. Interestingly at least one variant even demonstrated the ability to increase fluorescence in response to theophylline, counter to the intended mode of action. The isolated sequence displaying the largest fold-change in response to theophylline was named Theo_48. To characterize the sensitivity of Theo_48, its response was characterized at several different theophylline concentrations. Strikingly, it responded at very low concentrations, displaying an $EC_{50}$ of 47 μM (Figure 2A). Notably, this $EC_{50}$ is four times lower than has been observed from theophylline-responsive riboswitches in bacteria previously (FIGURE 13B). In fact, it is more than 10-fold lower than the average measured $EC_{50}$ from similar biosensors, and more than 10-fold lower than the only other engineered riboswitch known to exhibit kinetic behavior. This suggests that the pause site is indeed having the desired impact, resulting in extended co-transcriptional ligand binding windows, and enabling unprecedented sensitivity of the engineered biosensors.

To validate the role that the transcriptional pause site played in achieving the unprecedented sensitivity and high activation ratio of the Theo_48 switch, a series of Timer domain variants designed to reduce, or eliminate the duration of the transcriptional pause was created (FIGURES 14A and 14B). Point mutations designed to make the *ThiC* pause site look less like a transcriptional terminator were introduced, by mutating the poly-T stretch 3' of the hairpin within the pause site, or sections of the Timer domain or pause site were deleted. Introducing two point mutations within the poly-T stretch had essentially no impact on switch performance, but introducing 6 point mutations began to shift the $EC_{50}$ to higher values, while still maintaining the ligand activation ratio of the switch. This seems to suggest that the pause duration may be decreasing with increasing number of point mutations, resulting in a shortened ligand binding window, without otherwise impacting switch function. In the case of the deletions, all of the modified Timer domains resulted in reduced ligand activation ratios and significantly increased $EC_{50}$ values. This is largely expected in the cases of the Timer_Only and PolyT_Only constructs, where some, or all, of the pause site has been deleted (not shown). It is very interesting, however, that the ThiC_Only construct, in which the entire *ThiC* pause site is retained but only the variable sequence upstream of the pause site is removed, shows the lowest sensitivity of all the tested constructs. This suggests that the pause site alone is not sufficient for transcriptional pausing. To cause a significant transcriptional pause, the *ThiC* pause site must be surrounded by an appropriate sequence construct that enables it to fold correctly.

Increasing AS_RBS signal though screening of synonymous N-terminal codons

While the excellent sensitivity and ligand activation ratio of the Theo_48 construct represented a significant accomplishment for applying the kinetically-controlled biosensor design pipeline to another output domain, the maximum signal of the Theo_48 construct was substantially lower than the maximum signal observed from a positive control using the same RBS sequence and promoter. In fact, another candidate AS-RBS riboswitch designed to respond to pAF demonstrated a much higher maximum expression level, suggesting that there is no fundamental limitation on gene expression imposed by the architecture. Computationally predicted structures of the theophylline- and pAF responsive biosensor candidates suggested that the RBS in the theophylline construct maintained a larger degree of residual structure, even when the target molecule is absent, and that some of this residual structure was with the 5' end of the output sfGFP gene. In order to increase the maximum expression level of the Theo_48 construct, it was sought to decrease the degree of structure between the RBS and the 5' end of sfGFP. Inspired by the observation that rare codons are enriched at the 5' end of bacterial genes, likely to reduce unintended structure with the adjacent RBS. a similar strategy was used (Goodman, D. B.. et al. Causes and Effects of N-Terminal Codon Bias in Bacterial Genes. Science 342, 475-479 (2013) ).

Without changing any part of the Theo_48 biosensor itself, a pool of plasmids containing synonymous codons for the first 11 amino acid positions of sfGFP was created (not shown). Despite possessing different RNA structure, due to their divergent sequence, the pool variants should contain the same amino acid sequence, resulting in no modification to sfGFP when translated. Colonies exhibiting brighter green color when grown on LB-Agar plates were then picked. All the selected colonies indeed displayed increased GFP levels when grown in liquid culture (not shown). Interestingly, while each of the variants displayed different levels of fluorescence, the activation ratio in response to theophylline remained nearly identical in all of the isolates (not shown). This serves as additional evidence that the designed switches are behaving in a co-transcriptional, kinetic, manner where the behavior and identity of the sequence 3' of the switch do not impact the switch state once the co-transcriptional ligand binding window has closed.

Example 3

This example discloses Wayfinder, a computational algorithm to design and produce highly active gRNA sequences for CRISPR activation. Utilizing the described computational tools, the Wayfinder algorithm was developed to predict the activity of full-length and truncated scaffold RNAs (scRNAs), which are modified gRNAs used by the inventors to achieve CRISPR activation. Subsequently, the Wayfinder algorithm is compared to the state of the art for gRNA activity prediction tools. Finally, the sequence and structure conservation of the Cas9 binding handle was determined, allowing for the engineering of ligand-responsive scRNAs. The resulting scRNAs are useful for a wide array of applications, including advanced CRISPR-based applications, including CRISPR activation (CRISPRa) applications described herein as an exemplary output signal generation strategy.

Introduction

CRISPRa has emerged as a powerful new tool for the facile re-wiring of cellular metabolism in bacteria. The ability to simultaneously regulate the independent gene expression levels of multiple heterologous genes within the same cell provides tremendous opportunity for the combinatorial implementation of complex metabolic pathways. The ability to uniquely define the spacer sequence inserted within synthetic CRISPRa promoters, combined with the highly orthogonal nature of gRNA-mediated transcriptional activation, means that this system can theoretically be used to generate arbitrarily large networks of orthogonal transcription factors.

While many of the rules that govern CRISPRa activity have already been derived, there are no robust tools to predict the activity of gRNAs for CRISPRa applications based on their spacer sequence alone. To ensure high performance of CRISPR applications, it is critical to ensure that gRNA activity is optimal. Interactions between the variable spacer sequence of the gRNA with the constant parts of the gRNA sequence are often proposed as a cause for sub-optimal activity of gRNAs. To illustrate. FIGURE 15 schematically illustrates the differences between gRNAs that fold correctly versus incorrectly and the impact on resulting CRISPR activity. However, existing gRNA design tools, largely trained on eukaryotic gene editing data sets, have extremely poor predictive power over the spacer-specific variation in bacterial CRISPRa activity. For this reason, the inventors applied the lessons of RNA folding to determine whether or not CRISPRa activity levels are the result of the underlying biophysical relationships governing the folding of the gRNA folding.

In order to predict activity levels across different spacer sequences the computational RNA prediction tools described above are applied to identify a common set of biophysical parameters that correlate with CRISPRa activity. In addition to analyzing the effect of different spacer sequences on CRISPRa, the inventors also applied their computational tools to describe the effects on gRNA activity due to spacer truncations. Spacer truncations provide a convenient strategy to generate gRNAs with altered activity levels for CRISPR application. These gRNAs with altered activity can be delivered simultaneously to allow the implementation of different CRISPRa activities at multiple target genes, ideal for generating combinatorial libraries. In theory, spacer truncations represent the simplest solution to reducing gRNA activity. However, truncations often display non-monotonic behaviors and diverse activities at a given truncation length, making computational predictions of their activity attractive.

While the ability to predict the level of CRISPRa activity from the spacer sequence alone would represent a significant step forward for the forward engineering of CRISPRa-based systems, there are a number of applications for which the prediction of gRNA activity, when the non-spacer elements are varied, would be extremely useful. For example, as the number of scRNAs simultaneously expressed increases, so too does the genetic instability. The re-use of a large number of DNA components increases the likelihood of homologous recombination and therefore loss of desired system behavior. To combat this, creating modified sequence variants of the constant regions within gRNA, while retaining activity, has been a high priority. Additionally, the ability to develop a set of rules for the computational generation of functional Cas9-binding handle sequences would enable our kinetically-controlled biosensor design pipeline to be applied to the generation of ligand-responsive scRNAs for dynamic CRISPRa.

In this Example, the Wayfinder Algorithm is presented for computationally predicting the activity of an scRNA solely from its nucleotide sequence. See FIGURE 16. scRNAs are gRNAs with an additional ms2 hairpin added at 3', which recruit a transcriptional activator to the CRISPRa complex through binding to a MCP protein. See also FIGURE 25. It is demonstrated that Wayfinder can also predict the activity of scRNA truncations, allowing a wide range of expression levels to be achieved without preliminary *in vivo* validation. This allows for the forward engineering of complex systems in which the intermediate CRISPRa levels cannot be readily assayed. Next, the Wayfinder Algorithm is compared to other guide activity prediction tools, demonstrating that it dramatically outperforms the rest in our system. This suggests that the Wayfinder Algorithm provides a biophysical insight into guide activity that these other models do not, and it will provide important value to broader guide RNA design efforts. Finally, the sequence and structure-conservation rules of the Cas9-binding handle are determined in order to enable the engineering of ligand-responsive scRNAs.

Methods

Plasmid Assembly

Plasmids were cloned using standard molecular biology protocols. Plasmids expressing the CRISPRa components (dCas9, the activation domain and one or more scCRNAs) were constructed using a pl5A vector. *S. pyogenes* dCas9 (Sp-dCas9) was expressed using the endogenous *Sp.pCas9* promoter. The MCP-SoxS activation domain containing mutant SoxS was expressed using the BBa_J23107 promoter (parts.igem.org). scRNAs used the b2 design, in which where the endogenous tracr terminator hairpin upstream of MS2 is removed (Dong, C., et al. Synthetic CRISPR-Cas gene activators for transcriptional reprogramming in bacteria. Nat. Commun. 9, 2489 (2018) ).

The scRNAs, including the LR-scRNA were expressed using the BBa_J23105 promoter. Plasmids expressing target genes for CRISPRa were constructed using a low-copy pSC101** vector. mRFP1 and metabolic pathway genes were expressed from the weak BBa_J23117 minimal promoter (parts.igem.org) preceded by synthetic DNA sequences containing the CRISPRa target sites.

Wayfinder Algorithm for spacer activity prediction

A set of spacers was generated containing diverse sequence and structural properties. The only consistent rule was that the ms2 aptamer at the 3' end of the construct was predicted to fold correctly. This was done for two reasons. One reason was to eliminate any confounding cases where an scRNA does not fold in a way that allows it to readily bind to the MCP-SoxS activator, which could enable the scRNA to occupy the target DNA without the activator present, leading to unpredictable outcomes. The other reason was due to the ms2 hairpins resemblance to a transcriptional terminator, due to it being a hairpin immediately 5' of a poly-T stretch. This would potentially aid in transcriptional termination after transcription of the scRNA. as read-through due to transient misfolding could yield scRNA sequences with 3' tails of variable length, again confounding the results.

The scRNAs were expressed from a strong BBa_J23119 promoter. The scRNA-containing plasmids were transformed into *E. coli* strain MG1655 containing a second plasmid with the corresponding reporter gene. Three colonies for each double transformation were grown for 24 hours in 400 µL of MOPS EZ-Rich defined medium (Teknova) containing the appropriate antibiotic. Cultures were grown in 96 deepwell plates with rapid shaking at 37C. After 24 hours of growth, 200 µL of each culture was measured in a 96-well plate format in a Synergy HTX plate reader (BioTek) with gain 35.

Wayfinder predictions were generated using a combination of the MFEpath algorithm for co-transcriptional folding described above (Example 1; see FIGURE 17) or various algorithms from the ViennaRNA folding package version 2.3.5. MFEpath was used, with a mfthreshold parameter of 7.8 kcal/mol to predict the structures that the scRNAs would adopt during transcription, as well as estimates for how long the scRNAs would take to transition to their MFE structures. The mfthreshold parameter refers to the barrier height cutoff for structural rearrangements to occur more rapidly than an individual elongation step where a ribonucleotide is added to the growing RNA chain. The kinetic barrier was calculated by using the Findpath algorithm to predict the barrier height for the direct refolding pathway from the MFE structure or from the rapidly formed co-transcriptional structure to the structure wherein the Cas9-binding handle is correctly folded and the spacer is unstructured. The Wayfinder metric was calculated as a linear combination of the kinetic barrier and the Net Binding Energy. Net binding energy was calculated by calculating the RNA-RNA free energy of the spacer sequence binding to its reverse-complement sequence using RNAduplex in Vienna.

Handle seguence and structure conservation

Pools containing randomized bases within the Cas9 binding handle were transformed into *E. coli* strain DH10B along with a second plasmid containing the reporter gene. The reddest colonies on the plate were picked and grown up in LB for 24 hours in 14 mL culture tubes. After 24 hours, 200 µL of each culture was measured in a 96-well plate format in a Synergy HTX plate reader (BioTek) with gain 35. All cultures with RFU/$OD_{600}$ values greater than 90% of the wild type handle sequence's RFU/$OD_{600}$ value were subsequently submitted for sequencing.

For analysis of sequence and structure conservation, only the positions that were variable in a given sub-pool were considered. With the exception of the closing G-U (or U-G) bases, only bases represented in more than 10% of the total sequences were considered allowed bases. For structural conservation, only positions in which the pairing status (base-paired or not base-paired) matched the computationally-predicted pairing status at the corresponding position of the MFE structure of the wild-type sequence were considered. Only base-pair types (G-C, A-T, or G-U) that were represented in more than 10% of the total sequences were considered allowed base-pair types.

Using the sequence and structure conservation rules derived above, novel handles were generated and inserted into a common scRNA context with the J306 spacer sequence. The scRNAs were either expressed from a medium-strength BBa_J23105 promoter, or a strong BBa_J23119 promoter. The scRNA-containing plasmids were transformed into *E. coli* strain DH10B containing a second plasmid containing the reporter gene. Three colonies for each double transformation were grown for 24 hours in 400 µL of MOPS EZ-Rich defined medium (Teknova) containing the appropriate antibiotic. Cultures were grown in 96 deepwell plates with rapid shaking at 37C. After 24 hours of growth, 150 µL of each culture was measured in a 96-well plate format in a Synergy HTX plate reader (BioTek) with gain 35.

Results and Discussion

Wayfinder Algorithm for spacer activity prediction

In order to increase the predictability of the bacterial CRISPRa system a first step was to determine whether the kinetics of RNA folding were a significant cause of variation among scRNAs with different spacer sequences. It was observed that scRNAs with randomly-selected spacer sequences displayed wide variations in CRISPRa activity despite satisfying all of the known rules for basic CRISPRa activity in bacteria. To determine whether computational RNA folding predictions could be used to quantitatively predict scRNA activity, 39 scRNA constructs that varied only in the sequence of their 20-base spacer sequence and the corresponding 20-base target DNA sequence were built and tested within a synthetic CRISPRa promoter driving RFP expression. As expected, the tested scRNAs exhibited dramatic differences in reporter fluorescence, varying by almost 50-fold. The Wayfinder algorithm was applied to predict the barrier height (kinetic barrier) for conversion from the MFE structure (in which the spacer sequence may or may not form base-pairs with itself, the rest of the scRNAs, or both) to the structure in which the handle is correctly folded, the MS2 hairpin is correctly folded, and in which the spacer is unstructured (FIGURE 18A). Strikingly, the kinetic barrier parameter, alone, explained the majority of the variation that was observed amongst the tested scRNAs (FIGURE 18B). To investigate whether or not reducing the barrier value even further would result in higher activation, an additional 5 scRNAs containing little-to-no undesired structure were designed (FIGURE 24). All 5 scRNAs displayed high activation levels (FIGURE 24). The sigmoidal shape of the relationship between kinetic barrier and activation levels may explain some of the lack of increase, as it appears the response saturates at kinetic barrier heights smaller than 10 kcal/mol. Ultimately, the Way finder algorithm accurately predicted gRNA activity and was useful for designing gRNAs or scRNAs with high activity. See, e.g. FIGURES 19 and 24.

Next, the ability of the Wayfinder algorithm to predict scRNA activity was compared to the most commonly used guide RNA activity prediction tools from the literature (Moreno-Mateos, M. A. et al. CRISPRscan: designing highly efficient sgRNAs for CRISPR-Cas9 targeting in vivo. Nat. Methods 12, 982-988 (2015), Haeussler, M. et al. Evaluation of off-target and on-target scoring algorithms and integration into the guide RNA selection tool CRISPOR. Genome Biol. 17, 148 (2016); Doench, J. G. et al. Optimized sgRNA design to maximize activity and minimize off-target effects of CRISPR-Cas9. Nat. Biotechnol. 34, 184-191 (2016) ).

Interestingly, the other tools showed extremely poor correlation with the dataset created here (FIGURE 21). The Azimuth model displayed the best Spearman rank correlation with a value of 0.25, however this was much smaller to the value of 0.8 from the Way finder algorithm. FIGURES 22 graphically illustrates that the Wayfinder algorithm significantly reduces experimentation required to design gRNA and selecting target sites to achieve functional CRISPR activation as compared to available modeling approaches. Wayfinder significantly improved success rates in identifying effective gRNAs. This gain in efficiency is especially impactful when multiplexing CRISPR experiments with multiple complexes with a diversity of targets and, thus, a diversity of engineered gRNA sequences (FIGURE 23).

In order to implement different levels of transcriptional activation at a target promoter, without changing the DNA sequence of the target promoter, scRNAs with varied degrees of spacer truncation can readily be implemented. However, while truncating the spacer generally results in the reductions of CRISPRa activity, the response is often nonmonotonic. The length of the spacer sequence alone is a poor predictor of CRISPRa activity for scRNAs with truncated spacer sequences ($R^2$: 0.66). In order to improve predictions, the Wayfinder algorithm was applied in order to capture the decreased energetic favorability of binding with spacer truncations, and the net binding energy and kinetic barrier metrics were combined, yielding a unified metric (Way finder metric, defined above) with good prediction accuracy (FIGURE 20). One area for consideration is variability in transcription start site. Bacterial sigma70 promoters are known to have preference for initiating transcription from a G or A. When T or C are present at the +1 position, transcription can initiate from the -1 or +2 position instead, with additional impacts on the total transcriptional yields for a given promoter sequence. Truncations to bases other than G or C likely provide uncertainty in the actual sequence that is being transcribed, which in turn may have significant adverse effects on our ability to accurately predict their activity. Adding a constant 5' sequence element to standardize transcription initiation site and rate should enable the more accurate prediction of the activity of scRNAs with truncated spacers.

Handle sequence and structure conservation

In order for gRNAs to be compatible with the kinetically-controlled biosensor design pipeline, it is important to be able to vary the sequence of the dCas9-binding handle within the gRNA itself without interfering with any of the conserved sequence, or structure, elements necessary to bind to dCas9 effectively. To do so, the identity of the positions within the dCas9-binding handle were randomized, and then the resulting pool was screened for specific sequences that are capable of retaining the activity of the wild-type handle. By collecting sequence isolates the inventors began reconstructing the sequence- and structure-conservation rules necessary to generate highly functional alternative handles *de novo.* Due to the low probability of recovering a base-pair present in the wild-type handle, when a given position is allowed to be any base, a number of smaller pools were used in screening. Each of the smaller pools only varied a subset of the positions, in order to make sure that the odds of recovering function were greater than 1:1000, and would therefore be amenable to plate-based screening. After collecting 43 isolates possessing at least 90% of the wild-type activity, the conservation of base type, and base-pair type, at each position was determined by accepting only elements that occurred in more than 10% of the isolates (see TABLE 1). This screening approach differed from a previous iterative semi-rational design and identified similar, though not identical, sequence conservation rules.

After identifying the conservation rules, it was investigated how well the rules could predict functional handles *de novo.* Eleven novel handles were tested. All of the engineered handles except for one showed significant CRISPRa activity, while several retained nearly wild-type levels. The one handle with minimal activity appears to have been caused by folding issues other than the handle sequence, however, as when it is tested with a different spacer sequence, the activity increased significantly (not shown). In addition to testing the handle constructs under a high strength promoter (119), these were also tested when expressed from a weaker promoter (105), as it was suspected that the highest performing scRNAs were saturating the CRISPRa response. As expected, the difference in performance with the wild-type sequence was exacerbated for nearly all of the handles (not shown).

TABLE 1. Sequences of isolated Cas9-binding handle variants. All sequences that displayed at least 90% of the CRISPRa activity of the WT sequence when paired with the J3 spacer sequence. The pool from which the isolate was isolated is shown, with variable positions bold.

| Name | Isolate Sequence (SEQ ID NO) | Pool Sequence (SEQ ID NO) |
|------|------------------------------|---------------------------|
| sm_1 | CTTTTTGAGATCGAAATTCTAAGT AGAAAG (1) | NTTTNNGANNNNGAAANNNNAAG TNNAAAN (44) |
| sm_2 | GTTTAGGAAATTGAAAGGTTAAGT CTAAAA (2) | NTTTNNGANNNNGAAANNNNAAG TNNAAAN (44) |
| sm_3 | ATTTACGAACAGGAAAGGTAAAG TGTAAAT (3) | NTTTNNGANNNNGAAANNNNAAG TNNAAAN (44) |
| sm_4 | TTTTTCGATCTGGAAAAGGAAAGT GGAAAG (4) | NTTTNNGANNNNGAAANNNNAAG TNNAAAN (44) |
| sm_5 | CTTTATGAGGGTGAAATCCTAAGT GTAAAT (5) | NTTTNNGANNNNGAAANNNNAAG TNNAAAN (44) |
| sm_6 | ATTTAGGATTAAGAAATTTTAAGT CTAAAG (6) | NTTTNNGANNNNGAAANNNNAAG TNNAAAN (44) |
| sm_7 | CTTTATGAGGGTGAAATCCTAAGT GTAAAT (7) | NTTTNNGANNNNGAAANNNNAAG TNNAAAN (44) |
| sm_8 | TTTTTCGATCTGGAAAAGGAAAGT GGAAAG (8) | NTTTNNGANNNNGAAANNNNAAG TNNAAAN (44) |
| sm_9 | ATTTCCGATTTAGAAACAAGAAGT GGAAAG (9) | NTTTNNGANNNNGAAANNNNAAG TNNAAAN (44) |
| m_1 | TTTTTAGACCTCGAAAAAGGAAGT TAAAAT (10) | NTTTTNGANCTNGAAANAGNAAG TNAAAAN (45) |
| m_2 | ATTTTTGAACTAGAAAAAGTAAGT AAAAAG (11) | NTTTTNGANCTNGAAANAGNAAG TNAAAAN (45) |
| m_3 | TTTTTGGATCTTGAAATAGAAAGT CAAAAT (12) | NTTTTNGANCTNGAAANAGNAAG TNAAAAN (45) |

(continued)

| Name | Isolate Sequence (SEQ ID NO) | Pool Sequence (SEQ ID NO) |
|------|------------------------------|----------------------------|
| m_4 | ATTTTGGATCTAGAAATAGAAAGT CAAAAC (13) | NTTTTNGANCTNGAAANAGNAAG TNAAAAN (45) |
| m_5 | TTTTTCGACCTCGAAATAGTAAGT GAAAAG (14) | NTTTTNGANCTNGAAANAGNAAG TNAAAAN (45) |
| m_6 | ATTTTTGATCTAGAAATAGTAAGT AAAAAA (15) | NTTTTNGANCTNGAAANAGNAAG TNAAAAN (45) |
| m_7 | GTTTTTGAGCTCGAAAGAGCAAGT TAAAAT (16) | NTTTTNGANCTNGAAANAGNAAG TNAAAAN (45) |
| b_1 | GTTTTAGAGCTAGAAATAGCAAGT TAAAAT (17) | GTTTTAGNGCTAGAAATAGCNNNT TAAAAT (46) |
| b2 | GTTTTAGTGCTAGAAATAGCTCGT TAAAAT (18) | GTTTTAGNGCTAGAAATAGCNNNT TAAAAT (46) |
| b_3 | GTTTTAGTGCTAGAAATAGCTCGT TAAAAT (19) | GTTTTAGNGCTAGAAATAGCNNNT TAAAAT (46) |
| b_4 | GTTTTAGGGCTAGAAATAGCGTGT TAAAAT (20) | GTTTTAGNGCTAGAAATAGCNNNT TAAAAT (46) |
| b_5 | GTTTTAGCGCTAGAAATAGCATGT TAAAAT (21) | GTTTTAGNGCTAGAAATAGCNNNT TAAAAT (46) |
| b_6 | GTTTTAGAGCTAGAAATAGCGTGT TAAAAT (22) | GTTTTAGNGCTAGAAATAGCNNNT TAAAAT (46) |
| b_7 | GTTTTAGTGCTAGAAATAGCAAGT TAAAAT (23) | GTTTTAGNGCTAGAAATAGCNNNT TAAAAT (46) |
| b_8 | GTTTTAGGGCTAGAAATAGCATGT TAAAAT (24) | GTTTTAGNGCTAGAAATAGCNNNT TAAAAT (46) |
| b_9 | GTTTTAGCGCTAGAAATAGCATGT TAAAAT (25) | GTTTTAGNGCTAGAAATAGCNNNT TAAAAT (46) |

(continued)

| Name | Isolate Sequence (SEQ ID NO) | Pool Sequence (SEQ ID NO) |
|---|---|---|
| b_10 | GTTTTAGAGCTAGAAATAGCTGGT TAAAAT (26) | GTTTTAGNGCTAGAAATAGCNNNT TAAAAT (46) |
| r_1 | GCGTTATAGCTATTCTTAGCAAGT TAACGT (27) | GNNTTANAGCTANNNNTAGCAAG NTAANNT (47) |
| r_2 | GGATTAGAGCTAGTCATAGCAAGT TAATGT (28) | GNNTTANAGCTANNNNTAGCAAG NTAANNT (47) |
| r_3 | GCATTAGAGCTACAGATAGCAAG TTAATAT (29) | GNNTTANAGCTANNNNTAGCAAG NTAANNT (47) |
| r_4 | GAATTAGAGCTAGAGATAGCAAG TTAATCT (30) | GNNTTANAGCTANNNNTAGCAAG NTAANNT (47) |
| r_5 | GCATTAGAGCTACAGGTAGCAAG TTAATAT (31) | GNNTTANAGCTANNNNTAGCAAG NTAANNT (47) |
| r_6 | GAATTATAGCTATAGTTAGCAAGT TAATTT (32) | GNNTTANAGCTANNNNTAGCAAG NTAANNT (47) |
| r_7 | GCATTAGAGCTACTAGTAGCAAGT TAATTT (33) | GNNTTANAGCTANNNNTAGCAAG NTAANNT (47) |
| r_8 | GGTTTACAGCTAATTGTAGCAAGT TAAAGT (34) | GNNTTANAGCTANNNNTAGCAAG NTAANNT (47) |
| r_9 | GGTTTAGAGCTAAAGATAGCAAG TTAAAAT (35) | GNNTTANAGCTANNNNTAGCAAG NTAANNT (47) |
| r_10 | GAATTATAGCTAAGGGTAGCAAG TTAATAT (36) | GNNTTANAGCTANNNNTAGCAAG NTAANNT (47) |
| f_1 | GCATGAGAGCTAGAAATAGCAAG TTCGTGT (37) | GNNNNNNAGCTAGAAATAGCAAG NNNNNT (48) |
| f_2 | GGTATAGAGCTAGAAATAGCAAG TTTTACT (38) | GNNNNNNAGCTAGAAATAGCAAG NNNNNT (48) |

(continued)

| Name | Isolate Sequence (SEQ ID NO) | Pool Sequence (SEQ ID NO) |
|---|---|---|
| f_3 | GTTTTAGAGCTAGAAATAGCAAGT TAAAAT (39) | GNNNNNNAGCTAGAAATAGCAAG NNNNNNT (48) |
| f_4 | GTTTTAGAGCTAGAAATAGCAAGT TAAAAT (40) | GNNNNNNAGCTAGAAATAGCAAG NNNNNNT (48) |
| f_5 | GCTTCGGAGCTAGAAATAGCAAG TCGAAAT (41) | GNNNNNNAGCTAGAAATAGCAAG NNNNNNT (48) |
| f_6 | GCTTTTAAGCTAGAAATAGCAAGT AGAAGT (42) | GNNNNNNAGCTAGAAATAGCAAG NNNNNNT (48) |
| 20f_1 | GTTGGTGAGTTAGAAATAACAAGT ACCAAT (43) | GNNNNTGAGNNNGAAANNNCAAG TANNNNT (49) |

Example 4

This example discloses an exemplary approach to design synthetic promoters optimized for interaction with guide RNAs. The Wayfinder algorithm, discussed in Example 3, is employed to design gRNA-target sequences to enhance the precision and reliability of the gRNA constructs. This approach is further incorporated in the design of synthetic promoter regions for reporter genes. Elements of this work have been published in Fontana et al., Effective CRISPRa-mediated control of gene expression in bacteria must overcome strict target site requirements, Nature Comm. 11(1):1-11 (2020).

Several expression cassettes, each with different engineered promoter targets, can be selectively controlled, even in multiplex experiments. Further, it is shown that the scRNAs can be tuned for level of expression induced by manipulating the spacer length, providing for even more nuanced control of expression individually or even within the context of multiplexed reactions.

Results and Discussion

CRISPRa provides an exciting opportunity for directed gene expression and has many applications, such as engineered metabolic pathways and bioproduction. The Way finder algorithm is not only applicable to the design of highly active CRISPR guide RNAs, e.g.. for use in biosensors or CRISPRa scRNAs, but also for the targets of CRISPR guide RNAs. FIGURE 25 illustrates a scheme wherein a synthetic gene expression cassette is engineered to contain a promoter region that is optimized for binding by an scRNA construct. Upon binding, the transcription of a target gene, in this case a reporter gene, is activated. This design provides myriad features. The design enables independent targeting and programmability, where each distinct expression cassette target simply requires a unique target site for binding (e.g.. about 20 bases). As illustrated, PAM sites can be located at positions upstream of the transcription start site on the promoter (e.g., -83 to -79 or -73 to -69) on the non-template strand. PAM sites are NGG trinucleotides required for targeting dCas9 to a target sequence. The PAM site can also be positioned on the template strand following a similar scheme. Weak promoters can be used to ensure low background activity, if desired. Finally, each promoter uses a unique sequence between the target site and promoter to avoid repeating elements.

Using this scheme, a variety of assays were implemented to show proof of concept. As illustrated in FIGURE 26, a synthetic expression cassette was designed incorporating an scRNA target site (J306) and was exposed to a J306 scRNA or an scRNA with an irrelevant off-target gRNA sequence that cannot bind to the target site. As illustrated in FIGURE 26B, the promoter activity is demonstrated by detectable RFP signal, wherein binding of the scRNA to the J306 target site in upstream of the promoter resulted in 50X more expression than for an off-target sequence.

To illustrate the programmability of this CRISPRa approach and to demonstrate that promoter design can optimize performance of CRISPRa, a variety of changes were made to the scRNA spacer and cognate target sites (FIGURE 27A). The combinations of variant scRNA constructs and engineered target (reporter) expression cassettes were combined and assessed for expression activity. As illustrated in FIGURE 27B, the expression level of the target gene can be changed simply by changing the sequence of the gRNA target site in the promoter and expressing the matching scRNAs. Thus, the target sequence can be modified to contain unique promoter sequences that are activated only when matching scRNAs are present (e.g.. expressed in the same cell) and tune gene expression from the synthetic promoter. This can lead to a variety of precise regulation and control mechanisms to guide complex expression pathways in a cell, such as in metabolic production.

Next, a series of assays was conducted to characterize the design parameters of promoter sequences and their effects on CRISPRa activity.

It was determined that CRISPRa is sensitive to the strength of the promoter of the target gene. To evaluate whether the strength of the promoter affects CRISPRa, activation was tested on a set of fluorescent reporter genes with minimal promoters spanning a 200-fold range in basal expression level (parts.igem.org) (FIGURE 29). It was observed that the most effective gene activation was with a moderately weak J23117 promoter. With the weakest promoters, no activation was detected, even though their basal expression levels were only twofold weaker than the J23117 promoter. With stronger promoters. progressively smaller CRISPRa-mediated activation of gene expression was observed; the basal expression level increased, whereas the maximal, CRISPRa-induced expression remained roughly constant. Thus, the level of CRISPRa activity that can be achieved on a given gene may depend on the basal level of expression of its promoter.

CRISPRa was determined to be sensitive to the composition of the intervening sequence between the target site and the promoter of the target gene. To determine whether the sequence composition between the target site and the -35 site of the promoter of the target gene affects CRISPRa, a promoter library was constructed with randomized sequences in this intervening region. Single colonies from this library were analyzed and gene activation was observed with a broad distribution over a 27-fold range (FIGURE 30). Although most variant sequences could still be activated (more than twofold) with CRISPRa, the large variation in activity was unexpected because each reporter gene was driven by the same minimal promoter and contained the same scRNA target site. It was found that intervening sequences that give more effective CRISPRa tend to be more GC-rich, (rs = 0.42, p = 0.02). Nonetheless, these experiments indicate that the composition of the intervening sequence between the CRISPR-Cas complex and the minimal promoter is an important factor determining the level of CRISPRa.

CRISPRa was also determined to be sensitive to the position of the target sites. Assays demonstrated that CRISPRa is sharply dependent on single base shifts of the target site. An original hypothesis was that optimal target sites are located -60 to -100 bases upstream of the TSS was based on an experiment with scRNA sites spaced every 10 bases. To further test this hypothesis, the CRISPRa complex was targeted to a window from -61 to -113 at single base resolution. A reporter gene with five scRNA sites located at -61, -71, -81, -91, and -101 relative to the TSS was used, and 1-12 bases upstream of the -35 site was/were inserted to generate a set of reporter genes that allowed the CRISPRa complex to target every possible distance in the optimal targeting window. Using this reporter gene set, it was found that shifting the target site by 1-3 bases caused significant decreases in activation (FIGURES 28A and 28B). Shifting the target site further by 4-9 bases decreased expression to levels nearly indistinguishable from background. At 10-11 base shifts, corresponding to one full turn of a DNA helix, gene expression increased again. This periodic positional dependence of CRISPRa extended over the entire -60 to -100 window, with the strongest peaks centered at -81 and -91 and smaller peaks centered at -102 and -70. There was no recovery of activity when the site at -101 is shifted to -111, outside of the -60 to -100 window. This sharp periodic relationship suggests that the criteria for effective target sites are quite stringent, and that both distance and relative periodicity to the TSS are critical factors.

Notably, the distance to the TSS was not the sole determining factor for CRISPRa-mediated expression level. Sites that overlapped at the same distance, such as the original -81 site and the -71 site shifted by 10. did not give the same gene expression output (FIGURES 28A and 28B). These discrepancies could arise from differences in the activity of the gRNAs targeting each site with different 20 base spacers (FIGURE 18B) or from the effect of different intervening sequence composition between the scRNA target site and the minimal promoter (FIGURES 29 and 30).

Because it was demonstrated that sequence composition can have unexpected effects on CRISPRa (see, e.g., FIGURES 29 and 30), it was tested whether the periodicity of CRISPRa was similar in different sequence contexts. Comparable periodic phase dependence was obtained when different nucleotide sequences were used to shift the scRNA target site, and when the bases were inserted at a different location in the promoter (not shown). Similar results were also obtained when the base shift experiment was performed with a reporter that had a different 5' upstream sequence (not shown) or where the minimal BBa_J23117 promoter was replaced by endogenous aroK promoter (not shown). Further, the sharp positioning dependence was observed when targeting the template or non-template strand of the reporter (not shown). Finally, one possible confounding effect could arise if the basal expression level of the reporter gene changes when bases are inserted, which can affect the efficacy of CRISPRa (FIGURE 29). However, it was observed that basal expression from the original reporter and the +5 base shifted reporter were indistinguishable (not shown). Together, these experiments confirm that bacterial CRISPRa is sensitive to periodicity in multiple different sequence contexts.

The finding that CRISPRa displays the same ~10 base periodicity as the DNA helix suggests that the angular phase of the CRISPRa complex relative to the minimal promoter is critical for effective activation. The disclosed bacterial CRISPRa system requires a direct interaction between the SoxS activation domain and RNA polymerase, and this interaction appears to be highly sensitive to both the distance and relative phase of the target site to the minimal promoter. The sharp phase dependence of CRISPRa may be a general feature of transcriptional regulation in *E. coli*. The native SoxS protein and other transcription factors such as CAP and LacI have restrictive positioning requirements that correspond to DNA periodicity: this result was confirmed with an endogenous SoxS reporter (not shown). In practice, this periodic behavior means that effective target sites must be located at one of the narrow peaks of activation within the optimal distance range. These stringent requirements suggest that targeting endogenous genes will be extremely challenging. There is ~1 PAM site every 10 bases in the regions upstream of endogenous promoters in *E. coli* (not shown), and the likelihood that a PAM site will be located at the appropriate phase within a 10 base window is low.

It was demonstrated that a dCas9 variant expands the range of targetable sites. Considering there is a limited number of genes with an appropriate NGG PAM site at precisely the optimal position upstream of the promoter, it was attempted to expand the scope of targetable PAM sites for CRISPRa. A recently characterized dCas9 variant, dxCas9(3.7), that has improved activity at a variety of non-NGG PAM sites including NGN, GAA, GAT, and CAA6 was used. Reporter plasmids were generated by replacing AGG PAM sites with alternative PAM sequences and delivered a CRISPRa system with dxCas9(3.7) to target these reporters. dxCas9(3.7) maintained the ability to target the AGG PAM and showed significantly increased levels of activation at alternative PAM sites compared to dCas9 (FIGURE 31). Activation levels varied with different PAM sites and correlated well with dxCas9(3.7) activity previously reported in human cells (not shown). dxCas9(3.7) showed similar distance and phase dependent target site preferences as dCas9 (not shown), but its expanded PAM scope makes it more likely that an arbitrary gene will have a targetable PAM site at an effective position. Bioinformatic analysis of the sequences between transcriptional units in *E. coli* revealed that there are on average 6.4 times more dxCas9(3.7)-compatible PAM sites than NGG PAM sites (not shown). Accounting for the fact that dCas9 has some activity at non-NGG sites6 (FIGURE 31), there are still on average ~2.2-fold more dxCas9(3.7)-compatible PAM sites than dCas9-compatible PAM sites (not shown).

To demonstrate the utility of dxCas9(3.7) for CRISPRa at sites inaccessible to dCas9, a reporter plasmid was constructed that contains an AGG PAM site at the original position with maximum CRISPRa activity and an AGT PAM five bases downstream. Using this reporter, it was observed that both dCas9 and dxCas9(3.7) are effective for CRISPRa at the optimally positioned NGG PAM site, but neither is capable of activating the AGT PAM site, which is five bases out of phase from the optimal site. Five bases were then inserted into the reporter to shift the AGT PAM site into the peak activation range. With this reporter, neither dCas9 nor dxCas9(3.7) can activate the NGG PAM site, which is now out of phase, dxCas9(3.7) was now able to effectively activate the AGT PAM site, and dCas9 was ineffective at this site (not shown). This result confirms that dxCas9(3.7) is able to activate optimally positioned target sites that are inaccessible to dCas9. It is expected that this behavior will be effective at many σ70-family promoters, and a recent report demonstrated a similar behavior of dxCas9(3.7) at σ54-dependent promoters.

These data demonstrate that synthetic promoter sequences can be used to program specific implementations of CRISPRa of desired genes. CRISPRa is demonstrated to be highly sensitive to the position of the target site with respect to the target gene start site and promoter. Minimal promoters can be modified to set the expression level of the promoter and relatively weak promoters provide a maximum dynamic range. Unique sequences can be placed between the target site and the promoter and screen for facilitation of high levels of CRISPRa induced expression. Finally, noncanonical PAM sites can be incorporated into the promoters facilitating use of endonucleases other than Cas9.

Engineered metabolic or signaling pathways may rely on the operation of multiple, distinct engineered CRISPRa targets, including uniquely optimized promoters, and corresponding scRNAs to induce specific and controlled expression of the targets. Accordingly, the next step was to demonstrate that CRISPRa could be multiplexed by maintaining the selectivity and tunability of the activation. In one proof of concept assay, illustrated in FIGURE 32A, three CRISPRa target expression cassettes were developed, each with a unique target site, and exposed to distinct scRNAs specific for each target site. It was shown that activation of gene expression via CRISPRa only occurred with the matching scRNA. See FIGURE 32B.

It was also demonstrated that expression induced at each promoter could be tuned by truncating the matching scRNA from the 5' end, utilizing the same approach described in Example 3. See FIGURE 33. Briefly, the expression cassettes used in FIGURES 32A and 32B were again targeted with a series of corresponding scRNA constructs that this time had progressively shorter spacer lengths. As shown in FIGURE 32, the RFP reporter signal representative of gene expression levels diminished as the spacer length decreased from 19 bases. Moreover, it was shown that in a multiplex scenario, the gene expression for each of multiple unique promoters could be uniquely and independently tuned. This approach can be used to rapidly implement combinatorial variations in the expression of multiple genes by combining promoters with unique target sites and use of appropriately truncated scRNAs (FIGURE 34A). As shown, a representative library of scRNAs truncated to different degrees was created with all possible combinations of four tuned expression levels for three different target reporter genes. All possible combinations of the various library members were exposed to the target reporter construct incorporating engineered target promoter sequences, resulting in appropriate levels of high, medium, low, or no expression of the corresponding reporter gene (FIGURE 34B). This demonstrates that the scRNA/engineered promoter target sequences can be independently tuned for intensity of gene expression, even when implemented in a multiplexed context.

Example 5

This example describes an application of using the enhanced expression constructs and scRNAs to implement ligand-dependent control of CRISPRa expression using desired metabolites. An exemplary workflow to achieve this goal is illustrated. First, *in vitro* selection is performed to identify a novel RNA aptamer that binds to the metabolite of interest. Next, a CRISPRa-regulated metabolic pathway is engineered for the biosynthesis of the metabolite in cultured cells, e.g., *E. coli.* Finally, using the novel aptamer, kinetically-controlled ligand-responsive scRNAs (LR-scRNA) are engineered to be able to sense the production of the target metabolite from the engineered metabolic pathway. See FIGURES 35A and 35B.

Introduction

Example 2 discloses the applicability of the disclosed computational biosensor design pipeline to the regulation of gene expression in *E. coli,* through the engineering of AS-RBS riboswitches. This Example addresses engineering of genetically-encoded biosensors able to respond to the production of a target molecule being synthesized within the same cell. In order to develop a robust system for the engineering and screening of diverse metabolic pathway variants, the lessons learned about the engineering of kinetically-controlled RNA biosensors were combined with the lessons learned about optimizing scRNAs for CRISPRa in a single application. To do so, a class of kinetically-controlled RNA biosensors known as ligand-responsive scRNAs (LR-scRNAs) were developed. These LR-scRNAs utilize the Cas9-binding handle of an scRNA as the output domain of our kinetically-controlled biosensor molecular architecture. As the handle is critical for the formation of the scRNA-dCas9 complex it was hypothesized that the selective deformation of the handle will result in selective formation of the CRISPRa complex, and therefore ligand-responsive CRISPRa activity.

One significant benefit of LR-scRNAs as an alternative to AS-RBS Riboswitches is the direction of the response. As metabolic pathways express large amounts of burdensome enzymes, cellular fitness can be dramatically impacted by their production, often leading to genetic instability and suppression of the expression levels of heterologous genes. In turn, many metabolic pathway variants will express significantly lower levels of a reporter protein in response to excess burden from the pathway. In the case of an AS-RBS riboswitch this result could be falsely interpreted as a reduction signal due to the biosensor's regulation of the expression level of the reporter gene. While AS-RBS Riboswitches decrease translation levels upon detection of the target molecule, LR-scRNAs increase transcription levels in response. Thus, any increase in output gene expression is very unlikely to occur spontaneously, and furthermore gives an indication that the underlying genetics necessary for gene expression remain intact. In addition, an aptamer-regulated scRNA would not only enable regulation of a fluorescent protein for extracellular quantification of intracellular metabolite concentration, but would also allow implementation of complex genetic networks in response to those metabolite levels. For example, recent efforts have demonstrated that incoherent feed forward network motifs can be realized using CRISPRa and CRISPRi components.

In order to determine whether or not the molecular architecture applies to the engineering of LR-scRNAs able to respond to the *in vivo* production of biosynthetic products, it was first decided to validate the biosensors using a well-studied aptamer that would allow extracellular addition of the target molecule. To do so theophylline responsive LR-scRNAs were engineered. Bacterial transcriptional pause sites that resulted in highly-sensitive AS-RBS riboswitches (Example 2) were implemented to determine if the same sensitivity could be achieved, despite an entirely different mode of action. Ultimately, the production of theophylline-responsive scRNAs able to modulate CRISPRa activity in a highly-sensitive and dose-dependent fashion was demonstrated.

Methods

Computational screening for ligand-responsive scRNA switch candidates

Candidate LR-scRNAs were screened using the computational methods established with kinetically-controlled RNA biosensors and AS-RBS riboswitches. Elongation barrier heights of 7.8 kcal/mol were used for MFEpath predictions of co-transcriptional folding, corresponding to the rate of E. coli RNA polymerase elongation. Screening for B1 barrier heights <= 7.8 kcal/mol was implemented. Screening for B2 barrier heights <= 2.9 kcal/mol was implemented. Toe-target distance, calculated as ln(linear distance3), were screened for values <= 10.5 arbitrary units. Screening for pathway convergence >= 0.7 was implemented. The Cas9 binding handle of the scRNAs was treated as the output domain in order to define the Overhang, and Stem sequences within the molecular architecture. In order to generate enough diversity to find satisfactory computational solutions, the Cas9 binding handle was varied, using the sequence and structure conservation rules from Example 3. In order to optimize the likelihood of identifying sequences that were simultaneously good switches, and high-performing scRNAs, the Linker sequence was considered to be the 3' end of the spacer. The remaining 5' bases of the spacer were subsequently considered to be part of the Timer domain, and the entire switch (containing a full 20-base spacer) was re-screened using the same screening metrics.

In addition to the conventional switch screening metrics outlined in Example 1, the candidate switches were also screened for their ability to act as high-functioning scRNAs when the target molecule is bound to the aptamer domain. To do so, the aptamer domain was constrained and the candidate LR-scRNAs were screened using the previously established computational thresholds for highly-functional scRNA spacers. The following screening thresholds were applied: Net binding energy <= -25.0 kcal/mol, handle fraction >= 0.5, folding barrier < =10.0 kcal/mol, and folding barrier >=20.0 kcal/mol when evaluated without constraining the aptamer domain.

Once computational solutions were identified, Timer pools containing the *ThiC* transcriptional pause site were inserted 5' of the spacer sequence, and subsequently screened for performance. Colonies with low leak, corresponding to those without red coloring when plated on LB-Agar plates lacking the target molecule, were grown for 24 hours in 400 $\mu$L of MOPS EZ-Rich defined medium (Teknova) containing the appropriate antibiotic. Cultures were grown in 96 deepwell plates with rapid shaking at 37C. After 24 hours of growth, the cultures were diluted 1:100 into fresh media. The media contained varied concentrations of theophylline. After 24 hours of growth, 150 $\mu$L of each culture was measured in a 96-well plate format in a Synergy HTX plate reader (BioTek) with gain 35.

Results and Discussion

Development of Theophylline-Responsive scRNAs

In order to demonstrate that the molecular architecture could be used to measure the concentration of biosynthetic products, a first step was to validate that CRISPRa activity could be regulated in response to a membrane-permeable small molecule added to the cell culture media. To do so, scRNAs were computationally designed to be controlled by the binding state of the theophylline aptamer. In the presence of theophylline, the scRNA should fold correctly, giving rise to an increase in CRISPRa activity (FIGURE 36A-36C).

As in the case of AS-RBS riboswitches (Example 2), candidate switches were first designed *in silico,* and then subsequently a Timer pool containing the *ThiC* transcriptional pause site was inserted. The subsequent plasmid pool was screened using the plate-based method described previously. The computational screening yielded two initial candidate switches, Theo-1 and Theo-2, that were expected to produce high ligand activation ratios, and large maximum signals, coupled with the characteristic high sensitivity observed in AS-RBS riboswitches containing a pause site. In initial screening, both switches produced at least 2-fold increases in RFP levels when theophylline was added to the media (FIGURE 37A). Interestingly, the Theo1-derived sequence D5 showed a significant increase in RFP levels at sub-millimolar theophylline levels, unlike the rest of the isolated switches. When characterized at lower theophylline concentrations, it was determined that Theo1 D5 had an $EC_{50}$ of 42 $\mu$M, which is essentially the same as the 47 $\mu$M $EC_{50}$ that was observed for the Theo-48 AS-RBS riboswitch (FIGURE 37B). Considering the unprecedented sensitivity to theophylline in a bacterial host, as well as the shared transcriptional pause sequence, this result corroborates the hypothesis that the ThiC transcriptional pause site is enabling the kinetically-controlled biosensors to access increased sensitivities through increases in the co-transcriptional ligand binding window. While the overall activation ratio remained somewhat low in the theophylline-responsive isolates, this demonstration was sufficient to confirm that the molecular architecture could be applied to the regulation of scRNA activity at low concentrations of target molecule.

Finally, it is confirmed that the introduction of the transcription pause site in the Timer domain of the LR-scRNA biosensors increase the sensitivity of the biosensors for the corresponding ligand. See FIGURE 38. As with the AS-RBS riboswitches, the use of pause sites increases the transcription time for the LR-scRNA biosensors allowing more time for ligand binding. This additional time permits a binding window by >10s, which is significantly greater than what would be incurred simply by implementing longer Timer domains. Accordingly, there is more opportunity for ligands to bind and alter the state of the biosensor to an "active" state.

The development and validation of theophylline-responsive LR-scRNAs able to sense extracellularly added theophylline within *E. coli* demonstrates the feasibility of sensing intracellularly-produced metabolic products. This is because molecules that enter into the cell, such as theophylline, and molecules produced within the cell should be indistinguishable to the genetically-encoded LR-scRNAs. Combining LR-scRNAs, which act on transcription, with AS-RBS riboswitches, which act on translation, opens up the opportunity to program complex logical responses to the concentration of multiple target metabolites within a metabolic pathway.

While illustrative embodiments have been illustrated and described, it will be appreciated that various changes can be made therein .

**Claims**

1. A kinetically-controlled RNA biosensor construct, comprising from 5' to 3':

a sensor domain that specifically binds to a ligand of interest; and
an output domain configured to modulate a detectable output signal when folded into an active conformation;
wherein the output domain, when transcribed, folds into the active conformation when the sensor domain is bound to the ligand of interest, and wherein the output domain, when transcribed, folds into an inactive conformation when the sensor domain is not bound to the ligand of interest,
wherein the sensor domain comprises:

an aptamer domain, wherein the aptamer domain comprises, from 5' to 3', a stem sequence, a linker target sequence, an aptamer subsequence, and a stem target sequence; and
an overhang sequence 5' of the aptamer domain and, optionally, a linker sequence 3' of the aptamer domain; and

wherein the output domain comprises, from 5' to 3':

a stem target sequence,
an overhang target sequence, and
an output subsequence; and

wherein the overhang sequence of the sensor domain is the reverse complement of at least a portion of the overhang target sequence of the output domain.

2. The kinetically-controlled RNA biosensor construct of claim 1, wherein the sensor domain comprises the linker sequence.

3. The kinetically-controlled RNA biosensor construct of claim 1, wherein:

the stem sequence of the sensor domain is the reverse complement of at least a portion of the stem target sequence of the sensor domain and is the reverse complement of at least a portion of the stem target sequence of the output domain; and/or
the linker sequence of the sensor domain is the reverse complement of at least a portion of linker target sequence of the sensor domain.

4. The kinetically-controlled RNA biosensor construct of claim 3, wherein the linker target sequence is the reverse complement to a portion of the aptamer subsequence, optionally wherein the portion of the aptamer subsequence is a discontinuous portion.

5. The kinetically-controlled RNA biosensor construct of claim 3 or claim 4, wherein when the sensor domain is bound to the ligand of interest the stem sequence of the sensor domain is hybridized to the stem target sequence of the sensor domain thereby permitting folding of the output domain into the active conformation.

6. The kinetically-controlled RNA biosensor construct of claim 3 or claim 4, wherein when the sensor domain is not bound to the ligand of interest the overhang sequence of the sensor domain is hybridized to the portion of the overhang target sequence of the output domain, the stem sequence of the sensor domain is hybridized to the portion of the stem target sequence of the output domain, and/or the linker target sequence of the sensor domain is hybridized to the portion of the linker sequence in the sensor domain, thereby permitting folding of the output domain into the inactive conformation.

7. The kinetically-controlled RNA biosensor construct of claim 6, wherein at least two of the overhang sequence of the sensor domain, the stem sequence of the sensor domain, and the linker target sequence of the sensor domain form a continuous helix stem structure when hybridized to at least a portion of the overhang target sequence of the output domain, a portion of the stem target sequence of the output domain, and a portion the linker sequence of the sensor domain, respectively, thereby permitting the output subsequence of the output domain to fold into the inactive conformation.

8. The kinetically-controlled RNA biosensor construct of any of claims 1 to 7, wherein:

the overhang sequence is between 0 and about 15 nucleotides in length; and/or
the stem sequence is between 0 and about 15 nucleotides in length; and/or
the linker sequence is between 0 and about 15 nucleotides in length.

9. The kinetically-controlled RNA biosensor construct of any of claims 1 to 8, further comprising a timer domain disposed between the aptamer domain and the linker sequence of the sensor domain, wherein the timer domain has a length up to about 150 nucleotides in length, optionally wherein the timer domain further comprises at least one transcriptional pause sequence.

10. The kinetically-controlled RNA biosensor construct of any of claims 1 to 9, wherein when the output domain is folded into an active conformation the output domain is or comprises a functional ribozyme, a functional nuclease guide RNA (gRNA), a ribosome binding site, a transcriptional terminator, or RNA aptamer.

11. The kinetically-controlled RNA biosensor construct of claim 1, wherein the ligand of interest is a chemical, a metabolite, a protein, a peptide, a small molecule, optionally a drug molecule or drug precursor molecule, and the like.

12. A polynucleotide molecule comprising a sequence encoding the kinetically-controlled RNA biosensor construct of one of claims 1 to 11.

13. A vector comprising the polynucleotide molecule of claim 12 operatively linked to a promoter.

14. A cell comprising the polynucleotide molecule of claim 12 or the vector of claim 13.

15. A biosensor system, comprising:

a first expression cassette comprising a sequence encoding the kinetically-controlled RNA biosensor construct as recited in any of claims 1 to 11; and
an RNA polymerase and NTPs sufficient to facilitate synthesis of the kinetically-controlled RNA biosensor construct.

**Patentansprüche**

1. Ein kinetisch gesteuertes RNA-Biosensorkonstrukt, das von 5' nach 3' Folgendes umfasst:

eine Sensordomäne, die spezifisch an einen Liganden von Interesse bindet; und
eine Ausgangsdomäne, die dazu ausgelegt ist, ein detektierbares Ausgangssignal zu modulieren, wenn sie zu einer aktiven Konformation gefaltet wird;
wobei die Ausgangsdomäne, wenn sie transkribiert wird, zu der aktiven Konformation gefaltet wird, wenn die Sensordomäne an den Liganden von Interesse gebunden ist, und wobei die Ausgangsdomäne, wenn sie transkribiert wird, zu einer inaktiven Konformation gefaltet wird, wenn die Sensordomäne nicht an den Liganden von Interesse gebunden ist,
wobei die Sensordomäne Folgendes umfasst:

eine Aptamerdomäne, wobei die Aptamerdomäne von 5' nach 3' eine Stammsequenz, eine Linker-Zielsequenz, eine Aptamer-Subsequenz und eine Stamm-Zielsequenz umfasst; und
eine Überhangsequenz 5' von der Aptamerdomäne und optional eine Linkersequenz 3' von der Aptamerdomäne; und

wobei die Ausgangsdomäne von 5' nach 3' Folgendes umfasst:

eine Stamm-Zielsequenz,
eine Überhang-Zielsequenz und
eine Ausgangs-Subsequenz; und

wobei die Überhangsequenz der Sensordomäne das reverse Komplement mindestens eines Anteils der Überhang-Zielsequenz der Ausgangsdomäne ist.

2. Kinetisch gesteuertes RNA-Biosensorkonstrukt nach Anspruch 1, wobei die Sensordomäne die Linkersequenz umfasst.

3. Kinetisch gesteuertes RNA-Biosensorkonstrukt nach Anspruch 1, wobei:

   die Stammsequenz der Sensordomäne das reverse Komplement mindestens eines Anteils der Stamm-Zielsequenz der Sensordomäne ist und das reverse Komplement mindestens eines Anteils der Stamm-Zielsequenz der Ausgangsdomäne ist; und/oder
   die Linkersequenz der Sensordomäne das reverse Komplement mindestens eines Anteils der Linker-Zielsequenz der Sensordomäne ist.

4. Kinetisch gesteuertes RNA-Biosensorkonstrukt nach Anspruch 3, wobei die Linker-Zielsequenz das reverse Komplement eines Anteils der Aptamer-Subsequenz ist, wobei der Anteil der Aptamer-Subsequenz optional ein diskontinuierlicher Anteil ist.

5. Kinetisch gesteuertes RNA-Biosensorkonstrukt nach Anspruch 3 oder Anspruch 4, wobei, wenn die Sensordomäne an den Liganden von Interesse gebunden ist, die Stammsequenz der Sensordomäne an die Stamm-Zielsequenz der Sensordomäne hybridisiert wird, wodurch das Falten der Ausgangsdomäne zu der aktiven Konformation ermöglicht wird.

6. Kinetisch gesteuertes RNA-Biosensorkonstrukt nach Anspruch 3 oder Anspruch 4, wobei, wenn die Sensordomäne nicht an den Liganden von Interesse gebunden ist, die Überhangsequenz der Sensordomäne an den Anteil der Überhang-Zielsequenz der Ausgangsdomäne hybridisiert wird, die Stammsequenz der Sensordomäne an den Anteil der Stamm-Zielsequenz der Ausgangsdomäne hybridisiert wird und/oder die Linker-Zielsequenz der Sensordomäne an den Anteil der Linkersequenz in der Sensordomäne hybridisiert wird, wodurch das Falten der Ausgangsdomäne zu der inaktiven Konformation ermöglicht wird.

7. Kinetisch gesteuertes RNA-Biosensorkonstrukt nach Anspruch 6, wobei mindestens zwei von der Überhangsequenz der Sensordomäne, der Stammsequenz der Sensordomäne und der Linker-Zielsequenz der Sensordomäne eine kontinuierliche Helixstammstruktur bilden, wenn sie an mindestens einen Anteil der Überhang-Zielsequenz der Ausgangsdomäne, einen Anteil der Stamm-Zielsequenz der Ausgangsdomäne bzw. einen Anteil der Linkersequenz der Sensordomäne hybridisiert werden, wodurch der Ausgangs-Subsequenz der Ausgangsdomäne das Falten zu der inaktiven Konformation ermöglicht wird.

8. Kinetisch gesteuertes RNA-Biosensorkonstrukt nach einem der Ansprüche 1 bis 7, wobei:

   die Überhangsequenz eine Länge zwischen 0 und etwa 15 Nukleotiden aufweist; und/oder
   die Stammsequenz eine Länge zwischen 0 und etwa 15 Nukleotiden aufweist; und/oder
   die Linkersequenz eine Länge zwischen 0 und etwa 15 Nukleotiden aufweist.

9. Kinetisch gesteuertes RNA-Biosensorkonstrukt nach einem der Ansprüche 1 bis 8, das ferner eine Timer-Domäne umfasst, die zwischen der Aptamerdomäne und der Linkersequenz der Sensordomäne angeordnet ist, wobei die Timer-Domäne eine Länge von bis etwa 150 Nukleotiden aufweist, wobei die Timer-Domäne optional ferner mindestens eine Transkriptionspausensequenz umfasst.

10. Kinetisch gesteuertes RNA-Biosensorkonstrukt nach einem der Ansprüche 1 bis 9, wobei, wenn die Ausgangsdomäne zu einer aktiven Konformation gefaltet wird, die Ausgangsdomäne ein funktionelles Ribozym, eine funktionelle Nuklease-Guide-RNA (gRNA), eine Ribosomenbindungsstelle, ein Transkriptionsterminator oder RNA-Aptamer ist oder umfasst.

11. Kinetisch gesteuertes RNA-Biosensorkonstrukt nach Anspruch 1, wobei der Ligand von Interesse eine Chemikalie, ein Metabolit, ein Protein, ein Peptid, ein kleines Molekül, optional ein Wirkstoffmolekül oder Wirkstoffvorläufermolekül und Ähnliches ist.

12. Ein Polynukleotidmolekül, das eine Sequenz umfasst, die für das kinetisch gesteuerte RNA-Biosensorkonstrukt nach einem der Ansprüche 1 bis 11 kodiert.

**13.** Ein Vektor, der das Polynukleotidmolekül nach Anspruch 12, betriebsfähig mit einem Promoter verknüpft, umfasst.

**14.** Eine Zelle, die das Polynukleotidmolekül nach Anspruch 12 oder den Vektor nach Anspruch 13 umfasst.

**15.** Ein Biosensorsystem, das Folgendes umfasst:

eine erste Expressionskassette, die eine Sequenz umfasst, die für das kinetisch gesteuerte RNA-Biosensorkonstrukt nach einem der Ansprüche 1 bis 11 kodiert; und
eine RNA-Polymerase und ausreichend NTPs, um die Synthese des kinetisch gesteuerten RNA-Biosensorkonstrukts zu erleichtern.

**Revendications**

**1.** Construction de biocapteur à ARN sous contrôle cinétique, comprenant de 5' vers 3' :

un domaine capteur qui se lie spécifiquement à un ligand d'intérêt ; et
un domaine de sortie configuré pour moduler un signal de sortie détectable lorsqu'il est replié dans une conformation active ;
dans laquelle le domaine de sortie, quand il est transcrit, se replie dans la conformation active lorsque le domaine capteur est lié au ligand d'intérêt, et dans laquelle le domaine de sortie, quand il a été transcrit, se replie dans une conformation inactive lorsque le domaine capteur n'est pas lié au ligand d'intérêt,
dans laquelle le domaine capteur comprend :

un domaine aptamère, le domaine aptamère comprenant, de 5' vers 3', une séquence de tige, une séquence cible de liaison, une sous-séquence aptamère, et une séquence cible de tige ; et
une séquence de surplomb 5' du domaine aptamère et, optionnellement, une séquence de liaison 3' du domaine aptamère ; et

dans laquelle le domaine de sortie comprend, de 5' vers 3' :

une séquence cible de tige,
une séquence cible de surplomb, et
une sous-séquence de sortie ; et

dans laquelle la séquence de surplomb du domaine capteur est le complément inverse d'au moins une partie de la séquence cible de surplomb du domaine de sortie.

**2.** Construction de biocapteur à ARN sous contrôle cinétique selon la revendication 1, dans laquelle le domaine capteur comprend la séquence de liaison.

**3.** Construction de biocapteur à ARN sous contrôle cinétique selon la revendication 1, dans laquelle :

la séquence de tige du domaine capteur est le complément inverse d'au moins une partie de la séquence cible de tige du domaine capteur et est le complément inverse d'au moins une partie de la séquence cible de tige du domaine de sortie ; et/ou
la séquence de liaison du domaine capteur est le complément inverse d'au moins une partie de la séquence cible de liaison du domaine capteur.

**4.** Construction de biocapteur à ARN sous contrôle cinétique selon la revendication 3, dans laquelle la séquence cible de liaison est le complément inverse d'une partie de la sous-séquence aptamère, optionnellement dans laquelle la partie de la sous-séquence aptamère est une partie discontinue.

**5.** Construction de biocapteur à ARN sous contrôle cinétique selon la revendication 3 ou la revendication 4, dans laquelle, lorsque le domaine capteur est lié au ligand d'intérêt, la séquence de tige du domaine capteur est hybridée avec la séquence cible de tige du domaine capteur, en permettant ainsi un repliement du domaine de sortie dans la conformation active.

**6.** Construction de biocapteur à ARN sous contrôle cinétique selon la revendication 3 ou la revendication 4, dans laquelle, lorsque le domaine capteur n'est pas lié au ligand d'intérêt, la séquence de surplomb du domaine capteur est hybridée avec la partie de la séquence cible de surplomb du domaine de sortie, la séquence de tige du domaine capteur est hybridée avec la partie de la séquence cible de tige du domaine de sortie, et/ou la séquence cible de liaison du domaine capteur est hybridée avec la partie de la séquence de liaison dans le domaine capteur, en permettant ainsi un repliement du domaine de sortie dans la conformation inactive.

**7.** Construction de biocapteur à ARN sous contrôle cinétique selon la revendication 6, dans laquelle au moins deux de la séquence de surplomb du domaine capteur, de la séquence de tige du domaine capteur, et de la séquence cible de liaison du domaine capteur, forment une structure de tige hélicoïdale continue lorsqu'elles sont hybridées avec au moins une partie de la séquence cible de surplomb du domaine de sortie, une partie de la séquence cible de tige du domaine de sortie, et une partie de la séquence de liaison du domaine capteur, respectivement, en permettant ainsi à la sous-séquence de sortie du domaine de sortie de se replier dans la conformation inactive.

**8.** Construction de biocapteur à ARN sous contrôle cinétique selon l'une quelconque des revendications 1 à 7, dans laquelle :

la séquence de surplomb a une longueur entre 0 et environ 15 nucléotides ; et/ou
la séquence de tige a une longueur entre 0 et environ 15 nucléotides ; et/ou
la séquence de liaison a une longueur entre 0 et environ 15 nucléotides.

**9.** Construction de biocapteur à ARN sous contrôle cinétique selon l'une quelconque des revendications 1 à 8, comprenant en outre un domaine temporisateur disposé entre le domaine aptamère et la séquence de liaison du domaine capteur, dans laquelle le domaine temporisateur a une longueur de jusqu'à environ 150 nucléotides, optionnellement dans laquelle le domaine temporisateur comprend en outre au moins une séquence de pause transcriptionnelle.

**10.** Construction de biocapteur à ARN sous contrôle cinétique selon l'une quelconque des revendications 1 à 9, dans laquelle, lorsque le domaine de sortie est replié dans une conformation active, le domaine de sortie est ou comprend un ribozyme fonctionnel, un ARN guide (ARNg) de nucléase fonctionnel, un site de liaison au ribosome, un terminateur transcriptionnel, ou un aptamère ARN.

**11.** Construction de biocapteur à ARN sous contrôle cinétique selon la revendication 1, dans laquelle le ligand d'intérêt est une substance chimique, un métabolite, une protéine, un peptide, une petite molécule, optionnellement une molécule médicamenteuse ou une molécule précurseur médicamenteuse, et des composés similaires.

**12.** Molécule polynucléotidique comprenant une séquence codant la construction de biocapteur à ARN sous contrôle cinétique selon l'une quelconque des revendications 1 à 11.

**13.** Vecteur comprenant la molécule polynucléotidique selon la revendication 12 fonctionnellement lié à un promoteur.

**14.** Cellule comprenant la molécule polynucléotidique selon la revendication 12 ou le vecteur selon la revendication 13.

**15.** Système de biocapteur, comprenant :

une cassette de première expression comprenant une séquence codant la construction de biocapteur à ARN sous contrôle cinétique selon l'une quelconque des revendications 1 à 11 ; et
une ARN polymérase et des NTP suffisants pour faciliter la synthèse de la construction de biocapteur à ARN sous contrôle cinétique.

FIG. 1

# Input Domains

## Aptamer Domain

## Output Domain

*FIG. 2*

## Biosensor (ON)

**Ex.1**

**Ex.2**

*FIG. 2*
*(CONT.)*

Biosensor (OFF)

Ex.1

Ex.2

*FIG. 2*
*(CONT.)*

**FIG. 3A**

*Molecular architecture* — **Kinetic Aptazyme** — Aptamer, Ribozyme, Toe, Timer — (Invariable / Variable)

*In silico candidate sequences*

*Multi-state secondary structure objective functions* — $\Delta G2'$ — s3-co-txn, s4-co-txn, s5-full, s6-full, s5+full

*Rapid co-transcriptional folding pathways (MFEpath)* — $\Delta G2'$ — Text. <, Trearr., Fail, Text. >, Trearr., Pass — Sequence length

*Functional devices*

Insert candidate Timer domains

## FIG. 3B

Toehold Theophylline        S. mansoni
(5 nt)    Aptamer          Ribozyme
Theo1-0nt  $K_d = 540\ nM$   $k_{obs} = 1.3\ min^{-1}$

5'                                                    3'

Theo1-15nt              Timer      cleavage
                       (15 nt)      site

5'                                                    3'

**FIG. 4A**

*FIG. 4B*

*FIG. 4C*

FIG. 4D

*FIG. 4E*

EP 4 274 802 B1

*FIG. 5A*

**FIG. 5A**

*(CONT.)*

## FIG. 5A
### (CONT.)

FIG. 5B

**FIG. 5B**

*(CONT.)*

**FIG. 5B**

*(CONT.)*

*FIG. 5B*
*(CONT.)*

**FIG. 5B**
*(CONT.)*

**FIG. 5B**
*(CONT.)*

EP 4 274 802 B1

**FIG. 6A**

**FIG. 6B**

**FIG. 6C**

**FIG. 6D**

FIG. 7A

EP 4 274 802 B1

**FIG. 7B**

**FIG. 7C**

*FIG. 7D*

*FIG. 7E*

**FIG. 8**

**FIG. 9A**

**FIG. 9B**

**FIG. 9C**

**FIG. 10A**

**FIG. 10B**

**FIG. 11A**

**FIG. 11B**

**FIG. 12A**

**FIG. 12B**

## With Conventional Timer

Fraction Bound

Binding Window Time

Aptamer

[ ] binding window

Timer

## With Pause-Containing Timer

Fraction Bound

Binding Window Time

Aptamer

[ Pause Site → Transcriptional Pause → ] binding window

*FIG. 12C*

FIG. 13A

FIG. 13B

*FIG. 14A*

FIG. 14B

**Nature: Malfunctioning guide RNAs can drastically reduce CRISPR tool efficacy, or even cause complete loss of CRISPR function.**

*FIG. 15*

**FIG. 16**

**INPUT**

gRNA structure contraints

CRISPR application

spacer and activity objective functions

**WAYFINDER ALGORITHM**

generate candidate gRNA sequences

gGen

predict folding of expressed gRNAs

MFEpath

biophysical prediction of gRNA activity

gScore

**OUTPUT**

gRNAs with desired activity

*FIG. 17*

scRNA folding analysis
to calculate Folding Barrier

*FIG. 18A*

*FIG. 18B*

**FIG. 19**

EP 4 274 802 B1

FIG. 20

*R²: variance explained by regression model*
*Rs: rank-order correlation coefficient*

EP 4 274 802 B1

*FIG. 21*

$R^2$: variance explained by regression model
Rs: rank-order correlation coefficient

FIG. 21

(CONT.)

$R^2$: variance explained by regression model
Rs: rank-order correlation coefficient

FIG. 21

(CONT.)

*FIG. 22*

**FIG. 23**

# gRNA optimization using Wayfinder

*modify rest of the gRNA to maximize Wayfinder metrics*

*constrain 20 base target sequence*

## Available models are not compatible with changes outside the 20 base target sequence

**FIG. 24**

*Synthetic promoter for CRISPRa*

# FIG. 25

**FIG. 26A**

**FIG. 26B**

*Synthetic promoter for CRISPRa*

## FIG. 27A

## FIG. 27B

*FIG. 28A*

*FIG. 28B*

EP 4 274 802 B1

*FIG. 29*

FIG. 30

**FIG. 31A**

**FIG. 31B**

orthogonal synthetic promoters for CRISPRa

*FIG. 32A*

*FIG. 32B*

**FIG. 33**

FIG. 33
(CONT.)

EP 4 274 802 B1

combinatorial scRNA library

target genes under the control
of CRISPRa promoters

J306 ... J3 ... GFP

J506 ... J5 ... BFP

J606 ... J6 ... RFP

HIGH    MEDIUM    LOW    OFF

encoded activation

64 multi-gene expression programs

*FIG. 34A*

**FIG. 34B**

EP 4 274 802 B1

FIG. 34B

(CONT.)

EP 4 274 802 B1

*FIG. 35B*

*FIG. 35A*

**FIG. 36A**

FIG. 36B

FIG. 36C

FIG. 37A

*FIG. 37B*

*FIG. 38*

*3900*

START A
METHOD FOR DESIGNING KINETICALLY-
CONTROLLED FUNCTIONAL RNA
MOLECULES

A COMPUTING DEVICE DETERMINES ONE OR
MORE CANDIDATE KINETICALLY-CONTROLLED
FUNCTIONAL RNA SEQUENCES — 3902

FOR EACH CANDIDATE KINETICALLY-
CONTROLLED FUNCTIONAL RNA SEQUENCE — 3904

THE COMPUTING DEVICE PREDICTS ONE OR
MORE FOLDED STRUCTURES THAT THE
KINETICALLY-CONTROLLED FUNCTIONAL RNA
SEQUENCE FORMS OVER TIME — 3906

THE COMPUTING DEVICE DETERMINES ONE OR
MORE METRICS FOR THE KINETICALLY-
CONTROLLED FUNCTIONAL RNA SEQUENCE
BASED ON THE PREDICTED ONE OR MORE
FOLDED STRUCTURES — 3908

NEXT CANDIDATE KINETICALLY-CONTROLLED
FUNCTIONAL RNA SEQUENCE — 3910

THE COMPUTING DEVICE CHOOSES ONE OR
MORE OF THE ONE OR MORE CANDIDATE
KINETICALLY-CONTROLLED FUNCTIONAL RNA
SEQUENCES TO BE PROVIDED FOR SYNTHESIS
BASED ON THE METRICS — 3912

END

*FIG. 39*

*4000*

START A PROCEDURE
FOR PREDICTING ONE OR MORE FOLDED
STRUCTURES THAT A KINETICALLY-CONTROLLED
FUNCTIONAL RNA SEQUENCE FORMS
OVER TIME

A COMPUTING DEVICE SPECIFIES A
PREDETERMINED FOLDED STRUCTURE FOR A
PORTION OF THE KINETICALLY-CONTROLLED
FUNCTIONAL RNA SEQUENCE — *4002*

THE COMPUTING DEVICE PREDICTS AN OVERALL
FOLDED STRUCTURE FOR THE KINETICALLY-
CONTROLLED FUNCTIONAL RNA SEQUENCE GIVEN
THE PREDETERMINED FOLDED STRUCTURE FOR
THE PORTION OF THE KINETICALLY-
CONTROLLED FUNCTIONAL RNA SEQUENCE — *4004*

END
PROCEDURE

*FIG. 40*

4100

START A PROCEDURE
FOR PREDICTING ONE OR MORE FOLDED
STRUCTURES THAT A KINETICALLY-CONTROLLED
FUNCTIONAL RNA SEQUENCE FORMS
OVER TIME

A COMPUTING DEVICE PREDICTS A FIRST STRUCTURE FOR A
FIRST INCOMPLETE PORTION OF THE KINETICALLY-CONTROLLED
FUNCTIONAL RNA SEQUENCE WHILE BEING TRANSCRIBED ⟋4102

THE COMPUTING DEVICE PREDICTS A SECOND STRUCTURE FOR A
SECOND INCOMPLETE PORTION OF THE KINETICALLY-
CONTROLLED FUNCTIONAL RNA SEQUENCE WHILE BEING
TRANSCRIBED, WHERE THE SECOND INCOMPLETE PORTION OF
THE KINETICALLY-CONTROLLED FUNCTIONAL RNA SEQUENCE
INCLUDES THE FIRST INCOMPLETE PORTION OF THE
KINETICALLY-CONTROLLED FUNCTIONAL RNA SEQUENCE AND
ONE OR MORE ADDITIONAL NUCLEOTIDES OF THE KINETICALLY-
CONTROLLED FUNCTIONAL RNA SEQUENCE ⟋4104

THE COMPUTING DEVICE DETERMINES A BARRIER ENERGY FOR
CONVERTING FROM THE FIRST STRUCTURE TO THE SECOND
STRUCTURE ⟋4106

THE COMPUTING DEVICE DETERMINES A TIME FOR ADDING THE
SUBSEQUENT SET OF ONE OR MORE ADDITIONAL NUCLEOTIDES
OF THE KINETICALLY-CONTROLLED FUNCTIONAL RNA SEQUENCE
TO THE SECOND INCOMPLETE PORTION OF THE KINETICALLY-
CONTROLLED FUNCTIONAL RNA SEQUENCE ⟋4108

THE COMPUTING DEVICE CHOOSES THE FIRST STRUCTURE OR
THE SECOND STRUCTURE BASED ON WHETHER THE BARRIER
ENERGY IS TOO HIGH FOR THE SECOND INCOMPLETE PORTION
OF THE KINETICALLY-CONTROLLED FUNCTIONAL RNA SEQUENCE
TO TRANSITION FROM THE FIRST STRUCTURE TO THE SECOND
STRUCTURE DURING THE TIME FOR ADDING THE SUBSEQUENT
SET OF ONE OR MORE ADDITIONAL NUCLEOTIDES ⟋4110

END PROCEDURE

*FIG. 41*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WIDOM JR** ; **NEDIALKOV YA** ; **RAI V** ; **HAYES RL** ; **BROOKS CL 3RD** ; **ARTSIMOVITCH I** ; **WALTER NG**. Ligand Modulates Cross-Coupling between Riboswitch Folding and Transcriptional Pausing. *Mol Cell*, 01 November 2018, vol. 72 (3), 541-552. e6
- **BURKE, C.R.** ; **SPARKMAN-YAGER, D.** ; **CAROTHERS, J.M.** Multi-state design of kinetically-controlled RNA aptamer ribosensors. *bioRxiv*, 2017, 213538
- Chapter One - Automated 3D RNA Structure Prediction Using the RNAComposer Method for Riboswitches. **PURZYCKA. K. J. et al.** Methods in Enzymology. Academic Press, 2015, vol. 553, 3-34
- **HU, J. H. et al.** Evolved Cas9 variants with broad PAM compatibility and high DNA specificity. *Nature*, 2018, vol. 556, 57-63
- **CHAUVIER, A et al.** Role of a hairpin-stabilized pause in the Escherichia coli thiC riboswitch function. *RNA Biology*, 2019, vol. 16, 8, 1066-1073
- **KINGSTON, R. E.** ; **CHAMBERLIN, M. J.** Pausing and attenuation of in vitro transcription in the rrnB operon of E. coli.. *Cell*, 1981, vol. 27, 3, 523-531
- Viral Vectors for Gene Therapy: Methods and Protocols. Humana Press, 2003
- Current Topics in Microbiology and Immunology: Viral Expression Vectors. Springer-Verlag, 2012
- **FONTANA, J. et al.** Effective CRISPRa-mediated control of gene expression in bacteria must overcome strict target site requirements. *Nature Communications*, 2020, vol. 11, 1618
- **MALI P** ; **ESVELT KM** ; **CHURCH GM**. Cas9 as a versatile tool for engineering biology. *Nat Methods*, October 2013, vol. 10 (10), 957-63
- **DOMINGUEZ AA** ; **LIM WA** ; **QI LS. BEYOND**. editing: repurposing CRISPR-Cas9 for precision genome regulation and interrogation. *Nat Rev Mol Cell Biol.*, January 2016, vol. 17 (1), 5-15
- Current Protocols in Molecular Biology. John Wiley & Sons, 2010
- Current Protocols in immunology. John Wiley & Sons, 2010
- Modem Proteomics - Sample Preparation, Analysis and Practical Applications in Advances in Experimental Medicine and Biology. Springer International Publishing, 2016

- **MALI P.** ; **ESVELT KM** ; **CHURCH GM**. Cas9 as a versatile tool for engineering biology. *Nat Methods.*, October 2013, vol. 10 (10), 957-63
- **DOMINGUEZ AA** ; **LIM WA** ; **QI LS.** Beyond editing: repurposing CRISPR-Cas9 for precision genome regulation and interrogation. *Nat Rev Mol Cell Biol.*, January 2016, vol. 17 (1), 5-15
- **ALTSCHUL et al.** *J. Mol. Biol.*, 1990, vol. 215, 403-410
- **GEIS, M. et al.** Folding Kinetics of Large RNAs. *J. Mol. Biol.*, 2008, vol. 379, 160-173
- Chapter Sixteen - Kinetic Folding Design of Aptazyme-Regulated Expression Devices as Riboswitches for Metabolic Engineering. **SPARKMAN-YAGER, D. et al.** Methods in Enzymology. Academic Press, 2015, vol. 550, 321-340
- **SCHNEIDER, C. A. et al.** NIH Image to ImageJ: 25 years of image analysis. *Nature Method*, 2012, vol. 9.7, 671-675
- **ZIMMERMANN, G. R. et al.** Molecular interactions and metal binding in the theophylline-binding core of an RNA aptamer. *RNA*, 2000, vol. 6, 659-667
- **CAROTHERS, J. M et al.** Selecting RNA aptamers for synthetic biology: investigating magnesium dependence and predicting binding affinity. *Nucleic Acids Res.*, 2010, vol. 38, 2736-2747
- **CAROTHERS, J. M**. Model-Driven Engineering of RNA Devices to Quantitatively Program Gene Expression. *Science*, 2011, vol. 334, 1716-1719
- **LONG, D. M.** ; **UHLENBECK, O. C.** Kinetic characterization of intramolecular and intermolecular hammerhead RNAs with stem II deletions. *Proc. Natl. Acad. Sci.*, 1994, vol. 91, 6977-6981
- **ZHANG, D. Y** ; **SEELIG, G**. Dynamic DNA nanotechnology using strand-displacement reactions. *Nat. Chem.*, 2011, vol. 3, 103
- **LORENZ, R. et al.** ViennaRNA Package 2.0.. *Algorithms Mol. Biol.*, 2011, vol. 6, 26
- **CAROTHERS, J. M et al.** Model-Driven Engineering of RNA Devices to Quantitatively Program Gene Expression. *Science*, 2011, vol. 334, 1716-1719
- Chapter two - The Ribosome Binding Site Calculator. **SALIS, H. M.** Methods in Enzymology. Academic Press, 2011, vol. 498, 19-42
- **GOODMAN, D. B. et al.** Causes and Effects of N-Terminal Codon Bias in Bacterial Genes. *Science*, 2013, vol. 342, 475-479

- **DONG, C. et al.** Synthetic CRISPR-Cas gene activators for transcriptional reprogramming in bacteria. *Nat. Commun*, 2018, vol. 9, 2489
- **MORENO-MATEOS, M. A. et al.** CRISPRscan: designing highly efficient sgRNAs for CRISPR-Cas9 targeting in vivo. *Nat. Methods*, 2015, vol. 12, 982-988
- **HAEUSSLER, M. et al.** Evaluation of off-target and on-target scoring algorithms and integration into the guide RNA selection tool CRISPOR. *Genome Biol.*, 2016, vol. 17, 148
- **DOENCH, J. G. et al.** Optimized sgRNA design to maximize activity and minimize off-target effects of CRISPR-Cas9. *Nat. Biotechnol.*, 2016, vol. 34, 184-191
- **FONTANA et al.** Effective CRISPRa-mediated control of gene expression in bacteria must overcome strict target site requirements. *Nature Comm.*, 2020, vol. 11 (1), 1-11